# EUROPEAN PATENT SPECIFICATION

(11) **EP 3 820 859 B1**
(45) Date of publication and mention of the grant of the patent: **20.03.2024**
(21) Application number: 19746301.1
(22) Date of filing: 12.07.2019
(51) Int. Cl.: C07D 401/12, C07D 401/14, C07D 413/14, C07D 417/14, A61P 25/28, A61K 31/506

(54) **INHIBITORS OF HISTONE DEACETYLASE**
HISTONDEACETYLASE-HEMMER
INHIBITEURS DE L'HISTONE DÉSACÉTYLASE

(30) Priority: 13.07.2018 US 201862697498 P
(43) Date of publication of application: 19.05.2021
(73) Proprietor: Alkermes, Inc., Waltham, MA 02451-1420 (US)
(72) Inventor: FULLER, Nathan, Oliver, Arlington, MA 02474 (US); LOWE, John, A., III, Stonington, CT 06378 (US)
(74) Representative: Potter Clarkson
(86) International application number: PCT/US2019/041592
(87) International publication number: WO 2020/014605

(56) References cited:
- WO-A1-2017/007756
- US-A1- 2016 347 761

## Description

### RELATED APPLICATIONS

This application claims the benefit of priority to U.S. Provisional Application No. 62/697498, filed July 13, 2018.

### BACKGROUND

Inhibitors of histone deacetylases (HDAC) have been shown to modulate transcription and to induce cell growth arrest, differentiation and apoptosis. HDAC inhibitors also enhance the cytotoxic effects of therapeutic agents used in cancer treatment, including radiation and chemotherapeutic drugs. Marks, P., Rifkind, R. A., Richon, V. M., Breslow, R., Miller, T., Kelly, W. K. Histone deacetylases and cancer: causes and therapies. Nat Rev Cancer, 1, 194-202, (2001); and Marks, P. A., Richon, V. M., Miller, T., Kelly, W. K. Histone deacetylase inhibitors. Adv Cancer Res, 91, 137-168, (2004). Moreover, recent evidence indicates that transcriptional dysregulation may contribute to the molecular pathogenesis of certain neurodegenerative disorders, such as Huntington's disease, spinal muscular atrophy, amyotropic lateral sclerosis, and ischemia. Langley, B., Gensert, J. M., Beal, M. F., Ratan, R. R. Remodeling chromatin and stress resistance in the central nervous system: histone deacetylase inhibitors as novel and broadly effective neuroprotective agents. Curr Drug Targets CNS Neurol Disord, 4, 41-50, (2005). A recent review has summarized the evidence that aberrant histone acetyltransferase (HAT) and histone deacetylases (HDAC) activity may represent a common underlying mechanism contributing to neurodegeneration. Moreover, using a mouse model of depression, Nestler has recently highlighted the therapeutic potential of histone deacetylation inhibitors (HDAC5) in depression. Tsankova, N. M., Berton, O., Renthal, W., Kumar, A., Neve, R. L., Nestler, E. J. Sustained hippocampal chromatin regulation in a mouse model of depression and antidepressant action. Nat Neurosci, 9, 519-525, (2006).

There are 18 known human histone deacetylases, grouped into four classes based on the structure of their accessory domains. Class I includes HDAC1, HDAC2, HDAC3, and HDAC8 and has homology to yeast RPD3. HDAC4, HDAC5, HDAC7, and HDAC9 belong to class IIa and have homology to yeast. HDAC6 and HDAC10 contain two catalytic sites and are classified as class IIb. Class III (the sirtuins) includes SIRT1, SIRT2, SIRT3, SIRT4, SIRT5, SIRT6, and SIRT7. HDAC11 is another recently identified member of the HDAC family and has conserved residues in its catalytic center that are shared by both class I and class II deacetylases and is sometimes placed in class IV.

In contrast, HDACs have been shown to be powerful negative regulators of long-term memory processes. Nonspecific HDAC inhibitors enhance synaptic plasticity as well as long-term memory (Levenson et al., 2004, J. Biol. Chem. 279:40545-40559; Lattal et al., 2007, Behav Neurosci 121:1125-1131; Vecsey et al., 2007, J. Neurosci 27:6128; Bredy, 2008, Learn Mem 15:460-467; Guan et al., 2009, Nature 459:55-60; Malvaez et al., 2010, Biol. Psychiatry 67:36-43; Roozendaal et al., 2010, J. Neurosci. 30:5037-5046). For example, HDAC inhibition can transform a learning event that does not lead to long-term memory into a learning event that does result in significant long-term memory (Stefanko et al., 2009, Proc. Natl. Acad. Sci. USA 106:9447-9452). Furthermore, HDAC inhibition can also generate a form of long-term memory that persists beyond the point at which normal memory fails. HDAC inhibitors have been shown to ameliorate cognitive deficits in genetic models of Alzheimer's disease (Fischer et al., 2007, Nature 447:178-182; Kilgore et al., 2010, Neuropsychopharmacology 35:870-880). These demonstrations suggest that modulating memory via HDAC inhibition has considerable therapeutic potential for many memory and cognitive disorders.

Currently, the role of individual HDACs in long-term memory has been explored in two recent studies. Kilgore et al. 2010, Neuropsychopharmacology 35:870-880 revealed that nonspecific HDAC inhibitors, such as sodium butyrate, inhibit class I HDACs (HDAC1, HDAC2, HDAC3, HDAC8) with little effect on the class IIa HDAC family members (HDAC4, HDAC5, HDAC7, HDAC9). This suggests that inhibition of class I HDACs may be critical for the enhancement of cognition observed in many studies. Indeed, forebrain and neuron specific over expression of HDAC2, but not HDAC1, decreased dendritic spine density, synaptic density, synaptic plasticity and memory formation (Guan et al., 2009, Nature, 459:55-60). In contrast, HDAC2 knockout mice exhibited increased synaptic density, increased synaptic plasticity and increased dendritic density in neurons. These HDAC2 deficient mice also exhibited enhanced learning and memory in a battery of learning behavioral paradigms. This work demonstrates that HDAC2 is a key regulator of synaptogenesis and synaptic plasticity. Additionally, Guan et al. showed that chronic treatment of mice with SAHA (an HDAC 1,2,3,6, 8 inhibitor) reproduced the effects seen in the HDAC2 deficient mice and recused the cognitive impairment in the HDAC2 overexpression mice.

The inhibition of the HDAC2 (selectively or in combination with inhibition of other class I HDACs) is an attractive therapeutic target. Such inhibition has the potential for enhancing cognition and facilitating the learning process through increasing synaptic and dendritic density in neuronal cell populations. In addition, inhibition of HDAC2 may also be therapeutically useful in treating a wide variety of other diseases and disorders.

WO 2017/007756 describes compounds that are inhibitors of HDAC2.

### SUMMARY

Embodiments of the invention are described in the claims.

Provided herein are compounds of the Formula **VI** or **VII:** 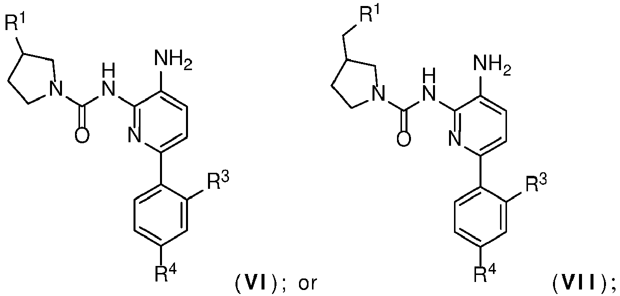 and pharmaceutically acceptable salts and compositions thereof, wherein R¹, R³, and R⁴ are as described herein. The disclosed compounds and compositions modulate histone deacetylases (HDAC) (see e.g., **Table 2** and **3**), and are useful in a variety of therapeutic applications such as, for example, in treating neurological disorders, memory or cognitive function disorders or impairments, extinction learning disorders, fungal diseases or infections, inflammatory diseases, hematological diseases, neoplastic diseases, psychiatric disorders, and memory loss.

Certain compounds described herein have an increase in inhibitory activity in a cell lysate assay over direct comparators. For example, it was found that introducing aromatic substitutions at the 3-positon of the pyrrolidine (e.g., the phenyl and heteroaryl variables for R¹) led to a significant increase in potency in an HDAC2 SH-SY5Y cell lysate assay when compared with counterparts possessing non-aromatic substitution at the pyrrolidine-3-position. See e.g., **Table 4,** where **Compounds 19** and **20,** each having an aromatic pyrimidinyl at R¹, have greater potency then the non-cyclic, non-aromatic, **Comparators A-C.**

### DETAILED DESCRIPTION

### 1. General Description of Compounds

Provided herein is a compound of the Formula **VI** or **VII:** 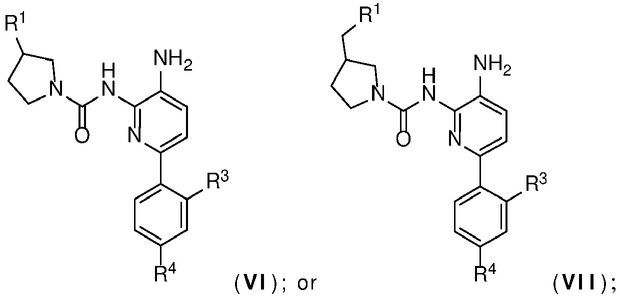 or a pharmaceutically acceptable salt thereof, wherein
R¹ is phenyl or heteroaryl, each of which are optionally substituted with 1 to 3 groups selected from R^{c};
R³ is hydrogen or fluoro;
R⁴ is fluoro; and
R^{c} is halo, (C₁-C₄)alkyl, halo(C₁-C₄)alkyl, (C₁-C₄)alkoxy, halo(C₁-C₄)alkoxy, (Ci-C₄)alkylO(C₁-C₄)alkyl, (C₁-C₄)alkylNH(C₁-C₄)alkyl, (C₁-C₄)alkylN((C₁-C₄)alkyl)₂, -(Ci-C₄)alkylheteroaryl, or -(C₁-C₄)alkylheterocyclyl, wherein said heteroaryl and heterocyclyl are each optionally and independently substituted with 1 to 3 groups selected from (C₁-C₄)alkyl, halo(C₁-C₄)alkyl, (C₁-C₄)alkoxy, and halo;

wherein heteroaryl represents a 5- to 12-membered aromatic radical containing 1-4 heteroatoms selected from N, O, and S; and
heterocyclyl represents a 4- to 12-membered saturated or partially unsaturated heterocyclic ring containing 1 to 4 heteroatoms independently selected from N, O, and S.

### 2. Definitions

When used in connection to describe a chemical group that may have multiple points of attachment, a hyphen (-) designates the point of attachment of that group to the variable to which it is defined. For example, -(C₁-C₄)alkylheteroaryl and -(C₁-C₄)alkylheterocyclyl means that the point of attachment occurs on the (C₁-C₄)alkyl residue.

The terms "halo" and "halogen" refer to an atom selected from fluorine (fluoro, -F), chlorine (chloro, -Cl), bromine (bromo, -Br), and iodine (iodo, -I).

The term "alkyl" when used alone or as part of a larger moiety, such as "haloalkyl", means a saturated straight-chain or branched monovalent hydrocarbon radical. Unless otherwise specified, an alkyl group typically has 1-6 carbon atoms, *i.e.,* (C₁-C₆)alkyl.

The term "haloalkyl" includes mono, poly, and perhaloalkyl groups where the halogens are independently selected from fluorine, chlorine, bromine, and iodine.

"Alkoxy" means an alkyl radical attached through an oxygen linking atom, represented by -O-alkyl. For example, "(C₁-C₄)alkoxy" includes methoxy, ethoxy, proproxy, and butoxy.

"Haloalkoxy" is a haloalkyl group which is attached to another moiety via an oxygen atom such as, e.g., but are not limited to -OCHF₂ or -OCF₃.

The term "heteroaryl" refers to a 5- to 12-membered (e.g., 5- or 6-membered) aromatic radical containing 1-4 heteroatoms selected from N, O, and S. A heteroaryl group may be mono- or bi-cyclic. Monocyclic heteroaryl includes, for example, thienyl, furanyl, pyrrolyl, imidazolyl, pyrazolyl, triazolyl, tetrazolyl, oxazolyl, isoxazolyl, oxadiazolyl, thiazolyl, isothiazolyl, thiadiazolyl, pyridyl, pyridazinyl, pyrimidinyl, pyrazinyl, etc. Bi-cyclic heteroaryls include groups in which a monocyclic heteroaryl ring is fused to one or more aryl or heteroaryl rings. Nonlimiting examples include indolyl, imidazopyridinyl, benzooxazolyl, benzooxodiazolyl, indazolyl, benzimidazolyl, benzthiazolyl, quinolyl, quinazolinyl, quinoxalinyl, pyrrolopyridinyl, pyrrolopyrimidinyl, pyrazolopyridinyl, thienopyridinyl, thienopyrimidinyl, indolizinyl, purinyl, naphthyridinyl, and pteridinyl. It will be understood that when specified, optional substituents on a heteroaryl group may be present on any substitutable position and, include, *e.g.,* the position at which the heteroaryl is attached.

The term "heterocyclyl" means a 4- to 12-membered (e.g., 4- to 6-membered) saturated or partially unsaturated heterocyclic ring containing 1 to 4 heteroatoms independently selected from N, O, and S. A heterocyclyl ring can be mononcyclic, bicyclic (e.g., a bridged, fused, or spiro bicyclic ring), or tricyclic. A heterocyclyl ring can be attached to its pendant group at any heteroatom or carbon atom that results in a stable structure. Examples of such saturated or partially unsaturated heterocyclic radicals include, without limitation, tetrahydrofuranyl, tetrahydrothienyl, terahydropyranyl, pyrrolidinyl, pyridinonyl, pyrrolidonyl, piperidinyl, oxazolidinyl, piperazinyl, dioxanyl, dioxolanyl, morpholinyl, dihydrofuranyl, dihydropyranyl, dihydropyridinyl, tetrahydropyridinyl, dihydropyrimidinyl, oxetanyl, azetidinyl and tetrahydropyrimidinyl. The term "heterocyclyl" also includes, e.g., unsaturated heterocyclic radicals fused to another unsaturated heterocyclic radical or aryl or heteroaryl ring, such as for example, tetrahydronaphthyridine, indolinone, dihydropyrrolotriazole, imidazopyrimidine, quinolinone, dioxaspirodecane. It will also be understood that when specified, optional substituents on a heterocyclyl group may be present on any substitutable position and, include, *e.g.,* the position at which the heterocyclyl is attached (e.g., in the case of an optionally substituted heterocyclyl or heterocyclyl which is optionally substituted).

The term "fused" refers to two rings that share two adjacent ring atoms with one another.

The term "spiro" refers to two rings that shares one ring atom (e.g., carbon).

The term "bridged" refers to two rings that share three ring atoms with one another.

Enantiomers are one type of stereoisomer that can arise from a chiral center or chiral centers. Enantiomers are pairs of stereoisomers whose mirror images are not superimposable, most commonly because they contain an asymmetrically substituted carbon atom or carbon atoms that acts as a chiral center(s). "R" and "S" represent the absolute configuration of substituents around one or more chiral carbon atoms, where each chiral center is assigned the prefix "R" or "S" according to whether the chiral center configuration is right- (clockwise rotation) or left-handed (counter clockwise rotation). If the turn is clockwise or right-handed about a chiral carbon, the designation is "R" for rectus. If the turn is counter clockwise or left-handed about a chiral carbon, the designation is "S" for sinister.

When a single enantiomer is named or depicted by structure, the depicted or named enantiomer is at least 60%, 70%, 80%, 90%, 99% or 99.9% by weight optically pure. Percent optical purity by weight is the ratio of the weight of the enantiomer over the weight of the enantiomer plus the weight of its optical isomer.

When a compound is depicted structurally without indicating the stereochemistry at a chiral center, the structure includes either configuration at the chiral center or, alternatively, any mixture of configurations at the chiral center stereoisomers.

"Racemate" or "racemic mixture" means a compound of equimolar quantities of two enantiomers, wherein such mixtures exhibit no optical activity, *i.e.,* they do not rotate the plane of polarized light.

As used herein the terms "subject" and "patient" may be used interchangeably, and means a mammal in need of treatment, *e.g.,* companion animals (*e.g.,* dogs, cats, and the like), farm animals (*e.g*., cows, pigs, horses, sheep, goats and the like) and laboratory animals (e.g., rats, mice, guinea pigs and the like). Typically, the subject is a human in need of treatment.

Pharmaceutically acceptable salts as well as the neutral forms of the compounds described herein are included. For use in medicines, the salts of the compounds refer to non-toxic "pharmaceutically acceptable salts." Pharmaceutically acceptable salt forms include pharmaceutically acceptable acidic/anionic or basic/cationic salts. Pharmaceutically acceptable basic/cationic salts include, the sodium, potassium, calcium, magnesium, diethanolamine, n-methyl-D-glucamine, L-lysine, L-arginine, ammonium, ethanolamine, piperazine and triethanolamine salts. Pharmaceutically acceptable acidic/anionic salts include, *e.g.,* the acetate, benzenesulfonate, benzoate, bicarbonate, bitartrate, carbonate, citrate, dihydrochloride, gluconate, glutamate, glycollylarsanilate, hexylresorcinate, hydrobromide, hydrochloride, malate, maleate, malonate, mesylate, nitrate, salicylate, stearate, succinate, sulfate, tartrate, and tosylate.

The term "pharmaceutically acceptable carrier" refers to a non-toxic carrier, adjuvant, or vehicle that does not destroy the pharmacological activity of the compound with which it is formulated. Pharmaceutically acceptable carriers, adjuvants or vehicles that may be used in the compositions described herein include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The terms "treatment," "treat," and "treating" refer to reversing, alleviating, reducing the likelihood of developing, or inhibiting the progress of a disease or disorder, or one or more symptoms thereof, as described herein. In some embodiments, treatment may be administered after one or more symptoms have developed, *i.e*., therapeutic treatment. In other embodiments, treatment may be administered in the absence of symptoms. For example, treatment may be administered to a susceptible individual prior to the onset of symptoms (*e.g*., in light of a history of symptoms and/or in light of genetic or other susceptibility factors), *i.e.,* prophylactic treatment. Treatment may also be continued after symptoms have resolved, for example to prevent or delay their recurrence.

The term "effective amount" or "therapeutically effective amount" includes an amount of a compound described herein that will elicit a biological or medical response of a subject e.g., between 0.01 - 100 mg/kg body weight/day of the provided compound, such as e.g., 0.1 - 100 mg/kg body weight/day.

### 5. Description of Exemplary Compounds

In an embodiment, R¹ in any one of Formula **VI** or **VII** is phenyl or 5- to 6-membered monocyclic heteroaryl, each of which is optionally substituted with one or more groups selected from R^{c}, wherein the remaining variables are as described above for Formula **VI** or **VII.** Alternatively, R¹ in any one of Formula **VI,** or **VII** is phenyl, pyridinyl, pyrazinyl, pyridazinyl, or pyrimidinyl, each of which is optionally substituted with 1 to 2 groups selected from R^{c}, wherein the remaining variables are as described above for Formula **VI** or **VII.** In another alternative, R¹ in any one of Formula **VI** or **VII** is thiazolyl, thiadiazolyl, imidazolyl, pyrazolyl, or oxazolyl, each of which is optionally substituted with 1 to 2 groups selected from R^{c}, wherein the remaining variables are as described above for Formula **VI** or **VII.**

In another embodiment, R^{c} in any one of Formula **VI** or **VII** is halo, (C₁-C₄)alkyl, or (C₁-C₄)alkylO(C₁-C₄)alkyl, wherein the remaining variables are as described above for Formula **VI** or **VII.** Alternatively, R^{c} in any one of Formula **VI** or **VII** is fluoro, methyl, or CH₂OCH₃, wherein the remaining variables are as described above for Formula **VI or VII.** In another alternative, R^{c} in any one of Formula **VI** or **VII** is halo, halo(C₁-C₄)alkyl, or (C₁-C₄)alkyl, wherein the remaining variables are as described above for Formula **VI** or **VII.** In another alternative, R^{c} in any one of Formula **VI** or **VII** is fluoro, methyl, or CHF₂, wherein the remaining variables are as described above for Formula **VI or VII.**

In another embodiment, provided is a compound as described below in the Exemplification section. Pharmaceutically acceptable salts and free forms of the exemplified compounds are included.

### 4. Uses, Formulation and Administration

The references to methods of treatment used herein are to be interpreted as references to the compounds, pharmaceutical compositions and medicaments of the present invention for use in a method for treatment of the human (or animal) body by therapy (or for diagnosis).

In some embodiments, the compounds and compositions described herein are useful in treating conditions associated with the activity of HDAC. Such conditions include for example, those described below.

Recent reports have detailed the importance of histone acetylation in central nervous system ("CNS") functions such as neuronal differentiation, memory formation, drug addiction, and depression (Citrome, Psychopharmacol. Bull. 2003, 37, Suppl. 2, 74-88; Johannessen, CNS Drug Rev. 2003, 9, 199-216; Tsankova et al., 2006, Nat. Neurosci. 9, 519-525). Thus, in one aspect, the provided compounds and compositions may be useful in treating a neurological disorder. Examples of neurological disorders include: (i) chronic neurodegenerative diseases such as familial and sporadic amyotrophic lateral sclerosis (FALS and ALS, respectively), familial and sporadic Parkinson's disease, Huntington's disease, familial and sporadic Alzheimer's disease, multiple sclerosis, muscular dystrophy, olivopontocerebellar atrophy, multiple system atrophy, Wilson's disease, progressive supranuclear palsy, diffuse Lewy body disease, fronto-temporal lobar degeneration (FTLD), corticodentatonigral degeneration, progressive familial myoclonic epilepsy, strionigral degeneration, torsion dystonia, familial tremor, Down's Syndrome, Gilles de la Tourette syndrome, Hallervorden-Spatz disease, diabetic peripheral neuropathy, dementia pugilistica, AIDS Dementia, age related dementia, age associated memory impairment, and amyloidosis-related neurodegenerative diseases such as those caused by the prion protein (PrP) which is associated with transmissible spongiform encephalopathy (Creutzfeldt-Jakob disease, Gerstmann-Straussler-Scheinker syndrome, scrapic, and kuru), and those caused by excess cystatin C accumulation (hereditary cystatin C angiopathy); and (ii) acute neurodegenerative disorders such as traumatic brain injury (e.g., surgery-related brain injury), cerebral edema, peripheral nerve damage, spinal cord injury, Leigh's disease, Guillain-Barre syndrome, lysosomal storage disorders such as lipofuscinosis, Alper's disease, restless leg syndrome, vertigo as result of CNS degeneration; pathologies arising with chronic alcohol or drug abuse including, for example, the degeneration of neurons in locus coeruleus and cerebellum, drug-induced movement disorders; pathologies arising with aging including degeneration of cerebellar neurons and cortical neurons leading to cognitive and motor impairments; and pathologies arising with chronic amphetamine abuse to including degeneration of basal ganglia neurons leading to motor impairments; pathological changes resulting from focal trauma such as stroke, focal ischemia, vascular insufficiency, hypoxic-ischemic encephalopathy, hyperglycemia, hypoglycemia or direct trauma; pathologies arising as a negative side-effect of therapeutic drugs and treatments (e.g., degeneration of cingulate and entorhinal cortex neurons in response to anticonvulsant doses of antagonists of the NMDA class of glutamate receptor) and Wernicke-Korsakoff's related dementia. Neurological disorders affecting sensory neurons include Friedreich's ataxia, diabetes, peripheral neuropathy, and retinal neuronal degeneration. Other neurological disorders include nerve injury or trauma associated with spinal cord injury. Neurological disorders of limbic and cortical systems include cerebral amyloidosis, Pick's atrophy, and Rett syndrome. In another aspect, neurological disorders include disorders of mood, such as affective disorders and anxiety; disorders of social behavior, such as character defects and personality disorders; disorders of learning, memory, and intelligence, such as mental retardation and dementia. Thus, in one aspect the disclosed compounds and compositions may be useful in treating schizophrenia, delirium, attention deficit disorder (ADD), schizoaffective disorder, Alzheimer's disease, Rubinstein-Taybi syndrome, depression, mania, attention deficit disorders, drug addiction, dementia, agitation, apathy, anxiety, psychoses, personality disorders, bipolar disorders, unipolar affective disorder, obsessive-compulsive disorders, eating disorders, post-traumatic stress disorders, irritability, adolescent conduct disorder and disinhibition.

Transcription is thought to be a key step for long-term memory processes (Alberini, 2009, Physiol. Rev. 89, 121-145). Transcription is promoted by specific chromatin modifications, such as histone acetylation, which modulate histone-DNA interactions (Kouzarides, 2007, Cell, 128:693-705). Modifying enzymes, such as histone acetyltransferases (HATs) and histone deacetylases (HDACs), regulate the state of acetylation on histone tails. In general, histone acetylation promotes gene expression, whereas histone deacetylation leads to gene silencing. Numerous studies have shown that a potent HAT, cAMP response element-binding protein (CREB)-binding protein (CBP), is necessary for long-lasting forms of synaptic plasticity and long term memory (for review, see Barrett, 2008, Learn Mem 15:460-467). Thus, in one aspect, the provided compounds and compositions may be useful for promoting cognitive function and enhancing learning and memory formation.

The compounds and compositions described herein may also be used for treating fungal diseases or infections.

In another aspect, the compounds and compositions described herein may be used for treating inflammatory diseases such as stroke, rheumatoid arthritis, lupus erythematosus, ulcerative colitis and traumatic brain injuries (Leoni et al., PNAS, 99(5); 2995-3000(2002); Suuronen et al. J. Neurochem. 87; 407-416 (2003) and Drug Discovery Today, 10: 197-204 (2005).

In yet another aspect, the compounds and compositions described herein may be used for treating a cancer caused by the proliferation of neoplastic cells. Such cancers include e.g., solid tumors, neoplasms, carcinomas, sarcomas, leukemias, lymphomas and the like. In one aspect, cancers that may be treated by the compounds and compositions described herein include, but are not limited to: cardiac cancer, lung cancer, gastrointestinal cancer, genitourinary tract cancer, liver cancer, nervous system cancer, gynecological cancer, hematologic cancer, skin cancer, and adrenal gland cancer. In one aspect, the compounds and compositions described herein are useful in treating cardiac cancers selected from sarcoma (angiosarcoma, fibrosarcoma, rhabdomyosarcoma, liposarcoma), myxoma, rhabdomyoma, fibroma, lipoma and teratoma. In another aspect, the compounds and compositions described herein are useful in treating a lung cancer selected from bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, and mesothelioma. In one aspect, the compounds and compositions described herein are useful in treating a gastrointestinal cancer selected from esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), and large bowel (adenocarcinoma, tubular adenoma, villous adenoma, hamartoma, leiomyoma). In one aspect, the compounds and compositions described herein are useful in treating a genitourinary tract cancer selected from kidney (adenocarcinoma, Wilm's tumor [nephroblastoma], lymphoma, leukemia), bladder and urethra (squamous cell carcinoma, transitional cell carcinoma, adenocarcinoma), prostate (adenocarcinoma, sarcoma), and testis (seminoma, teratoma, embryonal carcinoma, teratocarcinoma, choriocarcinoma, sarcoma, interstitial cell carcinoma, fibroma, fibroadenoma, adenomatoid tumors, lipoma). In one aspect, the compounds and compositions described herein are useful in treating a liver cancer selected from hepatoma (hepatocellular carcinoma), cholangiocarcinoma, hepatoblastoma, angiosarcoma, hepatocellular adenoma, and hemangioma.

In some embodiments, the compounds described herein relate to treating, a bone cancer selected from osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochondroma (osteocartilaginous exostoses), benign chondroma, chondroblastoma, chondromyxofibroma, osteoid osteoma and giant cell tumors.

In one aspect, the compounds and compositions described herein are useful in treating a nervous system cancer selected from skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma [pinealoma], glioblastoma multiform, oligodendroglioma, schwannoma, retinoblastoma, congenital tumors), and spinal cord (neurofibroma, meningioma, glioma, sarcoma).

In one aspect, the compounds and compositions described herein are useful in treating a gynecological cancer selected from uterus (endometrial carcinoma), cervix (cervical carcinoma, pre-tumor cervical dysplasia), ovaries (ovarian carcinoma [serous cystadenocarcinoma, mucinous cystadenocarcinoma, unclassified carcinoma], granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), and fallopian tubes (carcinoma).

In one aspect, the compounds and compositions described herein are useful in treating a skin cancer selected from malignant melanoma, basal cell carcinoma, squamous cell carcinoma, Karposi's sarcoma, moles dysplastic nevi, lipoma, angioma, dermatofibroma, keloids, and psoriasis.

In one aspect, the compounds and compositions described herein are useful in treating an adrenal gland cancer selected from neuroblastoma.

In one aspect, the compounds and compositions described herein are useful in treating cancers that include, but are not limited to: leukemias including acute leukemias and chronic leukemias such as acute lymphocytic leukemia (ALL), Acute myeloid leukemia (AML), chronic lymphocytic leukemia (CLL), chronic myelogenous leukemia (CML) and Hairy Cell Leukemia; lymphomas such as cutaneous T-cell lymphomas (CTCL), noncutaneous peripheral T-cell lymphomas, lymphomas associated with human T-cell lymphotrophic virus (HTLV) such as adult T-cell leukemia/lymphoma (ATLL), Hodgkin's disease and non-Hodgkin's lymphomas, large-cell lymphomas, diffuse large B-cell lymphoma (DLBCL); Burkitt's lymphoma; mesothelioma, primary central nervous system (CNS) lymphoma; multiple myeloma; childhood solid tumors such as brain tumors, neuroblastoma, retinoblastoma, Wilm's tumor, bone tumors, and soft-tissue sarcomas, common solid tumors of adults such as head and neck cancers (e.g., oral, laryngeal and esophageal), genito urinary cancers (e.g., prostate, bladder, renal, uterine, ovarian, testicular, rectal and colon), lung cancer, breast cancer, pancreatic cancer, melanoma and other skin cancers, stomach cancer, brain tumors, liver cancer and thyroid cancer.

In one aspect, provided herein is a method of treating a subject suffering from a neurological disorder, memory or cognitive function disorder or impairment, extinction learning disorder, fungal disease or infection, inflammatory disease, hematological disease, psychiatric disorders, and neoplastic disease, comprising administering to the subject an effective amount a compound described herein, or a pharmaceutically acceptable salt thereof, or the composition comprising a compound described herein.

Also provided herein is a method of treating a subject suffering from (a) a cognitive function disorder or impairment associated with Alzheimer's disease, posterior cortical atrophy, normal-pressure hydrocephalus, Huntington's disease, seizure induced memory loss, schizophrenia, Rubinstein Taybi syndrome, Rett Syndrome, depression, Fragile X, Lewy body dementia, vascular dementia, vascular cognitive impairment (VCI), Binswanger's Disease, fronto-temporal lobar degeneration (FTLD), ADHD, dyslexia, major depressive disorder, bipolar disorder and social, cognitive and learning disorders associated with autism, traumatic brain injury (TBI), chronic traumatic encephalopathy (CTE), multiple sclerosis (MS), attention deficit disorder, anxiety disorder, conditioned fear response, panic disorder, obsessive compulsive disorder, posttraumatic stress disorder (PTSD), phobia, social anxiety disorder, substance dependence recovery, Age Associated Memory Impairment (AAMI), Age Related Cognitive Decline (ARCD), ataxia, Parkinson's disease, or Parkinson's disease dementia; or (b) a hematological disease selected from acute myeloid leukemia, acute promyelocytic leukemia, acute lymphoblastic leukemia, chronic myelogenous leukemia, myelodysplastic syndromes, and sickle cell anemia; or (c) a neoplastic disease; or (d) a disorder of learning extinction selected from fear extinction and post-traumatic stress disorder; or (e) hearing loss or a hearing disorder; or (f) fibrotic diseases, such as pulmonary fibrosis, renal fibrosis, cardiac fibrosis, and scleroderma; or (g) bone pain in patients with cancer; or (h) neuropathic pain; comprising administering to the subject an effective amount a compound described herein, or a pharmaceutically acceptable salt thereof, or the composition comprising a compound described herein.

Also provided is a method of treating a subject suffering from Alzheimer's disease, Huntington's disease, frontotemporal dementia, Friedreich's ataxia, post-traumatic stress disorder (PTSD), Parkinson's disease, or substance dependence recovery, comprising administering to the subject an effective amount a compound described herein, or a pharmaceutically acceptable salt thereof, or the composition comprising a compound described herein.

Also provided is a compound described herein, or a pharmaceutically acceptable salt thereof, or a provided composition, for treating one or more of the disclosed conditions.

Also provided is a compound described herein, or a pharmaceutically acceptable salt thereof, or a provided composition, for the manufacture of a medicament for treating one or more of the disclosed conditions.

Subjects may also be selected to be suffering from one or more of the described conditions prior to treatment with a compound described herein, or a pharmaceutically acceptable salt thereof, or a provided composition.

The present disclosure also provides pharmaceutically acceptable compositions comprising a compound described herein, or a pharmaceutically acceptable salt thereof; and a pharmaceutically acceptable carrier. These compositions can be used to treat one or more of the conditions described above.

Compositions described herein may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Liquid dosage forms, injectable preparations, solid dispersion forms, and dosage forms for topical or transdermal administration of a compound are included herein.

It should also be understood that a specific dosage and treatment regimen for any particular patient will depend upon a variety of factors, including age, body weight, general health, sex, diet, time of administration, rate of excretion, drug combination, the judgment of the treating physician, and the severity of the particular disease being treated. The amount of a provided compound in the composition will also depend upon the particular compound in the composition.

### EXEMPLIFICATION

As depicted in the Examples below, in certain exemplary embodiments, compounds are prepared according to the following general procedures. It will be appreciated that, although the general methods depict the synthesis of certain compounds of the present invention, the following general methods, and other methods known to one of ordinary skill in the art, can be applied to all compounds and subclasses and species of each of these compounds, as described herein.

### General Information

Spots were visualized by UV light (254 and 365 nm). Purification by column and flash chromatography was carried out using silica gel (200-300 mesh). Solvent systems are reported as the ratio of solvents.

NMR spectra were recorded on a Bruker 400 (400 MHz) spectrometer. ¹H chemical shifts are reported in δ values in ppm with tetramethylsilane (TMS, = 0.00 ppm) as the internal standard. See, e.g., the data provided in **Table 1.**

LCMS spectra were obtained on an Agilent 1200 series 6110 or 6120 mass spectrometer with ESI (+) ionization mode. See, e.g., the data provided in **Table 1.**

### Example 1

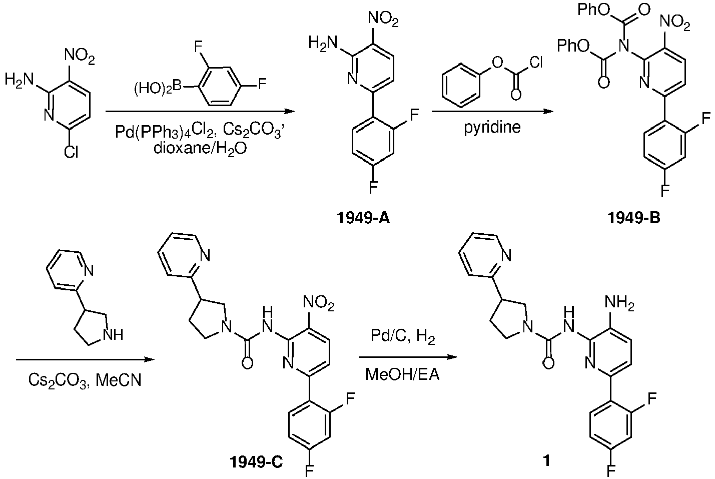

***Synthesis of 1949-A.*** A mixture of 6-chloro-3-nitropyridin-2-amine (4.58 g, 26.4 mmol), 2,4-difluorophenylboronic acid (5.00 g, 31.7 mmol) and Cs₂CO₃ (25.73 g, 79.2 mmol) in dioxane/H₂O (100 mL/10 mL) was treated with Pd(PPh₃)₄(1.10 g, 0.95 mmol) under a N₂ atmosphere. The mixture was stirred at 100 °C for 2 h and then concentrated *in vacuo.* The residue was dissolved with EtOAc (200 mL) and the resulting solution was washed with brine (100 mL × 3). The organic layer was dried over anhydrous Na₂SO₄ and then concentrated *in vacuo.* The residue was purified by column chromatography on silica gel (PE : EtOAc = 7 : 1 ~ 5 : 1) to give **1949-A** (4.0 g, 61%) as a yellow solid. MS 252.1 [M + H]⁺.

***Synthesis of 1949-B.*** A solution of **1949-A** (4.0 g, 15.94 mmol) in pyridine (60 mL) was cooled to 0 °C and then phenyl carbonochloridate (7.50 g, 47.81 mmol) was added dropwise. After the addition was completed, the mixture was heated to 50 °C and stirred at 50 °C for 4 h. The mixture was then concentrated *in vacuo,* and the residue was purified by column chromatography on silica gel (PE : DCM = 3: 2 ~ 1 : 1) to give **1949-B** (7.1 g, 91%) as a yellow solid. MS 492.1 [M + H]⁺.

***Synthesis of 1949-C.*** A mixture of **1949-B** (140 mg, 0.29 mmol), 2-pyrrolidin-3-yl-pyridine (51 mg, 0.34 mmol) and Cs₂CO₃ (279 mg, 0.86 mol) in acetonitrile (5 mL) was stirred at room temperature for 3 h. The mixture was then diluted with water (10 mL) and extracted with EtOAc (10 mL × 3). The combined organic layers were washed with brine (10 mL × 3), dried over anhydrous Na₂SO₄ and then concentrated *in vacuo.* The residue was purified by Prep-TLC (DCM : EtOAc = 1 : 1) to give **1949-C** (70 mg, 57%) as a yellow solid. MS 426.2 [M + H]⁺.

***Synthesis of Compound 1.*** A mixture of **1949-C** (70 mg, 0.16 mmol) and Pd/C (70 mg) in MeOH/EtOAc (3 mL/3 mL) was stirred at room temperature for 1 h under a H₂ atmosphere. The Pd/C was then removed by filtration through Celite, the filtrate was concentrated and the residue was purified by Prep-TLC (DCM : MeOH = 20 : 1) to give **Compound 1** (30 mg, 47%) as a yellow solid. MS 396.2 [M + H]⁺.

**Compounds 2-6** were synthesized in a similar manner using the appropriately substituted amine variant of the reagent used to synthesize **Compound 1.**

**Compound 2.** 40 mg, 54 %, a white solid.

**Compound 3.** 48 mg, 52 %, a white solid.

**Compound 4.** 70 mg, 69 %, a white solid.

**Compound 5.** 109 mg, 97 %, a white solid (prepared from commercially available chiral building blocks).

**Compound 6.** 95 mg, 73 %, a gray solid (prepared from commercially available chiral building blocks).

### Example 2

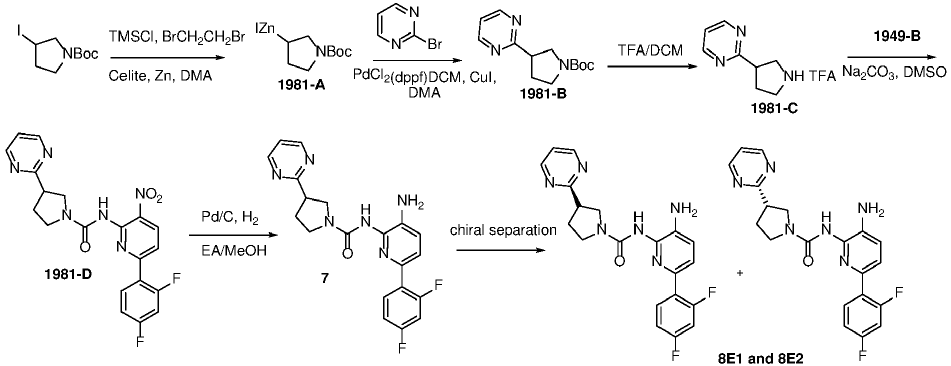

***Synthesis of 1981-A.*** A mixture of zinc dust (840 mg, 12.9 mmol) and Celite (180 mg) in a sealed flask was heated under vacuum with a heat gun for 5 min. The flask was purged with N₂ and cooled to room temperature. To the mixture was added anhydrous DMA (5.5 mL) followed by a mixture of TMSCl and 1,2-dibromoethane (0.3 mL, v/v=7/5). The mixture was stirred at room temperature for 15 min under a N₂ atmosphere, whereupon a solution of tert-butyl 3-iodopyrrolidine-1-carboxylate (3.10 g, 10.4 mmol) in DMA (5.5 mL) was added. The resulting mixture was stirred at room temperature for 4 h under N₂, and then the mixture was used taken on to the next step directly as **1981-A.** The concentration of **1981-A** was about 0.85 mol/L in DMA.

***Synthesis of 1981-B.*** A mixture of 2-bromopyrimidine (1.1 g, 6.92 mmol), CuI (197 mg, 1.04 mmol) and Pd(dppf)₂Cl₂ (452 mg, 0.55 mmol) in DMA (10 mL) under a N₂ atmosphere was treated with **1981-A** (10.0 mL). The resulting mixture was stirred at 85 °C for 48 h under a N₂ atmosphere, and then the mixture was diluted with water (50 mL) and extracted with EtOAc (20 mL × 3). The combined organic layers were washed with brine (20 mL × 3), dried over anhydrous Na₂SO₄ and then concentrated *in vacuo.* The residue was purified by column chromatography on silica gel (PE : EtOAc =10 : 1 ~ 1 : 1) to give **1981-B** (320 mg, 19%) as a yellow oil. MS 194.3 [M -56 + H]⁺.

***Synthesis of 1981-C.*** To a solution of **1981-B** (320 mg, 1.29 mmol) in DCM (6 mL) was added TFA (2 mL) dropwise. The reaction mixture was stirred at room temperature for 1 h, whereupon the solution was concentrated *in vacuo* to give **1981-C** as a crude product which was used directly in the next step without further purification. MS 150.3 [M + H]⁺.

***Synthesis of 1981-D.*** A mixture of **1981-C** (1.29 mmol, crude product from last step) and **1949-B** (352 mg, 0.72 mmol) in DMSO (10 mL) was stirred at room temperature for 10 min, then Na₂CO₃ (760 mg, 7.17 mmol) was added and the reaction mixture was stirred at room temperature for 2 h. The mixture was then diluted with water (30 mL) and extracted with EtOAc (20 mL × 3). The combined organic layers were washed with brine (20 mL × 3), dried over anhydrous Na₂SO₄ and then concentrated *in vacuo.* The residue was purified by Prep-TLC (DCM : MeOH = 40 : 1) to give **1981-D** (205 mg, 67%) as a yellow solid. MS 427.1 [M + H]⁺.

***Synthesis of Compound* 7.** A mixture of **1981-D** (205 mg, 0.48 mmol) and Pd/C (205 mg) in MeOH/EtOAc (5 mL/5 mL) was stirred at room temperature for 50 min under a H₂ atmosphere. The Pd/C was removed by filtration through Celite, the filtrate was concentrated *in vacuo* and the residue was purified by Prep-TLC (DCM : MeOH = 25 : 1) to give **Compound 7** (130 mg, 68%) as a brown solid. MS 397.2 [M + H]⁺.

***Chiral-separation of Compound* 7.** The enantiomers of racemic **Compound 7** (100 mg, 0.25 mmol) was separated by chiral chromatographic separation (Column: Chiralpak OJ-3; Solvent: MeOH; Flow rate: 2 mL/min; RT_{8E1} = 2.287 min, RT_{8E2} = 2.553 min) to give the first eluting peak **Enantiomer 1 (Compound 8E1)** (40 mg, 40%) as a yellow solid (MS 397.2 [M+H]⁺) and the second eluting peak **Enantiomer 2 (Compound 8E2)** (20 mg, 20%) as a yellow solid. MS 397.2 [M+H]⁺. Stereochemistry was randomly assigned.

**Compounds 9-21** were synthesized in a similar manner using appropriately substituted boronic acid and bromine variants of reagents used to synthesize **Compound 7.**

***Compound 9.*** 11 mg, 54%, a yellow solid.

***Compound 10.*** 11 mg, 47%, a white solid.

***Compound 11.*** 34 mg, 70%, a white solid.

***Compound 12.*** 18 mg, 48%, a white solid.

***Compound 13.*** 34 mg, 61%, a light yellow solid.

***Compound 15.*** 14 mg, 47%, a white solid.

***Compound 16.*** 35 mg, 63%, an off-white solid.

***Compound 17.*** 25 mg, 45%, a white solid.

***Compound 18.*** 30 mg, 52 %, an off-white solid.

***Compound 19.*** 15 mg, 28%, a light yellow solid.

***Compound 20.*** 11 mg, 47 %, a light yellow solid.

***Compound 21.*** 17 mg, 41%, a light yellow solid.

### Example 3. Synthesis of 14-E1 and 14-E2

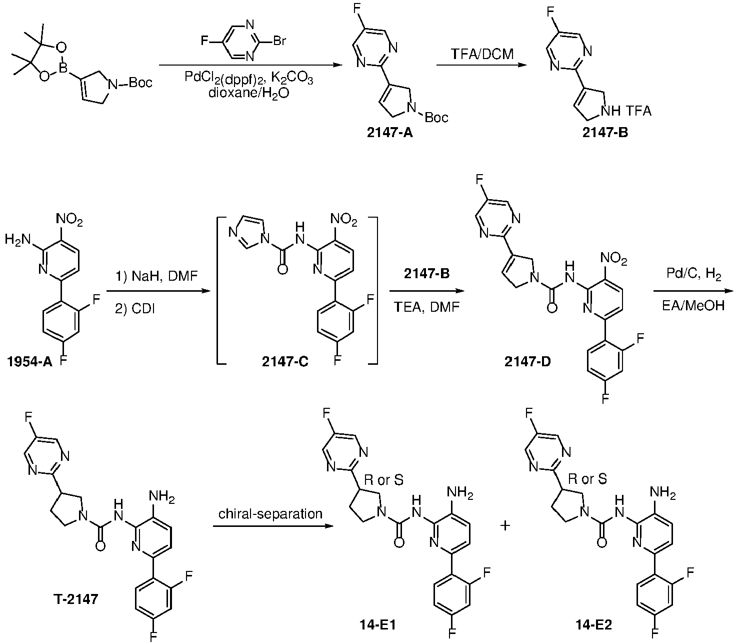

***Synthesis of 2147-A.*** To a mixture of *tert*-butyl 3-(4,4,5,5-tetramethyl-1,3,2-dioxaborolan-2-yl)-2,5-dihydro-1H-pyrrole-1-carboxylate (33.6 g, 113.9 mmol), 2-bromo-5-fluoropyrimidine (20 g, 113.6 mmol) and K₂CO₃ (47.1 g, 341 mmol) in dioxane/H₂O (500 mL/50 mL) was added PdCl₂(dppf)₂(4.6 g, 5.7 mmol) under a nitrogen atmosphere. The resulting mixture was stirred at 100 °C for 2 h and then concentrated *in vacuo.* The residue was dissolved with EtOAc (500 mL) and the solution was washed with brine (200 mL × 3). The organic layer was dried over anhydrous Na₂SO₄ and then concentrated *in vacuo.* The residue was purified by column chromatography on silica gel (PE : EtOAc = 10: 1 to 5 : 1) to give **2147-A** (25.5 g, 85 %) as a gray solid. MS 210.1 [M - 55]⁺.

***Synthesis of 2147-B.*** To a solution of **2147-A** (1.0 g, 6.0 mmol) in DCM (30 mL) was added TFA (10 mL) dropwise at 0 °C. The resulting solution was stirred at room temperature for 1 h, whereupon the solvent was removed *in vacuo* to give **2147-B** as a crude product which was used directly in the next step. MS 166.1 [M + H]⁺.

***Synthesis of 2147-D.*** A solution of **1954-A** (1.87 g, 7.45 mmol) in DMF (15 mL) was cooled to 0 °C and then was treated with NaH (60% in mineral oil) (596 mg, 14.9 mmol). The reaction mixture was stirred at 0 °C for 30 min, then CDI (1.20 g, 7.45 mmol) was added and stirring was continued at 0 °C for another 30 min. A solution of **2147-B** in DMF was added to the reaction mixture, and stirring was continued at 0 °C for 1 h. The mixture was then quenched with water (100 mL) and extracted with EtOAc (50 mL × 3). The combined organic layers were washed with brine (50 mL × 3), dried over anhydrous Na₂SO₄ and then concentrated *in vacuo.* The residue was purified by column chromatography (DCM : EtOAc = 15 : 1 to 2 : 1) to give **2147-D** (2.3 g, 70 %) as a yellow solid. MS 443.2 [M + H]⁺.

***Synthesis of 14.*** A mixture of **2147-D** (2.3 g, 5.2 mmol) and Pd/C (2.3 g) in MeOH/EtOAc (50 mL/50 mL) was stirred at room temperature for 1 h under a H₂ atmosphere. Pd/C was removed by filtration through a pad of Celite. The filtrate was concentrated *in vacuo* and the residue was purified by column chromatography (DCM : MeOH = 20 : 1) to give Compound **14** (1.14 g, 53%) as a white solid. MS 415.2 [M + H]⁺.

***Chiral-separation of 14-E1 and 14-E2.*** The enantiomers of **14** (1.14 g, 2.75 mmol) were separated by chiral SFC separation (Column: Chiralcel OD-3; Solvent: MeOH; Flow rate: 2 mL/min; RT_{T-2147-E1} = 1.849 min, RT_{T-2147-E2} = 2.175 min) to give Compound **14-E1 Isomer 1** (410 mg, 36%) as a yellow solid (MS 415.2 [M+H]⁺) and Compound **14-E2** (360 mg, 31%) as a yellow solid. MS 415.2 [M+H]⁺**Isomer 2.**

### Example 4. Synthesis of 19-E1 & 19-E2

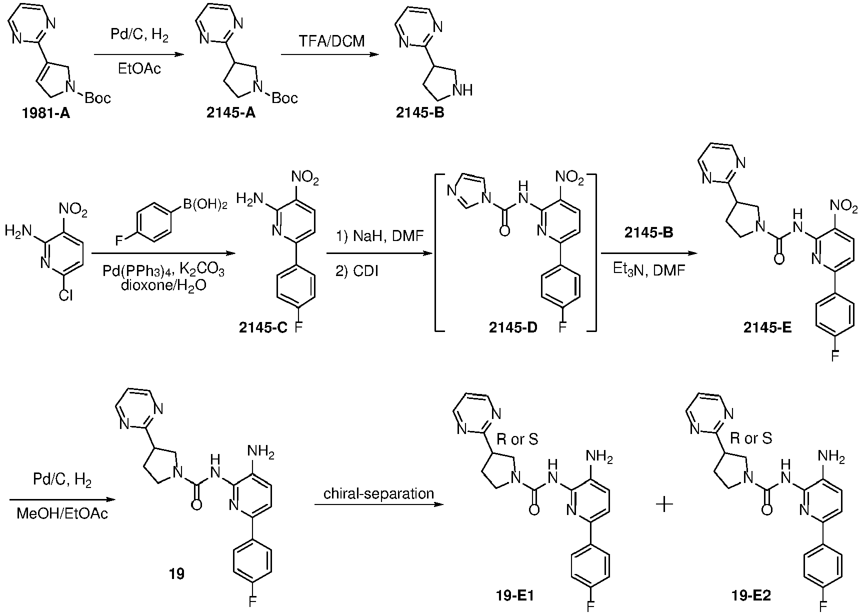

***Synthesis of 2145-A.*** A mixture of **1981-A** (1.7 g, 6.8 mmol) and Pd/C (850 mg) in EtOAc (80 mL) was stirred at room temperature for 1 h under a H₂ atmosphere. Pd/C was removed by filtration through Celite. The filtrate was concentrated *in vacuo* to give **2145-A** (1.6 g, 94%) as colorless oil. MS 194.2 [M -55]⁺

***Synthesis of 2145-B.*** To a solution of **2145-A** (1.3 g, 5.22 mmol) in DCM (18 mL) was added TFA (6 mL) dropwise at 0 °C. The reaction mixture was allowed to warm to room temperature and was stirred at room temperature for 1 h, whereupon the reaction mixture was concentrated *in vacuo.* The crude residue was dissolved in DMF (5mL) and treated with TEA (1.58 g, 15.66 mmol) to give **2145-B** as a solution used to next step directly. MS 150.0 [M + H]⁺.

***Synthesis of 2145-C.*** A mixture of 6-chloro-3-nitropyridin-2-amine (50.0 g, 289.0 mmol), 4-fluorophenylboronic acid (48.5 g, 346.8 mmol) and K₂CO₃ (119.6 g, 867 mmol) in dioxane/H₂O (1000 mL/100 mL) was treated with Pd(PPh₃)₄ (5.0 g, 4.33 mmol) under a N₂ atmosphere. The mixture was stirred at 95 °C for 4 h and then concentrated *in vacuo.* The residue was dissolved with EtOAc (2000 mL) and the solution was washed with brine (700 mL × 3). The organic layer was dried over anhydrous Na₂SO₄ and then concentrated *in vacuo.* The residue was purified by column chromatography on silica gel (PE : EtOAc = 10 : 1 ∼ 5 : 1) to give **2145-C** (36 g, 54%) as a yellow solid. MS 233.1 [M + H]⁺.

***Synthesis of 2145-E.*** A solution of **2145-C** (1.0 g, 4.35 mmol) in DMF (20 mL) was cooled to 0 °C and treated with NaH (60% in mineral oil)(210 mg, 5.22 mmol). The reaction mixture was stirred at 0 °C for 30 min, then CDI (846 mg, 5.22 mmol) was added into above mixture, and stirring was continued at 0 °C for another 30 min to give a solution as **2145-D.** The solution of **2145-B** was added into the solution of **2145-D** at 0 °C and stirred for 1 h. The reaction mixture was poured into water (420 mL), then extracted with EtOAc (50 mL × 3), washed with brine (50 mL × 3). The organic layer was dried over anhydrous Na₂SO₄ and then concentrated *in vacuo.* The residue was purified by column chromatography on silica gel (DCM : MeOH = 100 : 1 ∼ 30 : 1) to give **2145-E** (1.2 g, 56.3%) as yellow oil. MS 409.1 [M + H]⁺.

***Synthesis of 19.*** A mixture of **2145-E** (1.2 g, 2.94 mmol) and Pd/C (1.2 g) in MeOH/ EtOAc (20 mL/20 mL) was stirred at room temperature for 1 h under a H₂ atmosphere. Pd/C was removed by filtration through Celite. The filtrate was concentrated *in vacuo* and the residue was purified by column chromatography on silica gel (DCM : MeOH = 100 : 1 ∼ 15 : 1) to give **19** (700 mg, 63%) as a off-white solid. MS 379.4 [M + H]⁺.

***Chiral-separation of 19-E1 and 19-E2.*** The enantiomers of **19** (700 mg, 1.85 mmol) were separated by chiral SFC (Column: Chiralpak AD-3; Solvent: MeOH; Flow rate: 1.5 mL/min; RT_{T-2145-E2}= 5.544 min, to give **19-E2** (230 mg, 32.8%) as a white solid, and RT_{T-2145-E1} = 4.009 min, to give **19-E1** (260 mg, 37.1%) as a white solid. MS 379.4 [M+H]⁺.

### Example 5. Alternative Synthesis of 19-E1 & 19-E2

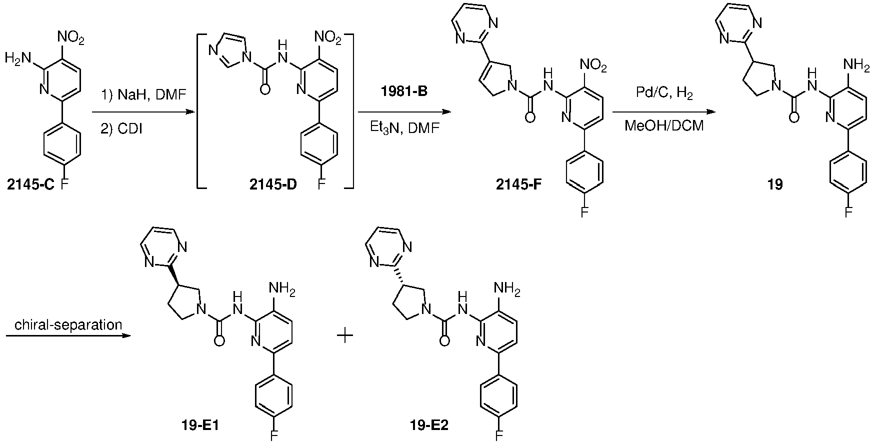

***Synthesis of 2145-F.*** A solution of **2145-C** (5.83 g, 25.0 mmol) in DMF (100 mL) was cooled to 0 °C and was treated with NaH(60% in mineral oil)(1.4 g, 35 mmol). The reaction mixture was stirred at 0 °C for 30 min, then CDI (4.86 g, 30 mmol) was added into above mixture and stirring was continued at 0 °C for another 30 min to give a solution as **2145-D.** Then a solution of **1981-B** (9.26 g, 37.5 mmol) and TEA (18.93 g, 187.5 mmol) in DMF (40 mL) was added into the solution of **2145-D** at 0 °C and the resulting reaction mixture was stirred at 0 °C for 1 h. The reaction mixture was then poured into water (420 mL) and stirred for 10 min. The precipitate was collected by filtered and the cake washed with water (150 mL), then acetone (150 mL). Finally, the cake was concentrated to dryness to give **2145-F** (9.5 g, 94%) as a light yellow solid. MS 407.1 [M + H]⁺.

***Synthesis of 19.*** A mixture of **2145-F** (8.5 g, 20.9 mmol) and Pd/C (8.5 g) in MeOH/DCM (250 mL/200 mL) was stirred at room temperature for 3 h under a H₂ atmosphere. Pd/C was removed by filtration through Celite. The filtrate was concentrated *in vacuo* and the residue was purified by column chromatography on silica gel (DCM : MeOH = 100 : 1 ~ 15 : 1) to give **19** (4.1 g, 50%) as a light yellow solid. MS 379.4 [M + H]⁺.

**Compounds 46, 50** and **51** were synthesized in a similar manner as **19** by using appropriately substituted boronic acid and aryl bromide reagents.

***Compound 46.*** 100 mg, 63%, a lighty yellow solid.

***Chiral-separation of 50 and 51.*** The enantiomers of **19** were separated by chiral SFC (Column: Chiralpak AD-3; Solvent: MeOH; Flow rate: 1.5 mL/min; RT₅₀= 3.267 min, and RT₅₁ = 5.375 min.

***Compound 50**.* 200 mg, 29%, a white solid.

***Compound 51.*** 230 mg, 33%, a white solid.

### Example 6. Synthesis of 20-E1 and 20-E2

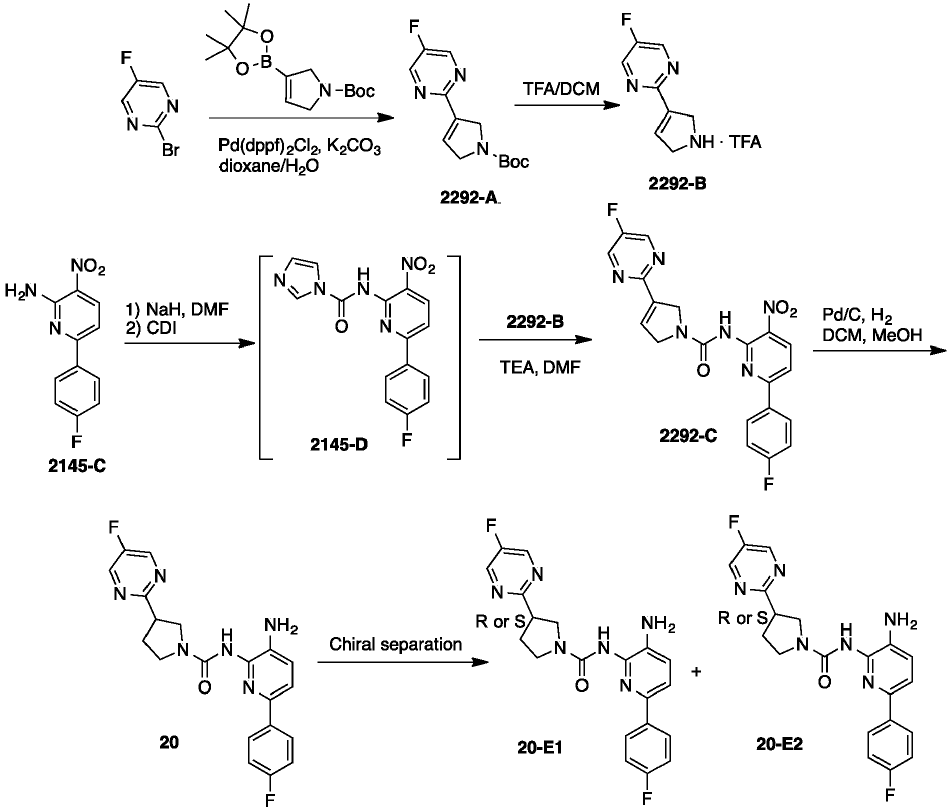

***Synthesis of 2292-A.*** A mixture of 2-bromo-5-fluoropyrimidine (20 g, 113.6 mmol), tert-butyl 3-(4,4,5,5-tetramethyl-1,3,2-dioxaborolan-2-yl)-2,5-dihydro-1H-pyrrole-1-carboxylate (33.7 g, 113.6 mmol) and K₂CO₃ (47.0 g, 340.8 mmol) in dioxane/H₂O (500 mL/50 mL) were treated with Pd(dppf)₂Cl₂ (4.64 g, 5.7 mmol) under a N₂ atmosphere. The reaction mixture was stirred at 90 °C for 3 h and then concentrated *in vacuo.* The residue was dissolved with EtOAc (200 mL) and the solution was washed with brine (100 mL × 3). The organic layer was dried over anhydrous Na₂SO₄ and then concentrated *in vacuo.* The residue was purified by column chromatography on silica gel (PE : DCM = 1 : 1 to DCM) to give **2292-A** (25.5 g, 85%) as a gray solid. MS 210.1 [M - 55]⁺.

***Synthesis of 2292-B.*** A mixture of **2292-A** (11.7 g, 44.1 mmol) in DCM (100 mL) was treated with TFA (30 mL) at 0 °C. The reaction mixture was allowed to warm to room temperature and was stirred at room temperature for 1 h. The solution was then concentrated *in vacuo,* and the residue was dissolved in DMF (50 mL) and treated with TEA (13.4 g, 132.3 mmol) to give **2292-B** as a solution used directly in the next step. MS 165.1 [M + H]⁺.

***Synthesis of 2292-C.*** A solution of **2145-C** (9.32 g, 40.0 mmol) in DMF (100 mL) was cooled to 0 °C and then was treated with NaH (60% in mineral oil)(1.92 g, 48.0 mmol). The reaction mixture was stirred at 0 °C for 30 min, then CDI (7.13 g, 44.0 mmol) was added into above mixture and stirring was continued at 0 °C another 30 min to give a solution as **2145-D.** Then a solution of **2292-B** was added into above mixture at 0 °C and the resulting mixture was stirred at 0 °C for 1 h. The reaction mixture was then poured into water (450 mL) and stirred for 10 min. The precipitate was collected by filtration and the cake washed with water (150 mL), then acetone (150 mL). Finally, the cake was concentrated to dryness to give **2292-C** (12.2 g, 70%) as a light yellow solid. MS 424.9 [M + H]⁺.

***Synthesis of 20.*** A solution of **2292-C** (12.2 g, 28.6 mmol) and Pd/C (12.2 g) in MeOH/DCM (400 mL/300 mL) was stirred at room temperature for 2.5 h under a H₂ atmosphere. Pd/C was removed by filtration through Celite. The filtrate was concentrated *in vacuo* and the residue was purified by column chromatography on silica gel (DCM to DCM : EA = 1 : 1) to give **20** (6.8 g, 60%) as a yellow solid. MS 397.0 [M + H]⁺.

***Chiral-separation of 20-E1 and 20-E2.*** The enantiomers of **20** (360 mg, 0.91 mmol) were separated by chiral SFC (Column: Chiralcel OJ-3; Solvent: MeOH; Flow rate: 1.5 mL/min; RT_{T-2292-E2} = 2.862 min, to give **20-E2** (100 mg, 28%) as a white solid, and RT_{T-2292-E1} = 2.338 min, to give **20-E1** (88 mg, 25%) as a white solid. MS 397.0 [M+H]⁺.

### Example 7 (reference example)

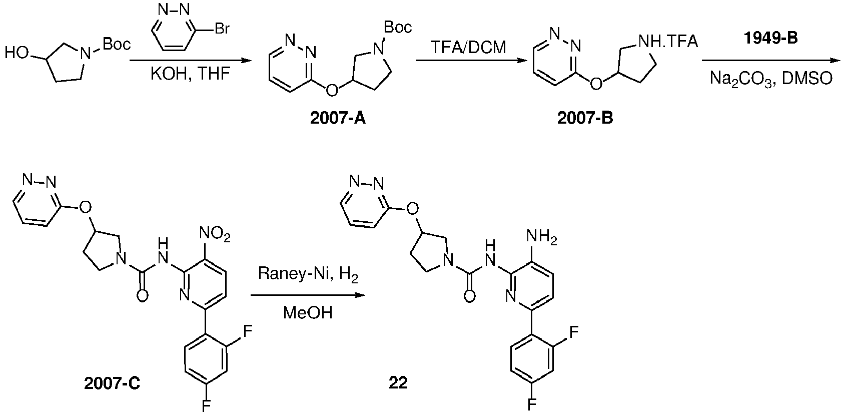

***Synthesis of 2007-A.*** A solution of tert-butyl 3-hydroxypyrrolidine-1-carboxylate (821 mg, 4.75 mmol) in THF (10 mL) was treated with 3-bromopyridazine (500 mg, 3.16 mmol) and KOH (798 mg, 4.25 mmol) at room temperature. The reaction mixture was then heated to 70 °C and stirred at 70 °C for 16 h. The mixture was then diluted with water (20 ml), and extracted with EtOAc (20 mL × 3). The combined organic layers were washed with brine (20 mL × 3), dried over anhydrous Na₂SO₄ and then concentrated *in vacuo.* The residue was purified by Prep-TLC (EtOAc : PE = 10 : 1) to give **2007-A** (70 mg, 8%) as a yellow oil. MS 288.2 [M + H]⁺.

***Synthesis of 2007-B.*** To a solution of **2007-A** (70 mg, 0.26 mmol) in DCM (6 mL) was added TFA (2 mL) dropwise. The reaction mixture was stirred at room temperature for 1 h, whereupon the solvent was removed *in vacuo* to give **2007-B** as a crude product which was used directly in the next step without further purification.

***Synthesis of 2007-C.*** A mixture of **1949-B** (71.0 mg, 0.14 mmol) and **2007-B** (0.26 mmol, crude product from last step) in DMSO (5 mL) was stirred at room temperature for 10 min, then was treated with Na₂CO₃ (276 mg, 2.6 mmol). The resulting reaction mixture was stirred at room temperature for 2 h, whereupon the mixture was diluted with water (20 mL) and extracted with EtOAc (10 mL × 3). The combined organic layer was washed with brine (10 mL × 3), dried over anhydrous Na₂SO₄ and then concentrated *in vacuo.* The residue was purified by Prep-TLC (EtOAc : PE = 5 : 1) to give **2007-C** (40 mg, 45%) as a yellow solid. MS 443.2 [M + H]⁺.

***Synthesis of 22.*** A mixture of **2007-C** (40 mg, 0.09 mmol) in MeOH (6 mL) was treated with Raney-Ni (20 mg) and stirred at room temperature for 30 min under a H₂ atmosphere. The Raney-Ni was then removed by filtration through Celite, the filtrate was concentrated and the residue was purified by Prep-TLC (DCM : MeOH = 10 : 1) to give **22** (18 mg, 48%) as a pink solid. MS 413.2 [M + H]⁺.

### Example 8 (reference example)

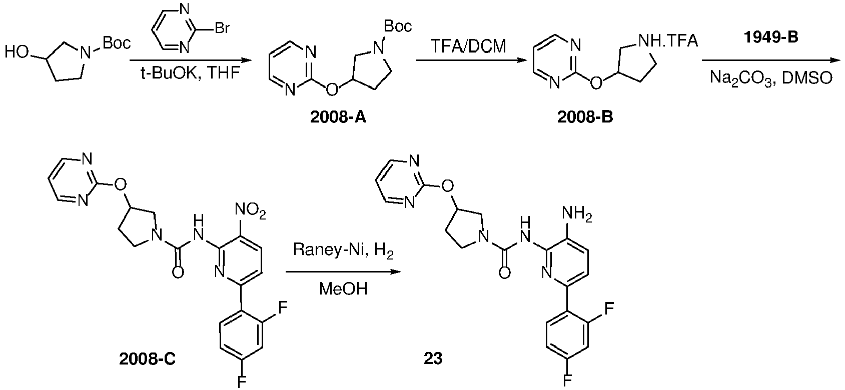

***Synthesis of 2008-A.*** A solution of tert-butyl 3-hydroxypyrrolidine-1-carboxylate (375 mg, 3.32 mmol) in THF (10 mL) was treated with 2-bromopyrimidine (350 mg, 3.22 mmol) and t-BuOK (1.08 g, 9.66 mmol) at room temperature. The reaction mixture was then heated to 70 °C, and was stirred at 70 °C for 3 h. The mixture was then diluted with water (30 ml), and extracted with EtOAc (20 mL × 3). The combined organic layers were washed with brine (20 mL × 3), dried over anhydrous Na₂SO₄ and then concentrated *in vacuo.* The residue was purified by Prep-TLC (EtOAc : PE = 10 : 1) to give **2008-A** (400 mg, 48%) as a colorless oil. MS 288.2 [M + H]⁺.

***Synthesis of 2008-B.*** To a solution of **2008-A** (400 mg, 1.51 mmol) in DCM (6 mL) was added TFA (2 mL) dropwise. The resulting reaction mixture was stirred at room temperature for 1 h, whereupon the solvent was removed *in vacuo* to give **2008-B** as a crude product which was used directly in the next step without further purification. MS 188.2 [M + H]⁺.

***Synthesis of 2008-C.*** A mixture of **1949-B** (370.0 mg, 0.76 mmol) and **2008-B** (1.51 mmol, crude product from last step) in DMSO (10 mL) was stirred at room temperature for 10 min, then was treated with Na₂CO₃ (800 mg, 7.55 mmol). The resulting reaction mixture was stirred at room temperature for 2 h, whereupon the mixture was diluted with water (30 mL) and extracted with EtOAc (30 mL × 3). The combined organic layers were washed with brine (30 mL × 3), dried over anhydrous Na₂SO₄ and then concentrated *in vacuo.* The residue was purified by Prep-TLC (EtOAc : PE = 5: 1) to give **2008-C** (250 mg, 75%) as a yellow solid. MS 443.2 [M + H]⁺.

***Synthesis of 23.*** A mixture of **2008-C** (200 mg, 0.45 mmol) in MeOH (8 mL) was treated with Pd/C (200 mg), and the reaction mixture was stirred at room temperature for 1 h under a H₂ atmosphere. The Pd/C was then removed by filtration through Celite, the filtrate was concentrated and the residue was purified by Prep-TLC (DCM : MeOH = 10 : 1) to give **23** (84 mg, 42%) as an off-white solid. MS 413.2 [M + H]⁺.

### Example 9

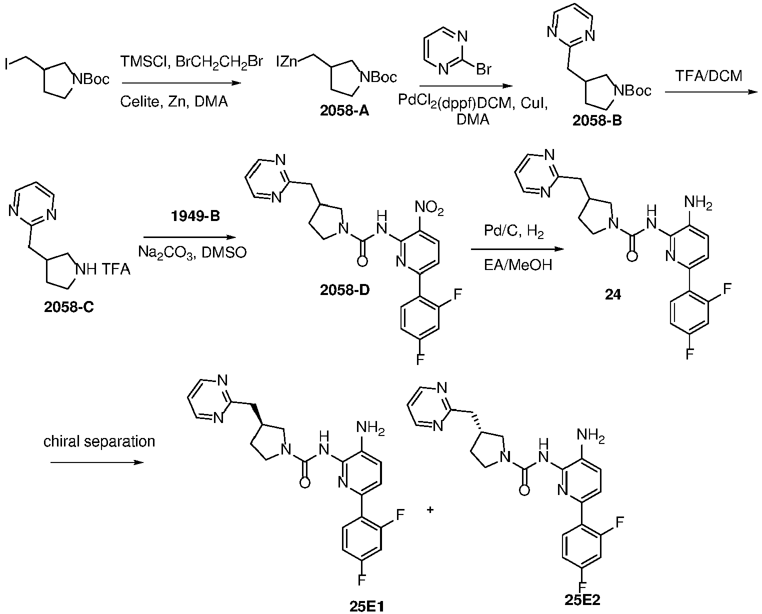

***Synthesis of 2058-A**.* A mixture of zinc dust (896 mg, 13.8 mmol) and anhydrous DMA (3 mL) was treated with TMSCl and 1,2-dibromoethane (0.24 mL, v/v=7/5), and the resulting reaction mixture was stirred at room temperature for 20 min under a N₂ atmosphere. A solution of tert-butyl 3-(iodomethyl)pyrrolidine-1-carboxylate (3.3 g, 10.6 mmol) in anhydrous DMA (4 mL) was then added, and the resulting reaction mixture was stirred at room temperature for 16 h under a N₂ atmosphere. The mixture was then used directly in the next step as **2058-A.** The concentration of **2058-A** was about 1.0 mol/L in DMA.

***Synthesis of 2058-B.*** A mixture of 2-bromopyrimidine (734 mg, 4.61 mmol), CuI (87 mg, 0.46 mmol) and Pd(PPh₃)₄ (266 mg, 0.23 mmol) in anhydrous DMA (15 mL) under a N₂ atmosphere was treated with **2058-A** (6.0 mL). The resulting mixture was stirred at 60 °C for 48 h under a N₂ atmosphere. The mixture was then diluted with water (50 mL) and extracted with EtOAc (30 mL × 3). The combined organic layers were washed with brine (30 mL × 3), dried over anhydrous Na₂SO₄ and then concentrated *in vacuo.* The residue was purified by Prep-TLC (EtOAc : DCM = 1 : 1) to give **2058-B** (500 mg, 41%) as a yellow solid. MS 264.2 [M + H]⁺.

***Synthesis of 2058-C.*** To a solution of **2058-B** (500 mg, 1.9 mmol) in DCM (10 mL) was added TFA (3 mL) dropwise. The reaction mixture was stirred at room temperature for 1 h, whereupon the solution was concentrated *in vacuo* to give **2058-C** as a crude product which was used directly in the next step without further purification. MS 164.2 [M + H]⁺.

***Synthesis of 2058-D.*** A mixture of **2058-C** (1.9 mmol, crude product from last step) and **1949-B** (518 mg, 1.05 mmol) in DMSO (15 mL) was stirred at room temperature for 10 min, then was treated with Na₂CO₃ (1.11 g, 10.5 mmol), and the resulting reaction mixture was stirred at room temperature for 2 h. The mixture was then diluted with water (50 mL) and extracted with EtOAc (30 mL × 3). The combined organic layers were washed with brine (30 mL × 3), dried over anhydrous Na₂SO₄ and then concentrated *in vacuo.* The residue was purified by Prep-TLC (DCM : EtOAc = 1 : 2) to give **2058-D** (400 mg, 86%) as a yellow solid. MS 441.2 [M + H]⁺.

***Synthesis of 24.*** A mixture of **2058-D** (400 mg, 0.91 mmol) and Pd/C (400 mg) in MeOH/EtOAc (10 mL/10 mL) was stirred at room temperature for 1 h under a H₂ atmosphere. The Pd/C was removed by filtration through Celite, the filtrate was concentrated *in vacuo* and the residue was purified by Prep-TLC (DCM : MeOH = 25 : 1) to give **24** (250 mg, 67%) as a yellow solid. MS 411.2 [M + H]⁺.

***Chiral-separation of 24.*** The enantiomers of **24** (250 mg, 0.61 mmol) were separated by chiral chromatographic separation (Column: Chiralpak AD-3; Solvent: MeOH; Flow rate: 2 mL/min; RT_{24E1} = 2.893 min, RT_{24E2} = 3.892 min) to give **Enantiomer** 1 **(25E1)** (62 mg, 25%) as a yellow solid (MS 411.2 [M+H]⁺) and **Enantiomer 2 (25E2)** (90 mg, 36%) as a yellow solid. MS 411.2 [M+H]⁺. Stereochemistry was randomly assigned.

**Compounds 26-28** were synthesized in a similar manner using the appropriately substituted bromine variants of the reagent used to synthesize **23.**

***Compound 26.*** 20 mg, 21%, a yellow solid.

***Compound 27.*** 110 mg, 59%, a white solid.

***Compound 28.*** 20 mg, 54%, a light yellow solid.

### Example 10 (reference example)

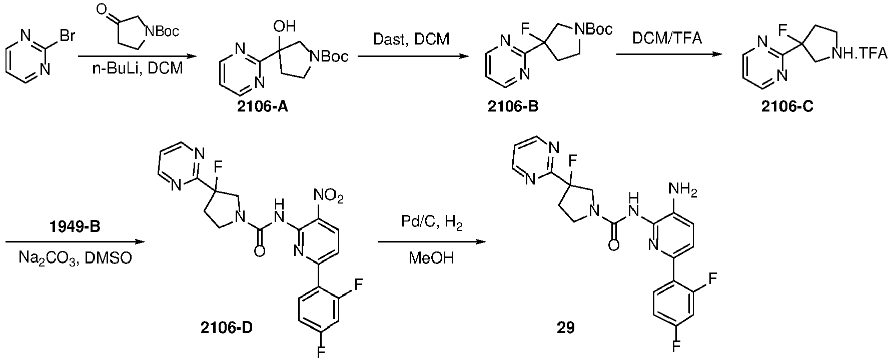

***Synthesis of 2106-A.*** To a solution of 2-bromopyrimidine (50.0 g, 314.5 mmol) in DCM (600 mL) under nitrogen atmosphere was added *n*-BuLi (150 mL, 377.5 mmol) dropwise at -78 °C and stirred at -78 °C for 2 h under nitrogen atmosphere. Then *tert*-butyl 3-oxopyrrolidine-1-carboxylate (70 g, 377.5 mmol) in DCM (200 mL) was added into above mixture dropwise at -78 °C. The resulting mixture was warmed to room temperature for 3 h. The mixture was quenched with saturated NH₄Cl (200 mL), extracted with DCM (400 mL × 3). The combined organic layers were washed with brine (200 mL × 3), dried over anhydrous Na₂SO₄ and then concentrated *in vacuo.* The residue was purified by column chromatography on silica gel (PE : EtOAc = 10 : 1 to EtOAc) to give **2106-A** (9.0 g, 11%) as a light yellow solid. MS 266.2 [M+H]⁺.

***Synthesis of 2106-B.*** To a solution of **2106-A** (9.0 g, 34.0 mmol) in DCM (50 mL) was added DAST (18 mL) dropwise at -78 °C and the solution was warmed to room temperature for 1 h under nitrogen atmosphere. The solvent was concentrated and the residue was purified by column chromatography on silica gel (PE : EtOAc = 10 : 1 to EtOAc) to give **2106-B** (2.2 g, 24%) as a brown solid. MS 268.2 [M+H]⁺.

***Synthesis of 2106-C.*** To a solution of **2106-B** (2.2 g, 8.21 mmol) in DCM (20 mL) was added TFA (8 mL) dropwise at 0 °C. Then the solution was stirred at room temperature for 1 h. The solvent was removed *in vacuo* to give **2106-C** as a crude product which was directly used in the next step. MS 168.2 [M+H]⁺.

***Synthesis of 2106-D.*** A mixture of **1949-B** (2.6 g, 5.47 mmol) and **2106-C** (8.21 mmol, crude product from last step) in DMSO (40 mL) was stirred at room temperature for 10 min, and then Na₂CO₃ (5.8 g, 54.7 mmol) was added into above mixture. The resulting mixture continued to stir at room temperature for 2 h. The mixture was diluted with water (200 mL), extracted with EtOAc (100 mL × 3). The combined organic layers were washed with brine (50 mL × 3), dried over anhydrous Na₂SO₄ and then concentrated *in vacuo.* The residue was purified by column chromatography on silica gel (PE : EtOAc = 10 : 1 to EtOAc) to give **2106-D** (2.0 g, 82%) as a yellow solid. MS 445.0 [M+H]⁺.

***Synthesis of 29.*** A mixture of **2106-D** (12.0 g, 27.0 mmol) and Raney-Ni (2.0 g) in MeOH (20 mL) was stirred at room temperature for 1 h under H₂ atmosphere. Raney-Ni was removed by filtration through a pad of Celite. The filtrate was concentrated in *vacuo* and the residue was purified by column chromatography on silica gel (PE : EtOAc = 10 : 1 to EtOAc) to give **29** (8.0 g, 71%) as a yellow solid. MS 415.2 [M+H]⁺.

***Chiral-separation of 29-E1 and 29-E2.*** The enantiomers of Compound **29** (8.0 g, 19.3 mmol) were separated by chiral SFC (Column: Chiralcel OX-3; Solvent: MeOH; Flow rate: 1.5 mL/min; RT_{2106-E1} = 2.814 min, RT_{T-2106-E2} = 4.362 min) to give **29-E1** (1.2 g, 11%) as a yellow solid (MS 415.2 [M+H]⁺) and **29-E2** (1.3 g, 12%) as a yellow solid. MS 415.2 [M+H]⁺.

**Compound 45** was synthesized in a similar manner as **29** by using 2-(5-fluorothiophen-2-yl)-4,4,5,5-tetramethyl-1,3,2-dioxaborolane as a reagent.

***Compound 45.*** 90 mg, 19%, a yellow solid.

### Example 11

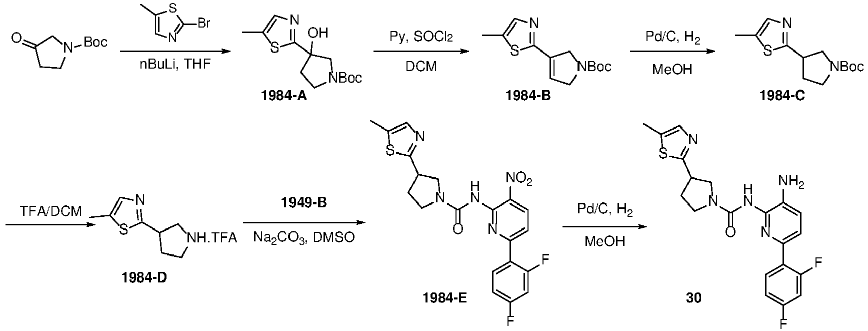

***Synthesis of 1984-A.*** A solution of 2-bromo-5-methylthiazole (1.0 g, 5.59 mmol) in THF (20 mL) under a N₂ atmosphere was treated with nBuLi (2.7 mL, 6.70 mmol) dropwise at -78°C, and the resulting reaction mixture was stirred at -78°C for 1 h. A solution of tert-butyl 3-oxopyrrolidine-1-carboxylate (1.2 g, 6.70 mmol) in THF (10 mL) was then added to the reaction mixture dropwise at -78 °C. The reaction mixture was then allowed to warm to room temperature, and was stirred at room temperature for 3 h. The mixture was diluted with saturated aqueous NH₄Cl (40 mL), and extracted with EtOAc (30 mL × 3). The combined organic layers were washed with brine (20 mL × 3), dried over anhydrous Na₂SO₄ and then concentrated *in vacuo.* The residue was purified by column chromatography on silica gel (PE : EtOAc = 10 : 1 to EtOAc) to give **1984-A** (760 mg, 48%) as a light yellow solid. MS 285.2 [M+H]⁺.

***Synthesis of 1984-B.*** A solution of **1984-A** (660 mg, 2.32 mmol) in DCM (10 mL) was cooled to 0 °C and treated with pyridine (1.09 g, 13.94 mmol), followed by dropwise addition of SOCl₂ (414 mg, 3.48 mmol). The resulting reaction mixture was then heated to 45 °C and stirred at 45 °C for 16 h. The mixture was then diluted with water (20 ml), and extracted with DCM (30 mL × 3). The combined organic layers were washed with brine (20 mL × 3), dried over anhydrous Na₂SO₄ and then concentrated *in vacuo.* The residue was purified by column chromatography on silica gel (PE : EtOAc = 10 : 1 ~ 1: 5) to give **1984-B** (120 mg, 19%) as a brown oil. MS 266.2 [M+H]⁺.

***Synthesis of 1984-C**.* A mixture of **1984-B** (100 mg, 0.38 mmol) and Pd/C (100 mg) in MeOH (6 mL) was stirred at room temperature for 1 h under a H₂ atmosphere. The Pd/C was then removed by filtration through Celite, the filtrate was concentrated and the residue was purified by Prep-TLC (EtOAc : PE = 5 : 1) to give **1984-C** (90 mg, 88%) as a light brown oil. MS 269.2 [M+H]⁺.

***Synthesis of 1984-D.*** To a solution of **1984-C** (90 mg, 0.34 mmol) in DCM (3 mL) was added TFA (1 mL) dropwise. The resulting reaction mixture was stirred at room temperature for 1 h, whereupon the solvent was removed *in vacuo* to give **1984-D** as a crude product which was used directly in the next step without further purification. MS 169.2 [M+H]⁺.

***Synthesis of 1984-E.*** A mixture of **1949-B** (93 mg, 0.19 mmol) and **1984-D** (0.34 mmol, crude product from last step) in DMSO (5 mL) was treated with Na₂CO₃ (200 mg, 1.89 mmol), and the resulting reaction mixture was stirred at room temperature for 2 h. The mixture was then diluted with water (20 mL), and extracted with EtOAc (20 mL × 3). The combined organic layers were washed with brine (20 mL × 3), dried over anhydrous Na₂SO₄ and then concentrated *in vacuo.* The residue was purified by Prep-TLC (EtOAc : PE = 5: 1) to give **1984-E** (80 mg, 95%) as a yellow solid. MS 446.2 [M+H]⁺.

***Synthesis of 30.*** A mixture of **1984-E** (80 mg, 0.18 mmol) and Pd/C (80 mg) in MeOH (5 mL) was stirred at room temperature for 1 h under a H₂ atmosphere. The Pd/C was then removed by filtration through Celite, the filtrate was concentrated and the residue was purified by Prep-TLC (EtOAc : MeOH = 15 : 1) to give **30** (44 mg, 59%) as a yellow solid. MS 416.2 [M+H]⁺.

### Example 12 Synthesis of 31

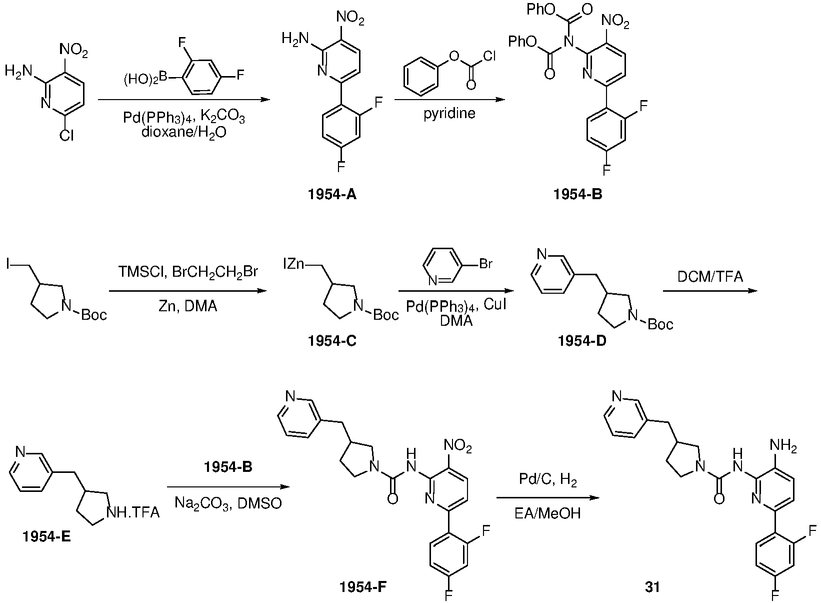

***Synthesis of 1954-A.*** A mixture of 6-chloro-3-nitropyridin-2-amine (4.58 g, 26.4 mmol), 2,4-difluorophenylboronic acid (5.00 g, 31.7 mmol) and K₂CO₃ (10.9 g, 79.2 mmol) in dioxane/H₂O (100 mL/10 mL) was treated with Pd(PPh₃)₄(1.10 g, 0.95 mmol) under a nitrogen atmosphere. The mixture was stirred at 100 °C for 3 h and then concentrated *in vacuo.* The residue was dissolved with EtOAc (200 mL) and the solution was washed with brine (100 mL × 3). The organic layer was dried over anhydrous Na₂SO₄ and then concentrated *in vacuo.* The residue was purified by column chromatography on silica gel (PE : EtOAc = 7 : 1 to 5 : 1) to give **1954-A** (4.0 g, 61%) as a yellow solid. MS 252.1 [M + H]⁺.

***Synthesis of 1954-B.*** To a stirred solution of **1954-A** (4.0 g, 15.94 mmol) in pyridine (60 mL) was added phenyl carbonochloridate (7.50 g, 47.81 mmol) dropwise at 0 °C. After the addition was completed, the mixture was stirred at 50 °C for 4 h. The mixture was concentrated *in vacuo.* The residue was purified by column chromatography on silica gel (PE : DCM = 3: 2 to 1 : 1) to give **1954-B** (7.1 g, 91%) as a yellow solid. MS 492.1 [M + H]⁺.

***Synthesis of 1954-C.*** To a mixture of zinc dust (449 mg, 6.9 mmol) in anhydrous DMA (2 mL) was added TMSC1 and 1,2-dibromoethane (0.24 mL, v/v=7/5), and the reaction mixture was stirred at room temperature for 20 min under a nitrogen atmosphere. Then a solution of tert-butyl 3-(iodomethyl)pyrrolidine-1-carboxylate (1.65 g, 5.3 mmol) in anhydrous DMA (1.5 mL) was added into above mixture, and the resulting mixture was stirred at room temperature for 16 h under a nitrogen atmosphere. The mixture was used directly in the next step as **1954-C.** The concentration of **1954-C** was about 1.0 mol/L in DMA.

***Synthesis of 1954-D.*** To a mixture of 3-bromopyrimidine (243 mg, 1.54 mmol), CuI (30 mg, 0.15 mmol) and Pd(PPh₃)₄ (89 mg, 0.077 mmol) in anhydrous DMA (6 mL) under nitrogen atmosphere was added **1954-C** (2.0 mL). The resulting mixture was stirred at 60 °C for 72 h under a nitrogen atmosphere. The mixture was then diluted with water (20 mL) and extracted with EtOAc (20 mL × 3). The combined organic layers were washed with brine (20 mL × 3), dried over anhydrous Na₂SO₄ and then concentrated *in vacuo.* The residue was purified by Prep-TLC (DCM :EtOAc = 2 : 1) to give **1954-D** (180 mg, 44%) as a yellow solid. MS 263.2 [M + H]⁺.

***Synthesis of 1954-E.*** To a solution of **1954-D** (160 mg, 0.69 mmol) in DCM (6 mL) was added TFA (2 mL) dropwise at 0°C. The resulting reaction mixture was stirred at room temperature for 1 h, then was concentrated *in vacuo* to give **1954-E** as a crude product which was directly used in the next step. MS 163.2 [M + H]⁺.

***Synthesis of 1954-F.*** A mixture of **1954-E** (0.69 mmol, crude product from previous step) and **1954-B** (188 mg, 0.38 mmol) in DMSO (6 mL) was stirred at room temperature for 10 min, then Na₂CO₃ (403 mg, 3.8 mmol) was added into above mixture and the resulting reaction mixture was stirred at room temperature for 2 h. The mixture was then diluted with water (30 mL) and extracted with EtOAc (10 mL × 3). The combined organic layers were washed with brine (10 mL × 3), dried over anhydrous Na₂SO₄ and then concentrated *in vacuo.* The residue was purified by Prep-TLC (DCM : EtOAc = 1 : 2) to give **1954-F** (110 mg, 66%) as a yellow solid. MS 440.1 [M + H]⁺.

***Synthesis of Compound 31.*** A mixture of **1954-F** (110 mg, 0.25 mmol) and Pd/C (110 mg) in MeOH/EtOAc (5 mL/5 mL) was stirred at room temperature for 50 min under a H₂ atmosphere. Pd/C was removed by filtration through a pad of Celite. The filtrate was concentrated *in vacuo* and the residue was purified by Prep-TLC (DCM : MeOH = 30 : 1) to give **31** (45 mg, 44%) as a yellow solid. MS 410.1 [M + H]⁺.

**Compound 33** was synthesized in a similar manner as **31** by using *tert*-butyl 3-iodopyrrolidine-1-carboxylate and 2-bromo-5-methylpyrimidine as reagents.

***Compound 33.*** 38 mg, 41%, a light yellow solid.

**Compound 48** was synthesized in a similar manner as **31** by using *tert*-butyl 3-iodopyrrolidine-1-carboxylate, 2-bromopyrimidine and 2-fluorophenylboronic acid as reagents.

***Compound 48.*** 38 mg, 41%, a light yellow solid.

### Example 13 Synthesis of Compound 32

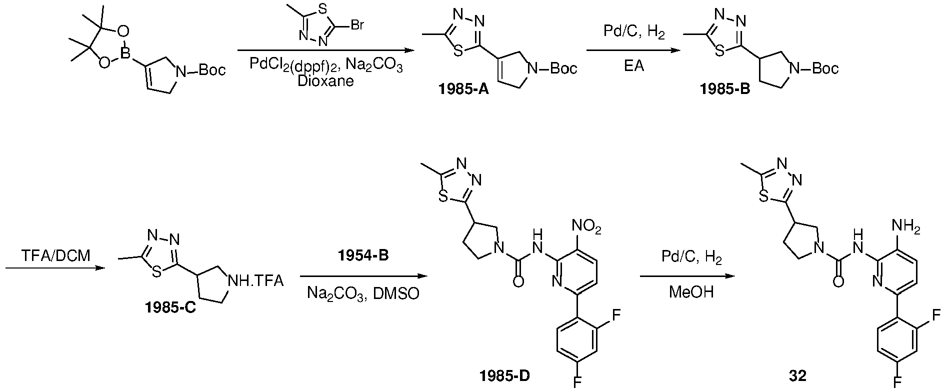

***Synthesis of 1985-A.*** A mixture of 2-bromo-5-methyl-1, 3, 4-thiadiazole (700 mg, 3.89 mmol), *tert*-butyl 3-(4,4,5,5-tetramethyl-1,3,2-dioxaborolan-2-yl)-2,5-dihydro-1H-pyrrole-1-carboxylate (1.15 g, 3.89 mmol) and Na₂CO₃ (1.2 g, 11.7 mmol) in dioxane (40 mL) was treated with PdCl2(dppf)₂(159 mg, 0.2 mmol) under a nitrogen atmosphere. The reaction mixture was stirred at 90 °C for 3 h and then concentrated *in vacuo.* The residue was dissolved with EtOAc (30 mL) and the solution was washed with brine (10 mL × 3). The combined organic layer was dried over anhydrous Na₂SO₄ and then concentrated *in vacuo.* The residue was purified by column chromatography on silica gel (PE : EtOAc = 10 : 1 to 1 : 1) to give **1985-A** (400 mg, 39%) as a yellow solid. MS 268.1 [M + H]⁺.

***Synthesis of 1985-B*** A mixture of **1985-A** (400 mg, 1.5 mmol) and Pd/C (400 mg) in EtOAc (10 mL) was stirred at room temperature for 1 h under a H₂ atmosphere. Pd/C was then removed by filtration through a pad of Celite. The filtrate was concentrated and the residue was purified by Prep-TLC (EA: PE = 3 : 1) to give **1985-B** (300 mg, 74%) as yellow solid. MS 270.2 [M+H]⁺.

***Synthesis of 1985-C.*** A solution of **1985-B** (300 mg, 1.1 mmol) in DCM (10 mL) was cooled to 0 °C and then TFA (4 mL) was added dropwise at 0 °C. The resulting solution was stirred at room temperature for 1 h, whereupon the solvent was removed *in vacuo* to give 1985-C as a crude product which was used directly in the next step. MS 170.2 [M+H]⁺.

***Synthesis of 1985-D.*** A mixture of **1954-B** (300 mg, 0.6 mmol) and **1985-C** (1.1 mmol, crude product from previous step) in DMSO (10 mL) was treated with Na₂CO₃ (636 mg, 6.0 mmol) and the resulting mixture was stirred at room temperature for 2 h. The mixture was then diluted with water (50 mL), extracted with EtOAc (50 mL × 3). The combined combined organic layers were washed with brine (20 mL × 3), dried over anhydrous Na₂SO₄ and then concentrated *in vacuo.* The residue was purified by Prep-TLC (EA : PE = 5 : 1) to give **1985-D** (150 mg, 56%) as a yellow solid. MS 447.2 [M+H]⁺.

***Synthesis of 32*** A mixture of **1985-D** (150 mg, 0.34 mmol) and Pd/C (150 mg) in MeOH (6 mL) was stirred at room temperature for 1 h under a H₂ atmosphere. Pd/C was then removed by filtration through a pad of Celite. The filtrate was concentrated and the residue was purified by Prep-TLC (EA: MeOH = 15 : 1) to give **32** (83 mg, 55%) as yellow solid. MS 417.2 [M+H]⁺.

**Compound 37** was synthesized in a similar manner as **32** by using the appropriately substituted aryl bromide reagent. ***Compound 37.*** 65 mg, 58%, a light yellow solid.

### Example 14 Synthesis of Compound 34

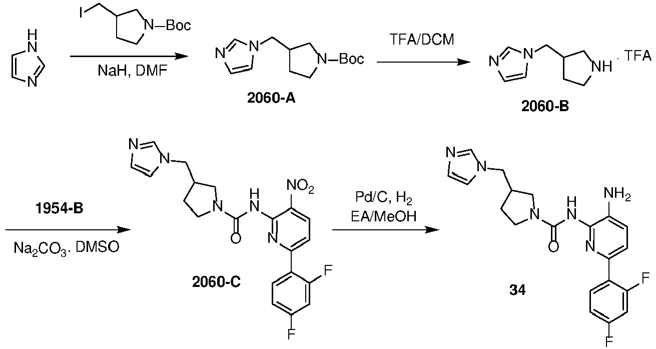

***Synthesis of 2060-A.*** A solution of 1*H*-imidazole (115 mg, 1.69 mmol) in DMF (5 mL) was cooled to 0 °C and treated with NaH (60% in mineral oil, 122 mg, 3.1 mmol). The reaction mixture was stirred at 0 °C for 10 min, then *tert*-butyl 3-(iodomethyl)pyrrolidine-1-carboxylate (687 mg, 2.21 mmol) was added and the reaction mixture was warmed to 40 °C and stirred at 40 °C for 3 h. The mixture was thendiluted with water (30 mL) and extracted with EtOAc (20 mL × 3). The combined organic layers were washed with brine (20 mL × 3), dried over anhydrous Na₂SO₄ and then concentrated *in vacuo.* The residue was purified by Prep-TLC (DCM : MeOH = 30 : 1) to give **2060-A** (105 mg, 25%) as a colorless oil. MS 197.2 [M + H]⁺.

***Synthesis of 2060-B.*** To a solution of **2060-A** (201 mg, 0.80 mmol) in DCM (6 mL) was added TFA (2 mL) dropwise at 0°C. The resulting reaction mixture was allowed to warm to room temperature and was stirred at room temperature for 1 h, then was concentrated *in vacuo* to give **2060-B** as a crude product which was directly used in the next step. MS 151.2 [M + H]⁺.

***Synthesis of 2060-C**.* A mixture of **2060-B** (0.80 mmol, crude product from previous step) and **1954-B** (216 mg, 0.44 mmol) in DMSO (6 mL) was stirred at room temperature for 10 min, then Na₂CO₃ (471 mg, 4.44 mmol) was added into above mixture and stirred at room temperature for 2 h. The mixture was diluted with water (20 mL) and extracted with EtOAc (10 mL × 3). The combined organic layers were washed with brine (10 mL × 3), dried over anhydrous Na₂SO₄ and then concentrated *in vacuo.* The residue was purified by Prep-TLC (DCM : MeOH = 30 : 1) to give **2060-C** (147 mg, 78%) as a yellow solid. MS 429.1 [M + H]⁺.

***Synthesis of 34.*** A mixture of **2060-C** (124 mg, 0.29 mmol) and Pd/C (124 mg) in MeOH/EtOAc (5 mL/5 mL) was stirred at room temperature for 2 h under a H₂ atmosphere. Pd/C was removed by filtration through a pad of Celite. The filtrate was concentrated *in vacuo* and the residue was purified by Prep-TLC (DCM : MeOH = 20 : 1) to give **34** (53 mg, 43%) as a white solid. MS 399.2. [M + H]⁺.

**Compounds 35** was synthesized in a similar manner as **34** by using pyrazole as a reagent.

***Compound 35.*** 60 mg, 64%, a white solid.

### Example 16 Synthesis of Compound 41 (reference example)

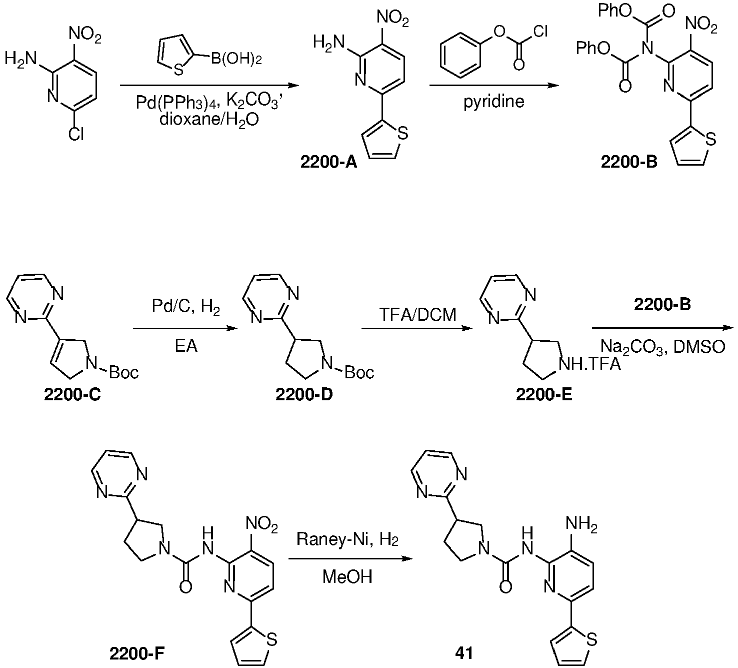

***Synthesis of 2200-A.*** To a mixture of thiophen-2-ylboronic acid (14.1 g, 110 mmol), 6-chloro-3-nitropyridin-2-amine (17.3 g, 100 mmol) and K₂CO₃ (41.4 g, 300 mmol) in dioxane/H₂O (500 mL/50 mL) was added Pd(PPh₃)₄ (5.8 g, 5.0 mmol) under a nitrogen atmosphere. The reaction mixture was stirred at 100 °C for 2 h and then concentrated *in vacuo.* The residue was dissolved with EtOAc (200 mL) and the solution was washed with brine (100 mL × 3). The organic layer was dried over anhydrous Na₂SO₄ and then concentrated *in vacuo.* The residue was purified by column chromatography on silica gel (PE : EtOAc = 10: 1 to 5 : 1) to give **2200-A** (20.4 g, 84 %) as a yellow solid. MS 222.0 [M + H]⁺.

***Synthesis of 2200-B.*** To a stirred solution of **2200-A** (4.42 g, 20 mmol) in pyridine (80 mL) was added phenyl carbonochloridate (3.12 g, 60 mmol) dropwise at 0 °C. After the addition was completed, the mixture was stirred at 50 °C for 4 h. The mixture was then concentrated *in vacuo,* and the residue was purified by column chromatography on silica gel (PE : DCM = 3: 2 to 1 : 1) to give **2200-B** (8.57 g, 93%) as a yellow solid. MS 462.1 [M + H]⁺.

***Synthesis of 2200-D.*** A mixture of **2200-C** (108 mg, 0.44 mmol) and Pd/C (108 mg) in EtOAc (15 mL) was stirred at room temperature for 1 h under a H₂ atmosphere. Pd/C was removed by filtration through a pad of Celite. The filtrate was concentrated *in vacuo* and the residue was purified by Prep-TLC (EtOAc : PE = 1 : 5) to give **41** (100 mg, 92%) as a white solid. MS 250.1 [M + H]⁺.

***Synthesis of 2200-E.*** To a solution of **2200-D** (100 mg, 0.40 mmol) in DCM (3 mL) was added TFA (1 mL) dropwise at °C. The resulting solution was stirred at room temperature for 1 h, and then the solvent was removed *in vacuo* to give **2200-E** as a crude product which was used directly in the next step. MS 194.1 [M + H]⁺.

***Synthesis of 2200-F.*** A mixture of **2200-E** (0.4 mmol, crude product from previous step) and **2200-B** (103 mg, 0.22 mmol) in DMSO (6 mL) was stirred at room temperature for 10 min, then Na₂CO₃ (234 mg, 2.2 mmol) was added and the reaction mixture was stirred at room temperature for 2 h. The mixture was diluted with water (30 mL) and extracted with EtOAc (10 mL × 3). The combined organic layers were washed with brine (10 mL × 3), dried over anhydrous Na₂SO₄ and then concentrated *in vacuo.* The residue was purified by Prep-TLC (DCM : EtOAc = 1 : 1) to give **2200-F** (80 mg, 91%) as a yellow solid. MS 397.0 [M + H]⁺.

***Synthesis of 41.*** A mixture of **2200-F** (80 mg, 0.20 mmol) and Raney-Ni (80 mg) in MeOH (15 mL) was stirred at room temperature for 1 h under a H₂ atmosphere. Raney-Ni was removed by filtration through a pad of Celite. The filtrate was concentrated *in vacuo* and the residue was purified by Prep-TLC (DCM : MeOH = 20 : 1) to give **41** (43 mg, 58%) as a brown solid. MS 367.2 [M + H]⁺.

**Compound 42** was synthesized in a similar manner as **41** by using methyl magnesium bromide and appropriately substituted boronic acid reagent.

***Compound 42.*** 23 mg, 31%, a yellow solid.

### Example 17 Synthesis of 43 and 44 (reference example)

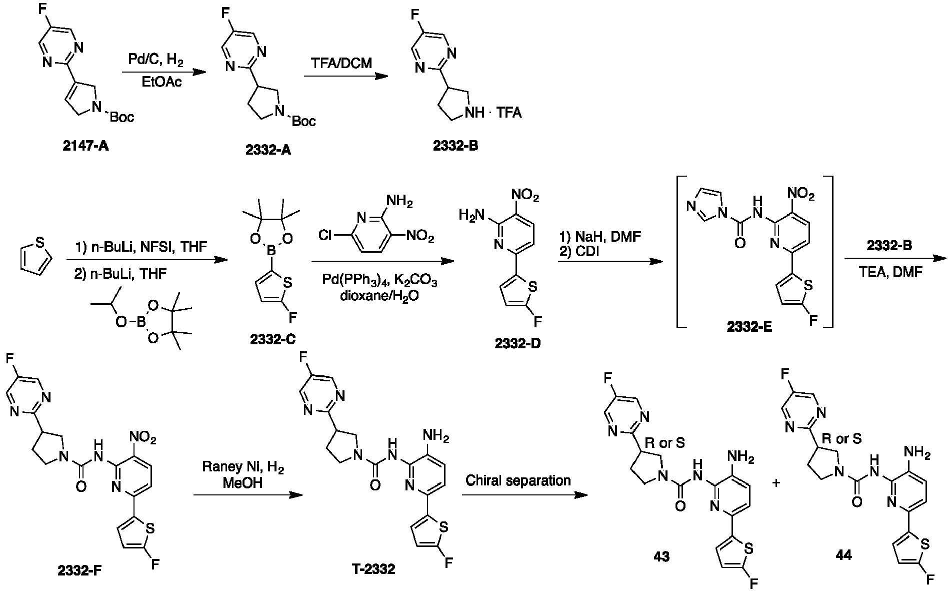

***Synthesis of 2332-A.*** A mixture of **2147-A** (1.0 g, 3.8 mmol) and Pd/C (1.0 g) in EtOAc (20 mL) was stirred at room temperature for 1 h under a H₂ atmosphere. Pd/C was removed by filtration through a pad of Celite. The filtrate was concentrated *in vacuo* and the residue was purified by column chromatography on silica gel (PE : EtOAc =5 : 1 to 3 : 1) to give **2332-A** (1.0 g, 99%) as a white solid. MS 268.1 [M + H]⁺.

***Synthesis of 2332-B.*** A solution of **2332-A** (1.0 g, 3.7 mmol) in DCM (21 mL) was cooled to 0 °C and TFA (7 mL) was added dropwise at 0 °C. The reaction mixture was allowed to warm to room temperature and was stirred at room temperature for 1 h. The solvent was then removed *in vacuo,* and the residue was dissolved in DMF (7 mL) and treated with TEA (1.01 g, 10 mmol) to give **2332-B** as a solution used to next step directly. MS 168.1 [M + H]⁺.

***Synthesis of 2332-C.*** To a solution of thiophene (20.0 g, 238 mmol) in THF (500 mL) under nitrogen atmosphere was added *n*-BuLi (100 mL, 250 mmol) dropwise at -78 °C and the reactin was stirred at -78 °C for 1 h under a nitrogen atmosphere. Then *N-*fluorobenzenesulfonimide (78.8 g, 250 mmol) in THF (300 mL) was added into above mixture dropwise at -78 °C and warmed to room temperature for 1 h. Then the reaction mixture was cooled to -78 °C, and another portion of *n*-BuLi (100 mL, 250 mmol) was added dropwise at -78 °C and stirred at -78 °C for 1 h. Finally, 2-isopropoxy-4,4,5,5-tetramethyl-1,3,2-dioxaborolane (46.5 g, 250 mmol) in THF (200 mL) was added into above mixture dropwise at -78 °C, and the reaction mixture was allowed to warm to room temperature and stirred for 16 h. The mixture was poured into cooled saturated NH₄Cl (2000 mL), extracted with PE (400 mL × 3), and the combined organic layers were washed with brine (400 mL × 3), dried over anhydrous Na₂SO₄ and then concentrated *in vacuo* to give **2332-C** (32 g) as a crude product used to next step directly. MS 229.0 [M+H]⁺.

***Synthesis of 2332-D.*** A mixture of 6-chloro-3-nitropyridin-2-amine (18.9 g, 109.6 mmol), **2332-C** (30 g, crude product from previous step) and K₂CO₃ (45.37 g, 328.8 mmol) in dioxane/H₂O (400 mL/40 mL) was treated with Pd(PPh₃)₄ (2.0 g) under a nitrogen atmosphere. The mixture was stirred at 95 °C for 3 h and then concentrated *in vacuo.* The residue was dissolved with EtOAc (500 mL) and the solution was washed with brine (200 mL × 3). The organic layer was dried over anhydrous Na₂SO₄ and then concentrated *in vacuo.* The residue was purified by column chromatography on silica gel (PE : DCM = 10 : 1 to 2 : 1) to give **2332-D** (9.0 g, 34%(two steps)) as a yellow solid. MS 240.0 [M + H]⁺.

***Synthesis of 2332-F.*** A solution of **2332-D** (820 mg, 3.43 mmol) in DMF (10 mL) was cooled to 0 °C and treated with NaH (60% in mineral oil) (275 mg, 6.86 mmol). The reaction mixture was stirred at 0 °C for 30 min, then CDI (556 mg, 3.43 mmol) was added into above mixture and stirring continued at 0 °C for another 30 min. Finally, the solution of **2332-B** was added into above mixture at 0 °C and stirred for 1 h. The mixture was then quenched with water (60 mL) and extracted with EtOAc (30 mL × 3). The combined organic layers were washed with brine (30 mL × 3), dried over anhydrous Na₂SO₄ and then concentrated *in vacuo.* The residue was purified by column chromatography on silica gel (DCM : EtOAc = 15 : 1 to 2 : 1) to give **2332-F** (1.18 g, 80 %) as a yellow solid. MS 433.0 [M + H]⁺.

***Synthesis of T-2332.*** A mixture of **2332-F** (1.18 g, 2.7 mmol) and Raney-Ni (1.2 g) in MeOH (20 mL) was stirred at room temperature for 1 h under a H₂ atmosphere. Raney-Ni was removed by filtration through apad of Celite. The filtrate was concentrated *in vacuo* and the residue was purified by column chromatography on silica gel (DCM : MeOH = 50 : 1 to 20 : 1) to give **T-2332** (850 mg, 77%) as a red solid. MS 403.0 [M + H]⁺.

***Chiral-separation of 43 and 44.* T-2332** (850 mg, 2.11 mmol) was separated by chiral separation (Column: Chiralcel OJ-3; Solvent: MeOH; Flow rate: 2 mL/min; RT₄₃ = 2.141 min, RT₄₄ = 2.689 min) to give **43** (300 mg, 35%) as a light purple solid (MS 403.0 [M+H]⁺) and **44** (190 mg, 22%) as a white solid. MS 403.0 [M+H]⁺.

### Example 18 Synthesis of Compound 52 & 53 (reference example)

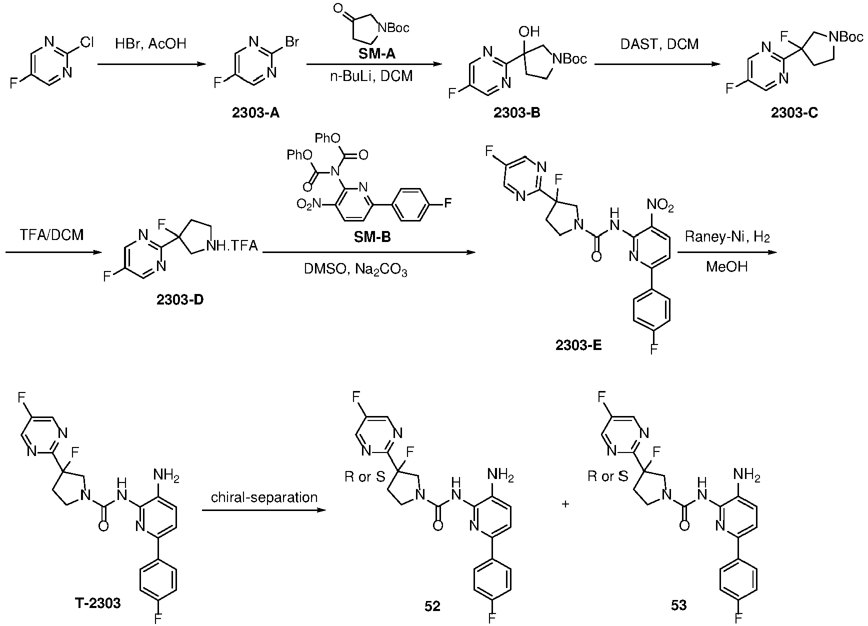

***Synthesis of 2303-A.*** 2-chloro-5-fluoropyrimidine (50 g, 378.0 mmol) was stirred in a solution of HBr in AcOH (33 wt %, 250 mL) at 40 °C for 16 h. The reaction mixture was then cooled to room temperature and the precipitate was collected by filtrate. The filter cake was dissolved in EtOAc (500 mL) and basified to pH = 9 with saturated Na₂CO₃. The resulting mixture was extracted with EtOAc (500 mL × 2). The combined organic layers were washed with brine (100 mL × 3), dried over anhydrous Na₂SO₄ and then concentrated *in vacuo.* PE (20 mL) was added into the residue and the precipitate was collected by filtration, then dried *in vacuo* to give **2303-A** (35.0 g, 53%) as a light brown solid. MS 177.2 [M+H]⁺.

***Synthesis of 2303-B.*** To a solution of **2303-A** (5.0 g, 28.4 mmol) in DCM (70 mL) was added n-BuLi (13.6 mL, 34.1 mmol) dropwise at -78 °C and the reaction mixture was stirred for 1 h under N₂ atmosphere. Then a solution of **SM-A** (6.3 g, 34.1 mmol) in DCM (20 mL) was added into the mixture dropwise. The resulting mixture was warmed to room temperature and stirred for 3 h. The mixture was then diluted with saturated NH₄Cl (100 mL), extracted with DCM (100 mL × 3). The combined organic layer was washed with brine (100 mL × 3), dried over anhydrous Na₂SO₄ and then concentrated *in vacuo.* The residue was purified by column chromatography on silica gel (PE : EtOAc = 10 : 1 to EtOAc) to give **2303-B** (550 mg) as a crude product. The crude product was purified by Prep-HPLC to give **2303-B** (177 mg, 2.2%) as a brown solid. MS 284.2 [M+H]⁺.

***Synthesis of 2303-C.*** A solution of **2303-B** (1.6 g, 5.63 mmol) in DCM (50 mL) was treated with DAST (3.2 mL) dropwise at -78 °C under a N₂ atmosphere. Then the solution was warmed to room temperature and stirred for 2 h. The reaction was then quenched with ice water, extracted with DCM (30 mL × 3). The combined organic layer was washed with brine (100 mL × 3), dried over anhydrous Na₂SO₄ and then concentrated in vacuo. The residue was purified by column chromatography on silica gel (PE : EtOAc = 10 : 1 ~ 1 : 1) to give **2303-C** (850 mg, 53%) as a brown solid. MS 286.2 [M+H]⁺.

***Synthesis of 2303-D.*** To a solution of **2303-C** (850 mg, 2.98 mmol) in DCM (30 mL) at 0 °C was added TFA (4 mL) dropwise. Then the solution was allowed to warm to room temperature and was stirred at room temperature for 1 h. The solvent was removed *in vacuo* to give **2303-D** as a crude product which was used directly in the next step.

***Synthesis of 2303-E.*** To a solution of **SM-B** (1.2 g, 2.48 mmol) and **2303-D** (1.1 g, crude product from last step) in DMSO (50 mL) was added Na₂CO₃ (3.2 g, 29.8 mmol). The mixture was stirred at room temperature for 2 h. The mixture was diluted with water (200 mL), extracted with EtOAc (100 mL × 3). The combined organic layers were washed with brine (100 mL × 3), dried over anhydrous Na₂SO₄ and then concentrated in vacuo. The residue was purified by Prep-TLC (PE : EtOAc = 1 : 5) to give **2303-E** (680 mg, 61%) as a yellow solid. MS 445.2 [M+H]⁺.

***Synthesis of 52 and 53.*** A mixture of **2303-E** (680 mg, 1.53 mmol) and Raney-Ni (680 mg) in MeOH (10 mL) was stirred at room temperature for 1 h under a H₂ atmosphere. Raney-Ni was then removed by filtration through the Celite. The filtrate was concentrated and the residue was purified by Prep-TLC (EA : MeOH = 10 : 1) to give **T-2303** (600 mg, 94%) as a yellow solid. The enantiomers were separated by chiral SFC (Column: Chiralcel OJ-3; Solvent: MeOH; Flow rate: 1.5 mL/min; RT₅₂ = 1.869 min, RT₅₃ = 2.848 min) to give **52** (250 mg, 41%) as a yellow solid . MS 415.2 [M+H]⁺. **53** (240 mg, 40%) as a yellow solid. MS 415.2 [M+H]⁺.

### Example 19 Synthesis of Compound 54 and 55 (reference example)

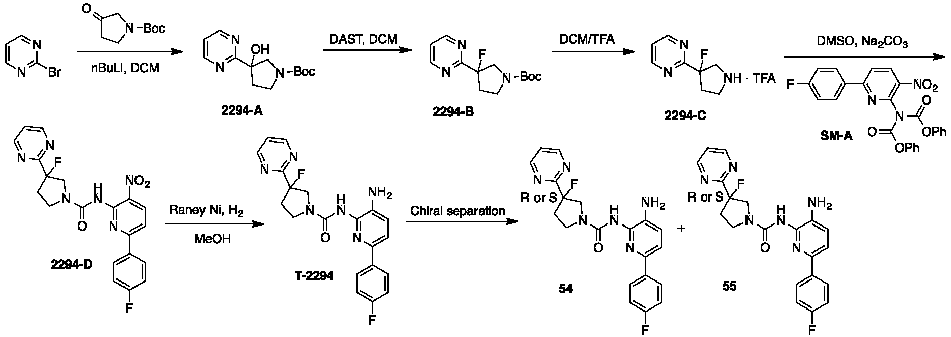

***Synthesis of 2294-A.*** To a solution of 3-bromopyridazine (50.0 g, 314.5 mmol) in DCM (400 mL) was added n-BuLi (2.5 M in hexane)(150 mL) dropwise at -78°C under a N₂ atmosphere and the reaction mixture was stirred at -78°C for 1 h. Then a solution of tert-butyl 3-oxopyrrolidine-1-carboxylate (69.7 g, 377.0 mmol) in DCM (200 mL) was added into the above mixture and the mixture was warmed to room temperature and stirred for 3 h. The reaction mixture was poured into saturated NH₄Cl (300 mL), then extracted with DCM (400 mL × 3), washed with brine (300 mL × 3). The organic layer was dried over anhydrous Na₂SO₄ and then concentrated *in vacuo.* The residue was purified by column chromatography on silica gel (PE : EtOAc = 10 : 1 to EtOAc) to give **2294-A** (7.0 g) as a crude product. MS 266.2 [M+H]⁺.

***Synthesis of 2294-B.*** A solution of **2294-A** (7.0 g, crude product from last step) in DCM (100 mL) was cooled to -78 °C and treated with DAST (3.0 mL) dropwise, and the reaction mixture was then allowed to warm to room temperature and stirred at room temperature for 2 h. The mixture was then diluted with water (100 mL), extracted with DCM (50 mL × 3). The combined organic layer was washed with brine (80 mL × 3), dried over anhydrous Na₂SO₄ and then concentrated *in vacuo.* The residue was purified by column chromatography on silica gel (EtOAc : PE = 10 : 1 ~ 1 : 1) to give **2294-B** (2.5 g, 3%)(two steps) as a brown solid. MS 268.2 [M+H]⁺.

***Synthesis of 2294-C.*** A solution of **2294-B** (2.5 g, 9.4 mmol) in DCM (24 mL) was cooled to 0 °C and treated with TFA (8 mL). The reaction mixture was allowed to warm to room temperature following the addition, and was stirred at room temperature for 1 h. The solution was concentrated *in vacuo* to give **2294-C** as a crude product which was used directly in the next step. MS 168.2 [M+H]⁺.

***Synthesis of 2294-D.*** A solution of **SM-A** (2.5 g, 5.3 mmol) and **2294-C** (9.4 mmol, crude product from last step) in DMSO (50 mL) was treated with Na₂CO₃ (5.5 g, 52.2 mmol). The reaction mixture was stirred at room temperature for 2 h. The mixture was then diluted with water (150 mL), extracted with EtOAc (100 mL × 3), and the combined organic layers were washed with brine (100 mL × 3), dried over anhydrous Na₂SO₄ and then concentrated *in vacuo.* The residue was purified by column chromatography on silica gel (EtOAc to EtOAc : MeOH = 30 : 1) to give ***2294-D*** (1.4 g, 82%) as a yellow solid. MS 427.2 [M+H]⁺.

***Synthesis of T-2294.*** A mixture of **2294-D** (1.4 g, 3.3 mmol) and Raney-Ni (1.0 g) in DCM/MeOH (10 mL/10 mL) was stirred at room temperature for 1 h under a H₂ atmosphere. Raney-Ni was then removed by filtration through Celite. The filtrate was concentrated and the residue was purified by column chromatography on silica gel (EtOAc to EtOAc : MeOH = 10 : 1) to give **T-2294** (800 mg, 61%) as a gray solid. MS 397.2 [M+H]⁺.

***Chiral-separation of 55.* T-2294** (800 mg, 2.02 mmol) was separated by chiral SFC (Column: Chiralcel OJ-3; Solvent: MeOH; Flow rate: 2 mL/min; RT₅₅ = 2.599 min) to give **55** (226 mg, 17%) as a yellow solid, (RT₅₄ = 1.854 min) to give **54** (226 mg, 17%) as a yellow solid.. MS 397.2 [M+H]⁺.

### Example 20 Synthesis of 39 and 40 (reference example)

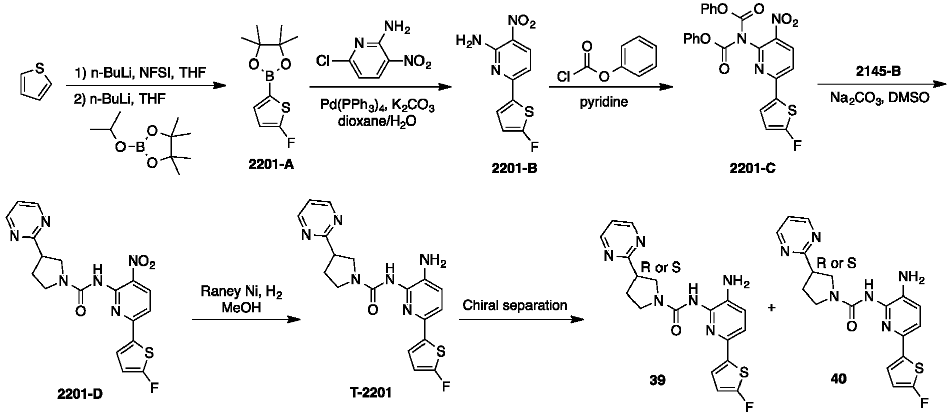

***Synthesis of 2201-A.*** To a solution of thiophene (20.0 g, 238 mmol) in THF (400 mL) was added n-BuLi (2.5 M in hexane)(100 mL) dropwise at -78 °C and the reaction mixture was stirred at -78 °C for 1 h. Then a solution of NFSI (78.8 g, 250 mmol) in THF (400 mL) was added into the above solution dropwise at -78 °C, and the reaction mixture was warmed to room temperature and stirred for 1 h. Then the reaction mixture was cooled again to -78 °C another portion of n-BuLi (2.5 M in hexane)(100 mL) was added dropwise into the above mixture at -78 °C and stirring was continued at -78 °C for 1 h. Finally, a solution of 2-isopropoxy-4,4,5,5-tetramethyl-1,3,2-dioxaborolane (46.5 g, 250 mmol) in THF (200 mL) was added into above solution dropwise at -78 °C. The reaction mixture was warmed to room temperature for 16 h. The reaction mixture was poured into saturated NH₄Cl (1000 mL), then extracted with PE (300 mL × 3), and washed with brine (300 mL × 3). The organic layer was dried over anhydrous Na₂SO₄ and then concentrated *in vacuo* to give **2201-A** (32 g) as a crude product. MS 147.1 [M - 82]⁺.

***Synthesis of 2201-B.*** A mixture of 6-chloro-3-nitropyridin-2-amine (18.9 g, 109.6 mmol), **2201-A** (32 g, crude product from last step) and K₂CO₃ (45.4 g, 328.8 mmol) in dioxane/H₂O (400 mL/40 mL) was added Pd(PPh₃)₄ (2.0 g, 1.73 mmol) under N₂ atmosphere. The mixture was stirred at 95 °C for 4 h and then concentrated *in vacuo.* The residue was dissolved with EtOAc (300 mL) and the solution was washed with brine (100 mL × 3). The organic layer was dried over anhydrous Na₂SO₄ and then concentrated *in vacuo.* The residue was purified by column chromatography on silica gel (PE : DCM = 10 : 1 ∼ 2 : 1) to give **2201-B** (9.0 g, 34%) as a yellow solid. MS 240.1 [M + H]⁺.

***Synthesis of 2201-C.*** A stirred solution of **2201-B** (460 mg, 1.92 mmol) in pyridine (10 mL) was treated with phenyl carbonochloridate (900 mg, 5.77 mmol) dropwise. After the addition was completed, the mixture was stirred at 55 °C for 2 h. The mixture was concentrated *in vacuo.* The residue was purified by column chromatography on silica gel (PE: DCM = 20 : 1 ∼ 1 : 3) to give **2201-C** (700 mg, 76%) as a yellow solid. MS 479.8 [M+H]⁺.

***Synthesis of 2201-D.*** To a solution of ***2201-C*** (106 mg, 0.22 mmol) and **2145-B** (200 mg, 0.40 mmol) in DMSO (10 mL) was added Na₂CO₃ (233 mg, 2.2 mmol). The reaction mixture was stirred at room temperature for 2 h. The mixture was diluted with water (30 mL), extracted with EtOAc (20 mL × 3). The combined organic layers were washed with brine (20 mL × 3), dried over anhydrous Na₂SO₄ and then concentrated *in vacuo.* The residue was purified by Prep-TLC (PE : EA = 1 : 5) to give **2201-D** (75 mg, 82%) as a yellow solid. MS 415.2 [M+H]⁺.

***Synthesis of T-2201.*** To a solution of **2201-D** (75 mg, 0.18 mmol) and Raney-Ni (75 mg) in DCM/MeOH (3 mL/5 mL) was stirred at room temperature for 0.5 h. Raney-Ni was removed by filtration through the Celite. The filtrate was concentrated and the residue was purified by Prep-TLC (EA : MeOH = 10 : 1) to give **T-2201** (15 mg, 22%) as a gray solid. MS 385.2 [M+H]⁺.

***Chiral-separation of 39 and 40.* T-2201** (1.0 g, 2.6 mmol) was separated by chiral separation (Column: Chiralcel OJ-3; Solvent: MeOH; Flow rate: 1.5 mL/min; RT₃₉ = 2.225 min, RT₄₀ = 2.667 min) to give **39** (300 mg, 30%) as a brown solid (MS 385.0 [M+H]⁺) and **40** (300 mg, 30%) as a purple solid. MS 385.0 [M+H]⁺.

**Compound 49** was synthesized in a similar manner as **39** and **40** by using the appropriately substituted boronic acid and aryl bromide reagents. ***Compound 49.*** 30 mg, 23%, a yellow solid.

### Example 22 Synthesis of 36

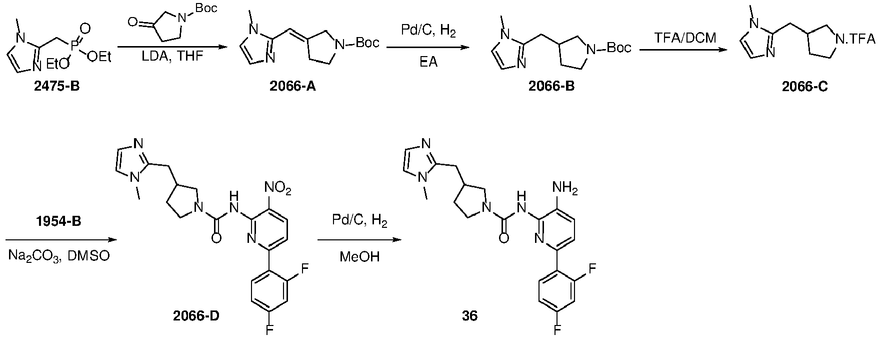

***Synthesis of 2066-A.*** A solution of **2475-B** (400 mg, 1.7 mmol) in THF (10 mL) was treated with LDA (2.6 mL, 5.2 mmol) dropwise at -78 °C under a nitrogen atmosphere and stirred for 1 h at -78 °C. Then a solution of *tert*-butyl 3-oxoazetidine-1-carboxylate (414 mg, 2.2 mmol) in THF (5 mL) was added into above mixture dropwise at -78 °C and then the reaction mixture was allowed to warm to room temperature and stirred for 16 h. The mixture was diluted with saturated NH₄Cl (40 mL), extracted with EtOAc (30 mL × 3). The combined organic layers were washed with brine (30 mL × 3), dried over anhydrous Na₂SO₄ and then concentrated *in vacuo.* The residue was was purified by column chromatography on silica gel (PE : EtOAc = 3 : 1 to EtOAc) to give **2066-A** (380 mg, 84%) as a colorless oil. MS 264.2 **[M+H]⁺*.Synthesis of 2066-B*** A mixture of **2066-A** (380 mg, 1.4 mmol) and Pd/C (380 mg) in EtOAc (10 mL) was stirred at room temperature for 1 h under a H₂ atmosphere. Pd/C was then removed by filtration through a pad of Celite. The filtrate was concentrated to *in vacuo* and the residue was purified by column chromatography on silica gel (PE : EtOAc = 3 : 1 to EtOAc) give **2066-B** (300 mg, 79%) as a colorless oil. MS 266.2 [M+H]⁺.

***Synthesis of 2066-C.*** To a solution of **2066-B** (150 mg, 0.57 mmol) in DCM (6 mL) was added TFA (2 mL) dropwise at 0°C. The reaction mixture was allowed to warm to room temperature and was stirred at room temperature for 1 h. The solution was concentrated *in vacuo* to give **2066-C** as a crude product which was directly used in the next step. MS 166.2 [M + H]⁺.

***Synthesis of 2066-D.*** A mixture of **2066-C** (0.57 mmol, crude product from previous step) and **1954-B** (154 mg, 0.32 mmol) in DMSO (6 mL) was stirred at room temperature for 10 min, then Na₂CO₃ (339 mg, 3.2 mmol) was added and the reaction mixture was stirred at room temperature for 2 h. The mixture was then diluted with water (20 mL) and extracted with EtOAc (10 mL × 3). The combined organic layers were washed with brine (10 mL × 3), dried over anhydrous Na₂SO₄ and then concentrated *in vacuo.* The residue was purified by Prep-TLC (EtOAc : MeOH = 40 : 1) to give **2066-D** (100 mg, 73%) as a yellow solid. MS 443.1 [M + H]⁺.

***Synthesis of 36.*** A mixture of **2066-D** (100 mg, 0.23 mmol) and Pd/C (100 mg) in MeOH/EtOAc (5 mL/5 mL) was stirred at room temperature for 50 min under a H₂ atmosphere. Pd/C was removed by filtration through a pad of Celite. The filtrate was concentrated *in vacuo* and the residue was purified by Prep-TLC (DCM : MeOH = 30 : 1) to give **36** (40 mg, 42%) as a yellow solid. MS 413.0 [M + H]⁺.

### Example 23 Synthesis of Compound 47 (reference example)

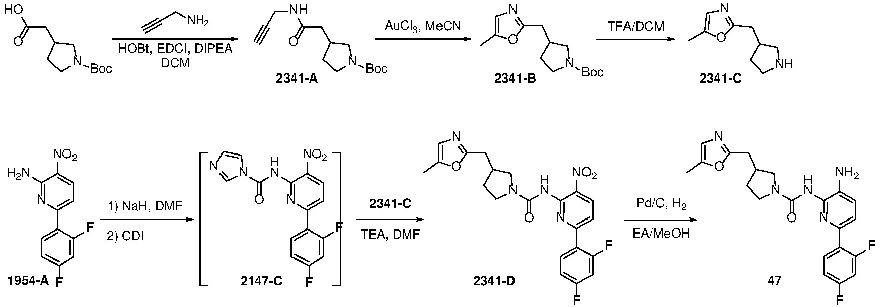

***Synthesis of 2341-A.*** To a solution of 2-(1-(*tert*-butoxycarbonyl)pyrrolidin-3-yl)acetic acid (1.15 g, 5.0 mmol), HOBt (810 mg, 6.0 mmol) and EDCI (1.44 g, 7.5 mmol) in DCM (20 mL) was added DIPEA ( 1.94 g, 15.0 mmol) and stirred at room temperature for 30 min under a nitrogen atmosphere. Then a solution of prop-2-yn-1-amine (413 mg, 7.5 mmol) in DCM (10 mL) was added into above mixture and the resulting mixture was stirred at room temperature for 24 h. The mixture was diluted with DCM (30 mL), washed with 0.5 N HCl (20 mL × 2), saturated NaHCO₃ (20 mL× 2) and brine (20 mL × 2). The organic layer was dried over anhydrous Na₂SO₄ and then concentrated *in vacuo.* The residue was purified by column chromatography on silica gel (PE : EtOAc = 10 : 1 to 2 : 1) to give **2341-A** (1.0 g, 75%) as color oil. MS 211.0 [M -55]⁺.

***Synthesis of 2341-B.*** A solution of **2341-A** (580 mg, 2.2 mmol) in acetonitrile (20 mL) was treated with gold trichloride (50 mg, 0.075 mmol) and the reaction mixture was stirred at 45 °C for 72 h under a nitrogen atmosphere. The mixture was concentrated *in vacuo.* The residue was purified by column chromatography on silica gel (PE : EtOAc = 10 : 1 to 1 : 1) to give **2341-B** (380 mg, 66 %) as colorless oil. MS 267.0 [M + H]⁺.

***Synthesis of 2341-C.*** A solution of **2341-B** (380 mg, 1.4 mmol) in DCM (12 mL) was cooled to 0 °C and then TFA (4 mL) was added dropwise. Following the addition the reaction was allowed to warm to room temperature and was stirred at room temperature for 1 h. The reaction mixture was then concentrated *in vacuo* to give **2341-*C*** as a crude product. Then the residue was dissolved in DMF (6 mL) and was treated with TEA (424 mg, 4.2 mmol) to give **2341-C** as a solution which was used directly in the next step. MS 167.0 [M + H]⁺.

***Synthesis of 2341-D.*** A solution of **1954-A** (252 mg, 1.0 mmol) in DMF (5 mL) was cooled to 0 °C and was treated with NaH (60% in mineral oil, 80 mg, 2.0 mmol). The reaction was stirred at 0 °C for 30 min, then CDI (162 mg, 1.0 mmol) was added and the reaction mixture was stirred at 0 °C for another 30 min. Finally, the solution of **2341-C** was added into the above mixture at 0 °C and the mixture was stirred at 0 °C for 1 h. The mixture was quenched with water (50 mL) and extracted with EtOAc (20 mL × 3). The combined organic layers were washed with brine (20 mL × 3), dried over anhydrous Na₂SO₄ and then concentrated *in vacuo.* The residue was purified by column chromatography on silica gel (PE : EtOAc = 10 : 1 to 1 : 2) to give **2341-D** (280 mg, 63%) as a yellow solid. MS 444.1 [M + H]⁺.

***Synthesis of 47.*** A mixture of **2341-D** (280 mg, 0.63 mmol) and Pd/C (280 mg) in MeOH/EtOAc (10 mL/10 mL) were stirred at room temperature for 1 h under a H₂ atmosphere. Pd/C was removed by filtration through a pad of Celite. The filtrate was concentrated *in vacuo* and the residue was purified by Pre-HPLC to give **47** (220 mg, 85%) as a light yellow solid. MS 414.2 [M + H]⁺.

**Table 1**

| **No.** | **Structure** | **MS Calc** | **MS found** | **¹H NMR Data (400 MHz, DMSO-*d*₆)** |
|---|---|---|---|---|
| **1** | 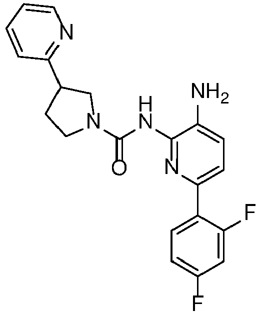 | 395 | 396 | δ 8.54 (d, *J* = 4.8 Hz, 1H), 8.32 (s , 1H), 7.98 - 7.92 (m, 1H), 7.78 - 7.76 (m, 1H), 7.38 (d, *J* = 7.6 Hz, 2H), 7.31 - 7.26 (m, 2H), 7.18 - 7.13 (m, 2H), 5.23 (s, 2H), 3.88 - 3.86 (m, 1H), 3.69 - 3.46 (m, 4H), 2.32 - 2.29 (m, 1H), 2.17 - 2.13 (m, 1H). |
| **2** | 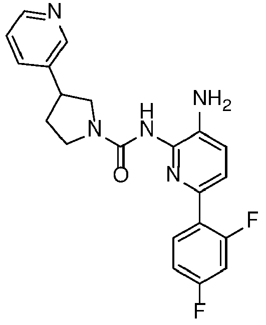 | 395 | 396 | δ 8.57 (d, J = 8.0 Hz, 1H), 8.47 (dd , *J* = 4.8, 1.2 Hz, 1H), 8.35 (s, 1H), 7.98 - 7.92 (m, 1H), 7.76 (d, *J* =8.0 Hz, 1H), 7.40 - 7.26 (m, 3H), 7.18 - 7.14 (m, 2H), 5.24 (s, 2H), 3.96 - 3.91 (m, 1H), 3.70 - 3.66 (m, 1H), 3.53 - 3.33 (m, 3H), 2.35 - 2.30 (m, 1H), 2.10 - 2.00 (m,1H). |
| **3** | 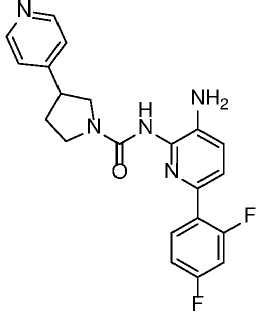 | 395 | 396 | δ 8.53 - 8.51 (m, 2H), 8.36 (s, 1 H), 7.98 - 7.91 (m, 1H), 7.40 - 7.35 (m, 3H), 7.32 - 7.26 (m, 1H), 7.18 - 7.14 (m, 2H), 5.23 (s, 2H), 3.94 - 3.90 (m, 1H), 3.66 -3.63 (m, 1H), 3.50 - 3.41 (m, 3H), 2.33 - 2.31 (m, 1H), 2.05 - 1.98 (m, 1H). |
| **4** | 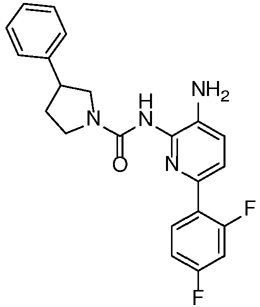 | 394 | 395 | δ 8.32 (s, 1H), 7.98 - 7.92 (m, 1H), 7.40 - 7.24 (m, 7H), 7.16 - 7.14 (m, 2H), 5.23 (s, 2H), 3.93 - 3.89 (m, 1H), 3.69 - 3.65 (m, 1H), 3.49 - 3.21 (m, 3H), 2.29 - 2.27 (m, 1H), 2.04 - 1.99 (m, 1H). |
| **5**** | 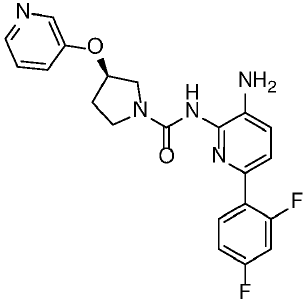 | 411 | 412 | δ 8.39 (s, 1H), 8.19 (dd, *J* = 5.2, 1.2 Hz, 1H), 7.94 - 7.90 (m, 1H), 7.75 - 7.71 (m, 1H), 7.40 - 7.39 (m, 1H), 7.32 - 7.18 (m, 1H), 7.16 - 7.02 (m, 2H), 7.01 - 6.99 (m, 1H), 6.85 (d, *J* = 8.4 Hz, 1H), 5.71 (s, 1H), 3.79 - 3.76 (m, 1H), 3.67 - 3.38 (m, 3 H), 2.27 - 2.13 (m, 2 H). |
| **6**** | 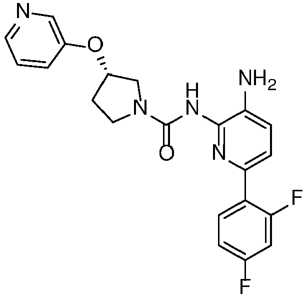 | 411 | 412 | δ 8.39 (s, 1H), 8.20 - 8.18 (m, 1H), 7.97 - 7.90 (m, 1H), 7.75 - 7.71 (m, 1H), 7.38 (dd, *J* = 8.0, 2.4 Hz, 1H), 7.31-7.25 (m, 1H), 7.17 - 7.13 (m, 2H), 7.00 (dd, *J* = 6.0, 5.2 Hz, 1H), 6.85 (d, *J* = 8.0 Hz, 1H), 5.57 (s, 1H), 5.21 (s, 2H), 3.79 - 3.53 (m, 4H), 2.27 - 2.07 (m, 2H). |
| **7** | 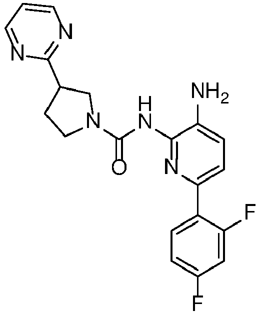 | 396 | 397 | δ 8.80 (d, *J* = 4.8 Hz, 2H), 8.34 (s, 1H), 7.98 - 7.91 (m, 1H), 7.42 - 7.37 (m, 2H), 7.32 - 7.26 (m, 1H), 7.18 - 7.13 (m, 2H), 5.23 (s, 2H), 3.90 - 3.87 (m, 1H),3.77 - 3.75 (m, 2H), 3.64 - 3.59 (m, 1H), 3.55 - 3.51 (m, 1H), 2.36 - 2.33 (m, 1H), 2.27 - 2.22 (m, 1H). |
| **8E1** | 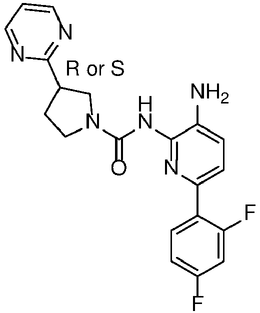 | 396 | 397 | δ 8.79(t, *J* = 4.8 Hz, 2H), 8.33 (s, 1H), 7.98 - 7.91 (m, 1H), 7.42 - 7.37 (m, 2H), 7.31-7.25 (m, 1H), 7.17 - 7.13 (m, 2H), 5.22 (s, 2H), 3.90 - 3.87 (m, 1H), 3.76 - 3.75 (m, 2H), 3.65 - 3.60 (m, 1H), 3.56 - 3.49 (m, 1H), 2.37 - 2.33 (m, 1H), 2.28 - 2.22 (m, 1H). |
| **8E2** | 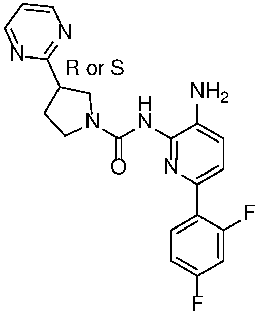 | 396 | 397 | δ 8.79(t, *J* = 4.8 Hz, 2H), 8.33 (s, 1H), 7.98 - 7.91 (m, 1H), 7.42 - 7.37 (m, 2H), 7.31-7.25 (m, 1H), 7.17 - 7.13 (m, 2H), 5.22 (s, 2H), 3.90 - 3.87 (m, 1H), 3.76 - 3.75 (m, 2H), 3.65 - 3.60 (m, 1H), 3.56 - 3.49 (m, 1H), 2.37 - 2.33 (m, 1H), 2.27 - 2.22 (m, 1H). |
| **9** | 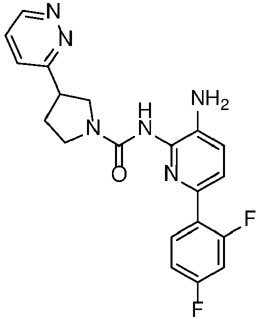 | 396 | 397 | δ 9.14 - 9.12 (m, 1H), 8.36 (s, 1H), 7.96 - 7.90 (m, 1H), 7.71 - 7.65 (m, 2H), 7.37 (dd, *J* = 8.4, 2.4 Hz, 1H), 7.30 - 7.24 (m, 1H), 7.16 - 7.11 (m, 2H), 5.22 (s, 2H), 3.97 - 3.93 (m, 1H), 3.80 - 3.76 (m, 1H), 3.68 - 3.65 (m, 2H), 3.55 - 3.49 (m, 1H), 2.39 - 2.31 (m, 1H), 2.21-2.18 (m, 1H). |
| **10** | 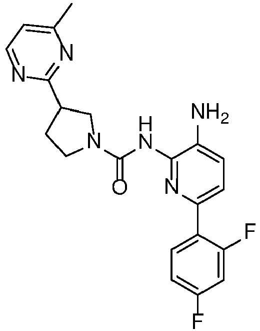 | 410 | 411 | δ 8.62 (d, *J* = 5.2 Hz, 2H), 8.32 (s, 1H), 7.98 - 7.91 (m, 1H), 7.31-7.28 (m, 2H), 7.17 - 7.13 (m, 2H), 5.22 (s, 2H), 3.90 - 3.86 (m, 1H), 3.76 - 3.64 (m, 3H), 3.52 - 3.49 (m, 1H), 2.46 (s, 3H), 2.33 - 2.23 (m, 2H). |
| **11** | 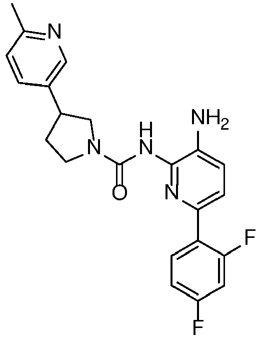 | 409 | 410 | δ 8.41 (d, *J* = 2.4 Hz, 1H), 8.01 - 7.95 (m, 1H), 7.58 (dd, *J* = 8.0, 2.4 Hz, 1H), 7.45 (dd, *J* = 8.0, 4.8 Hz, 1H), 7.19 - 1.17 (m, 3H), 7.07 - 6.98 (m, 2H), 4.74 (s, 2H), 3.95 - 3.91 (m, 1H), 3.73 - 3.69 (m, 1H), 3.56 - 3.38 (m, 3H), 2.47 (s, 3H), 2.35 - 2.34 (m, 1H), 2.18 - 1.93 (m, 1H). |
| **12** | 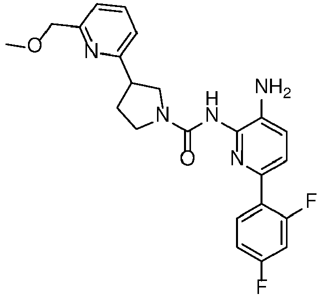 | 439 | 440 | δ 8.33 (s, 1H), 7.98 - 7.92 (m, 1H), 7.78 (t, *J* = 7.6 Hz, 1H), 7.38 (dd, *J* = 8.0, 2.0 Hz, 1H), 7.32 - 7.26 (m, 3H), 7.18 - 7.14 (m, 2H), 5.23 (s, 2H), 4.48 (s, 2H), 3.69 - 3.87 (m, 1H), 3.59 - 3.48 (m, 4H), 3.37 (s, 3H), 2.28 - 2.27 (m, 1H), 2.16 - 2.11 (m, 1H). |
| **13** | 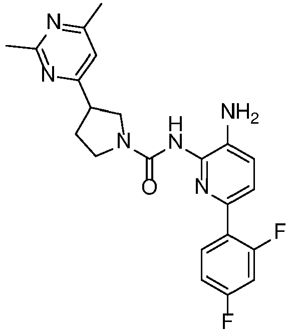 | 424 | 425 | δ 8.35 (s, 1 H), 7.97 - 7.91 (m, 1H), 7.40 - 7.37 (m, 1H), 7.32 - 7.26 (m, 1H), 7.18 - 7.13 (m, 3H), 5.22 (s, 2H), 3.83 (t, *J* = 7.6 Hz, 1H), 3.67 - 3.60 (m, 2H), 3.57 - 3.46 (m, 2H), 2.55 (s, 3H), 2.51 (s, 3H).2.50 - 2.39 (m, 1 H), 2.33 - 2.10 (m, 1H). |
| **14** | 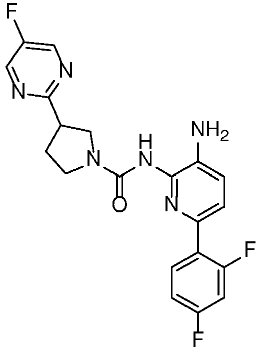 | 414 | 415 | δ 8.89 (d, *J* = 0.8 Hz, 2H), 8.35 (s, 1H), 7.97-7.91 (m, 1H), 7.40-7.37 (m, 1H), 7.32-7.26 (m, 1H), 7.18-7.13 (m, 2H), 5.22 (s, 2H), 3.94 - 3.88 (m, 1H), 3.80 - 3.72 (m, 2H), 3.61 - 3.59 (m, 1H), 3.55 - 3.52 (m, 1H), 2.38 - 2.33 (m, 1H), 2.25 - 2.22 (m, 1H). |
| **14E1** | 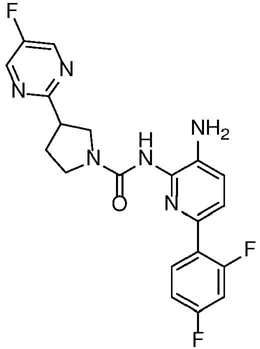 | 414 | 415 | δ 8.88 (s, 2H), 8.54 (s, 1H), 7.97 - 7.91 (m, 1H), 7.38 (dd, *J* = 8.4, 2.4 Hz, 1H), 7.31 - 7.25 (m, 1H), 7.18 - 7.13 (m, 2H), 5.22 (s, 2H), 3.92-3.88(m, 1H), 3.80-3.71 (m, 2H), 3.62-3.59 (m, 1H), 3.53-3.51 (m, 1H), 2.36-2.32 (m, 1H), 2.32-2.22 (m, 1H). |
| **14E2** | 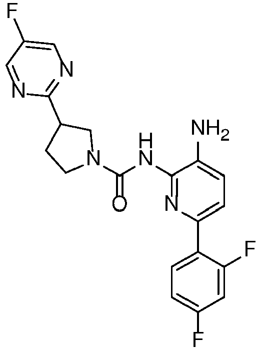 | 414 | 415 | δ 8.88 (s, 2H), 8.54 (s, 1H), 7.97 - 7.91 (m, 1H), 7.38 (dd, *J* = 8.4, 2.4 Hz, 1H), 7.31 - 7.25 (m, 1H), 7.18 - 7.13 (m, 2H), 5.22 (s, 2H), 3.92-3.88(m, 1H), 3.80-3.71 (m, 2H), 3.62-3.59 (m, 1H), 3.53-3.51 (m, 1H), 2.36-2.32 (m, 1H), 2.32-2.22 (m, 1H). |
| **15** | 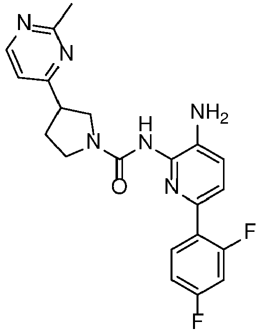 | 410 | 411 | CD₃CN as solvent. δ 8.56 (d, *J* = 5.2 Hz, 1H), 8.02 - 7.96 (m, 1H), 7.46 (dd, *J* = 8.0, 2.4 Hz, 1H), 7.19 - 7.14 (m, 3H), 7.07 - 6.98 (m, 2H), 4.73 (s, 2H), 3.91-3.87 (m, 1H), 3.72-3.67 (m, 2H), 3.60-3.54 (m, 2H), 2.62 (s, 3H), 2.37 - 2.36 (m, 1H), 2.23 - 2.15 (m, 1H). |
| **16** | 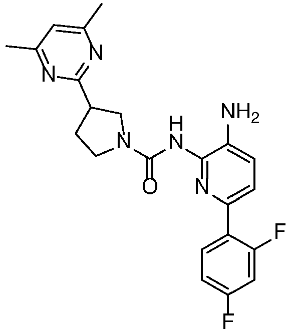 | 424 | 425 | δ 8.33 (s, 1H), 7.98-7.91 (m, 1H), 7.38 (dd, *J* = 8.0, 2.4 Hz, 1H), 7.32 - 7.26 (m, 1H), 7.17 - 7.14 (m, 3H), 5.22 (s, 2H), 3.88 - 3.84 (m, 1H), 3.74 - 3.63 (m, 3H), 3.16 - 3.48 (m, 1H), 2.41 (s, 6H), 2.30-2.19 (m, 2H). |
| **17** | 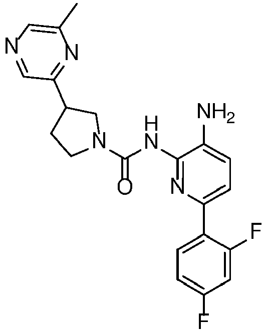 | 410 | 411 | δ 8.44 (s, 1H), 8.41 (s, 1H), 8.33 (s, 1H), 7.95-7.89 (m, 1H), 7.36 (dd, *J* = 8.0, 2.4 Hz, 1H), 7.30 - 7.24 (m, 1H), 7.16 - 7.11 (m, 2H), 5.20 (s, 2H), 3.89 - 3.85 (m, 1H), 3.69 - 3.47 (m, 4H), 2.48 (s, 3H), 2.30-2.16 (m, 1H), 2.13-2.10 (m, 1H). |
| **18** | 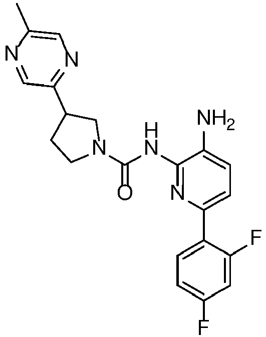 | 410 | 411 | δ 8.54 (s, 1H), 8.51 (s, 1H), 8.35 (s, 1H), 7.98-7.91 (m, 1H), 7.39 (dd, *J* = 8.0, 2.4 Hz, 1H), 7.32 - 7.26 (m, 1H), 7.18 - 7.13 (m, 2H), 5.22 (s, 2H), 3.89 (t, *J* = 4.0 Hz, 1H), 3.67 - 3.64 (m, 2H), 3.59 - 3.49 (m, 2H), 2.18(s, 3H), 2.33-2.29 (m, 1H), 2.17-2.14 (m, 1H). |
| **19** | 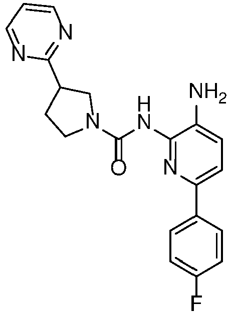 | 378 | 379 | δ 8.81 (t, *J* = 3.6 Hz, 2H), 8.31 (s, 1H), 7.99-7.95 (m, 2H), 7.53 (d, *J* = 8.4 Hz, 1H), 7.41 (t, *J* = 4.8 Hz, 1H), 7.24-7.15 (m, 3H), 5.13 (s, 2H), 3.90 - 3.87 (m, 1H), 3.78 - 3.72 (m, 2H), 3.65 - 3.60 (m, 1H), 3.56 - 3.50 (m, 1H), 2.38 - 2.32 (m, 1H), 2.28 - 2.22 (m, 1H). |
| **19E1** | 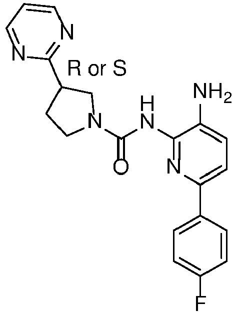 | 378 | 379 | δ 8.81 (d, *J* = 4.8 Hz , 2H), 8.32(s, 1H), 7.99 2-7.96 (m, 2H), 7.54 (d, *J* = 8 Hz, 1H), 7.42 (t, *J* = 4.8 Hz, 1H), 7.24 - 7.19 (m, 2H), 7.16 (d, *J* = 8 Hz, 1H), 5.14 (s, 2H), 3.93 - 3.87 (m, 1H), 3.78 - 3.75 (m, 2H), 3.65 - 3.62 (m, 1H), 3.56-3.50 (m, 1H), 2.38 - 2.33 (m, 1H), 2.29-2.22 (m, 1H). |
| **19E2** | 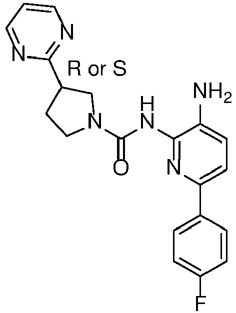 | 378 | 379 | 6 8.81 (d, *J* = 4.8 Hz , 2H), 8.32(s, 1H), 7.99-7.96 (m, 2H), 7.54 (d, *J* = 8 Hz, 1H), 7.42 (t, *J* = 4.8 Hz, 1H), 7.24 - 7.19 (m, 2H), 7.16 (d, *J* = 8 Hz, 1H), 5.14 (s, 2H), 3.93 - 3.87 (m, 1H), 3.78 - 3.75 (m, 2H), 3.65 - 3.62 (m, 1H), 3.56-3.50 (m, 1H), 2.38 - 2.33 (m, 1H), 2.29-2.22 (m, 1H). |
| **20** | 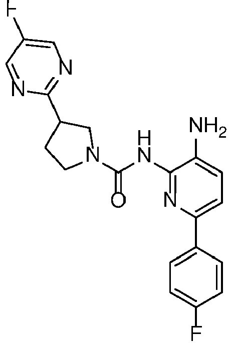 | 396 | 397 | δ 8.89 (s, 2H), 8.32 (s, 1H), 7.99 - 7.95 (m, 2H), 7.54 (d, *J* = 8 Hz, 1H), 7.24 - 7.15 (m, 3H), 5.12 (s, 2H), 3.94 (t, *J* = 6 Hz, 1H), 3.80-3.72 (m, 2H), 3.63-3.60 (m, 1H), 3.56-3.50 (m, 1H), 2.37-2.32 (m, 1H), 2.26-2.21 (m, 1H). |
| **20E1** | 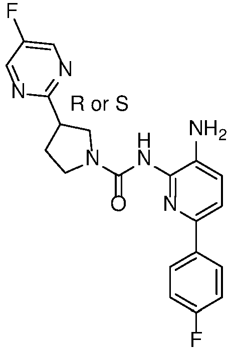 | 396 | 397 | δ 8.89 (s, 2H), 8.32-8.28 (m, 1H), 7.97 (dd, *J* = 8.8, 5.6 Hz, 2H), 7.54 (d, *J* = 8.0 Hz, 1H), 7.22 (t, *J* = 8.8 Hz, 2H), 7.16 (d, *J* = 8.0 Hz, 1H), 5.13 (s, 2H), 3.93 - 3.89 (m, 1H), 3.80 - 3.71 (m, 2H), 3.65 - 3.59 (m, 1H), 3.56 - 3.52 (m,1H), 2.38 - 2.34 (m, 1H), 2.27 - 2.21 (m, 1H). |
| **20E2** | 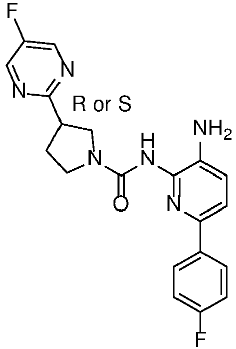 | 396 | 397 | δ 8.89 (s, 2H), 8.32-8.28 (m, 1H), 7.97 (dd, *J* = 8.8, 5.6 Hz, 2H), 7.54 (d, *J* = 8.0 Hz, 1H), 7.22 (t, *J* = 8.8 Hz, 2H), 7.16 (d, *J* = 8.0 Hz, 1H), 5.13 (s, 2H), 3.93 - 3.89 (m, 1H), 3.80 - 3.71 (m, 2H), 3.65 - 3.59 (m, 1H), 3.56 - 3.52 (m,1H), 2.38 - 2.34 (m, 1H), 2.27 - 2.21 (m, 1H). |
| **21**** | 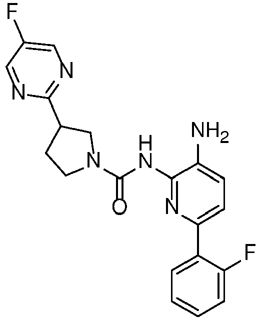 | 396 | 397 | δ 8.89 (s, 2H), 8.34 (s, 1H), 7.93 - 7.88 (m, 1H), 7.41 (dd, *J* = 8, 2.4 Hz, 1H), 7.37 -7.31 (m, 1H), 7.27 - 7.22 (m, 2H), 7.16 (d, *J* = 8 Hz, 1H), 5.20 (d, *J* = 3.6 Hz, 2H), 3.90 (t, *J* = 7.2 Hz, 1H), 3.80 - 3.70 (m, 2H), 3.65 - 3.59 (m, 1H), 3.56 - 3.52 (m, 1H), 2.38 - 2.32 (m, 1H), 2.25 - 2.20 (m, 1H). |
| **22**** | 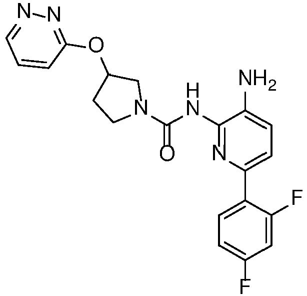 | 412 | 413 | δ 8.92 - 8.91 (m, 1H), 8.40 (s, 1H), 7.96-7.90 (m, 1H), 7.67-7.63 (m, 1H), 7.39-7.37 (m, 1H), 7.32-7.25 (m, 2H), 7.17-7.13 (m, 2H), 5.77 (s, 1H), 5.22 (s, 2H), 3.83 (q, *J* = 4.0 Hz, 1H), 3.70 (q, *J* = 5.6 Hz, 2H), 3.59 - 3.55 (m, 1H), 2.33 - 2.23 (m, 1H), 2.23 - 2.22 (m, 1H). |
| **23**** | 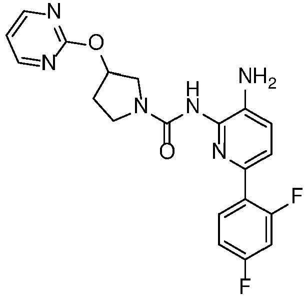 | 412 | 413 | δ 8.64 (d, *J* = 4.8 Hz, 2H), 8.39 (s, 1H), 7.98-7.91 (m, 1H), 7.39-7.36 (m, 1H), 7.31-7.25 (m, 1H), 7.19-7.13 (m, 3H), 5.55 (s, 1H), 5.21 (s, 2H), 3.77 (q, *J* = 3.6 Hz, 1H), 3.66 (t, *J* = 7.2 Hz, 2H), 3.58 - 3.54 (m, 1H), 2.29-2.53 (m, 1H), 2.20-2.17 (m, 1H). |
| **24** | 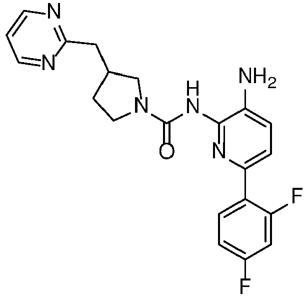 | 410 | 411 | δ 8.74 (d, *J* = 5.2 Hz, 2H), 8.22 (s, 1H), 7.95-7.88 (m, 1H), 7.35 (t, *J* = 4.8 Hz, 2H), 7.29-7.23 (m, 1H), 7.16-7.12 (m, 2H), 5.18 (s, 2H), 3.60 - 3.53 (m, 2H), 3.39 - 3.35 (m, 1H), 3.12 - 3.07 (m, 1H), 3.04 - 2.94 (m, 2H), 2.76 - 2.48 (m, 1H), 2.01 - 1.97 (m, 1H), 1.68 - 1.64 (m, 1H). |
| **25E1** | 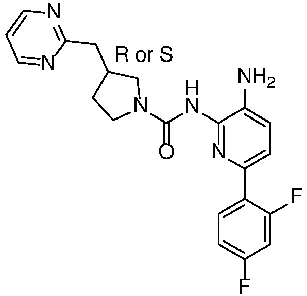 | 410 | 411 | δ 8.76 (d, *J* = 4.8 Hz, 2H), 8.24 (s, 1H), 7.97 - 7.90 (m, 1H), 7.38-7.36 (m, 2H), 7.31-7.25 (m, 1H), 7.18-7.13 (m, 2H), 5.20 (s, 2H), 3.63 - 3.53 (m, 2H), 3.45 - 3.41 (m, 1H), 3.12 (t, *J* = 8.0 Hz, 1H), 3.04 - 2.95 (m, 2H), 2.78 - 2.75 (m, 1H), 2.05 - 2.02 (m, 1H), 1.70 - 1.66 (m, 1H). |
| **25E2** | 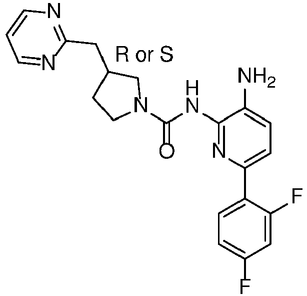 | 410 | 411 | δ 8.76 (d, *J* = 4.8 Hz, 2H), 8.24 (s, 1H), 7.97 - 7.90 (m, 1H), 7.38-7.36 (m, 2H), 7.31-7.25 (m, 1H), 7.18-7.13 (m, 2H), 5.20 (s, 2H), 3.63 - 3.53 (m, 2H), 3.45 - 3.41 (m, 1H), 3.12 (t, *J* = 8.0 Hz, 1H), 3.04 - 2.95 (m, 2H), 2.78 - 2.75 (m, 1H), 2.05 - 2.02 (m, 1H), 1.70 - 1.66 (m, 1H). |
| **26** | 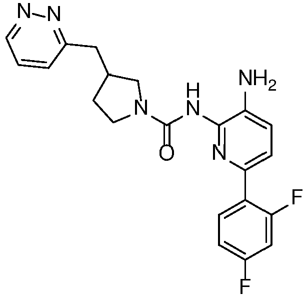 | 410 | 411 | δ 9.11 (t, *J* = 3.2 Hz, 1H), 8.27 (s, 1H), 7.97-7.90 (m, 1H), 7.90-7.62 (m, 2H), 7.37 (dd, *J* = 8.0, 2.0 Hz, 1H), 7.32 - 7.26 (m, 1H), 7.18 - 7.13 (m, 2H), 5.20 (s, 2H), 3.58 - 3.54 (m, 2H), 3.39 - 3.36 (m, 1H), 3.16 - 3.04 (m, 3H), 2.69 - 2.67 (m, 1H), 2.00 -1.98 (m, 1H), 1.71 - 1.65 (m, 1H). |
| **27** | 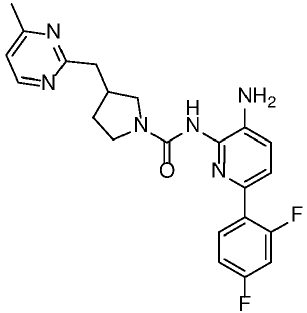 | 424 | 425 | δ 8.58 (d, *J* = 5.2 Hz, 1H), 8.24 (s, 1H), 7.97-7.91 (m, 1H), 7.38-7.36 (m, 1H), 7.32-7.23 (m, 2H), 7.78-7.13 (m, 2H), 5.01 (s, 2H), 3.61 - 3.53 (m, 2H), 3.41 - 3.30 (m, 1H), 3.12 - 3.08 (m, 1H), 3.00 - 2.90 (m, 2H), 2.77 - 2.51 (m, 1H), 2.44 (s, 3H), 2.12-1.96 (m, 1H), 1.73-1.59 (m, 1H). |
| **28** | 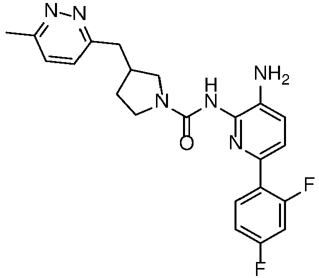 | 424 | 425 | δ 8.26 (s, 1H), 7.97-7.90 (m, 1H), 7.54-7.49 (m, 2H), 7.37 (dd, *J* = 8.0, 2.4 Hz, 1H), 7.31-7.25 (m, 1H), 7.18 - 7.13 (m, 2H), 5.20 (s, 2H), 3.56 - 3.54 (m, 2H), 3.40 - 3.36 (m, 1H), 3.31 - 3.10 (m, 1H), 3.04 - 2.95 (m, 2H), 2.68 - 2.63 (m, 1H), 2.58 (s, 3H), 1.99-1.97 (m, 1H), 1.71-1.64 (m, 1H). |
| **29**** | 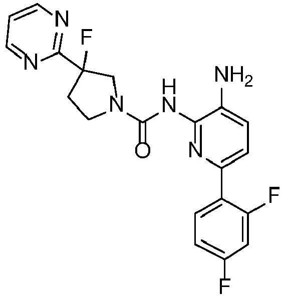 | 414 | 415 | δ 8.94 (d, *J* = 5.2 Hz, 2H), 8.50 (s, 1H), 7.94 (t, *J* = 7.2 Hz, 1H), 7.60 (t, *J* = 4.4 Hz, 1H), 7.40 (q, *J* = 2.4 Hz, 1H), 7.30 (t, *J* = 2.4 Hz, 1H), 7.16 (t, *J* = 8.0 Hz, 2H), 5.24 (s, 2H), 4.13 -4.02 (m, 2H), 3.87 (t, *J* = 11.6 Hz, 1H), 3.66 (t, *J* = 4.8 Hz, 1H), 2.60 - 2.55 (m, 2H). |
| **29E1* *** | 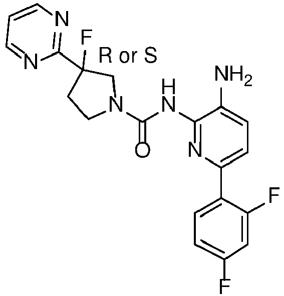 | 414 | 415 | δ 8.94 (d, *J* = 4.8 Hz, 2H), 8.51 (s, 1H), 7.98-7.92 (m, 1H), 7.60 (t, *J* = 4.4 Hz, 1H), 7.40 (q, *J* = 2.4 Hz, 1H), 7.32-7.26 (m, 1H), 7.16 (t, *J* = 8.0 Hz, 2H), 5.25 (s, 2H), 4.13 -4.02 (m, 2H), 3.87 (t, *J* = 9.2 Hz, 1H), 3.67 (q, *J* = 10.0 Hz, 1H), 2.71 - 2.66 (m, 2H). |
| **29E2* *** | 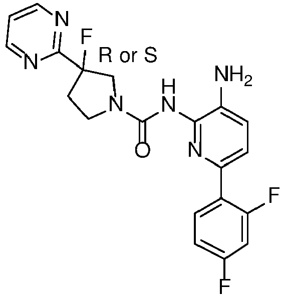 | 414 | 415 | δ 8.94 (d, *J* = 5.2 Hz, 2H), 8.51 (s, 1H), 7.98-7.92 (m, 1H), 7.60 (t, *J* = 4.8 Hz, 1H), 7.40 (q, *J* = 2.4 Hz, 1H), 7.32-7.26 (m, 1H), 7.16 (t, *J* = 6.0 Hz, 2H), 5.25 (s, 2H), 4.13 -4.05 (m, 2H), 3.88 (t, *J* = 8.8 Hz, 1H), 3.67 (q, *J* = 7.2 Hz, 1H), 2.74 - 2.57 (m, 2H). |
| **30** | 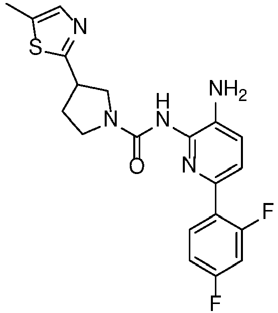 | 415 | 416 | δ 8.38 (s, 1H), 7.97-7.91 (m, 1H), 7.41-7.37 (m, 2H), 7.32-7.26 (m, 1H), 7.18-7.13 (m, 2H), 5.21 (s, 2H), 3.88 - 3.79 (m, 2H), 3.64 - 3.60 (m, 2H), 3.57 - 3.49 (m, 1H), 2.42 (s, 3H), 2.38-2.32 (m, 1H), 2.16-2.11 (m, 1H). |
| **31** | 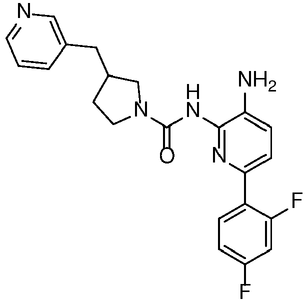 | 409 | 410 | δ 8.46 (s, 1H), 8.32 (s , 1H), 8.41 (d, *J* = 3.6 Hz, 1H), 8.26 (s, 1H), 7.94-7.88 (m, 1H), 7.66 (d, *J* = 7.6 Hz, 1H), 7.36-7.23 (m, 3H), 7.15-7.12 (m, 2H), 5.17 (s, 2H), 3.53 - 3.47 (m, 2H), 3.38 - 3.30 (m, 2H), 3.08 - 3.04 (m, 1H), 2.72 - 2.69 (m, 2H), 1.93 - 1.92 (m, 1H), 1.63 - 1.58 (m, 1H). |
| **32** | 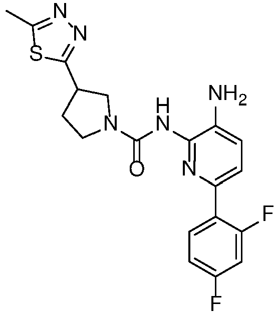 | 416 | 417 | δ 8.43 (s, 1H), 7.97-7.91 (m, 1H), 7.40-7.38 (m, 1H), 7.32-7.26 (m, 1H), 7.18-7.14 (m, 2H), 5.23 (s, 2H), 4.01 - 3.89 (m, 2H), 3.67 - 3.58 (m, 2H), 3.56 - 3.52 (m, 1H), 2.71 (s, 3H), 2.45-2.41 (m, 1H), 2.19-2.14 (m, 1H). |
| **33** | 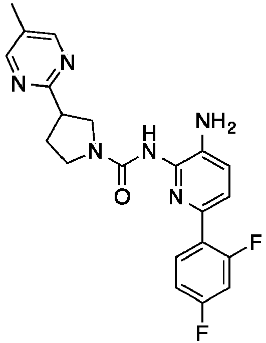 | 410 | 411 | δ 8.60 (s, 2H), 8.01-7.95 (m, 1H), 7.46 (dd , *J* = 8.4, 6.4 Hz, 1H), 7.20 - 7.16 (m, 2H), 7.07 - 6.99 (m, 2H), 4.73 (s, 2H), 3.98 - 3.93 (m, 1H), 3.75 - 3.69 (m, 1H), 3.59 - 3.35 (m, 1H), 3.47 - 3.42 (m, 2H), 2.62 (s, 3H), 2.41-2.38 (m,1H), 2. 10-2.09 (m,1H). |
| **34** | 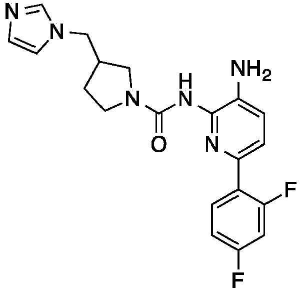 | 398 | 399 | *δ* 8.00 - 7.93 (m, 1H), 7.50 (s ,1H), 7.44 (d , *J* = 7.2 Hz, 1H), 7.16 (d , *J* = 8.0 Hz, 1H), 7.09-6.98 (m, 4H), 6.95 (s ,1H), 4.70 (s, 2H), 4.03 (d, *J* = 8.4 Hz, 2H), 3.57 - 3.50 (m, 2H), 3.46 - 3.40 (m, 1H), 3.20 - 3.16 (m, 1H), 2.71 (s, 1H), 2.06 - 1.99 (m, 1H), 1.73 - 1.68 (m, 1H). |
| **35** | 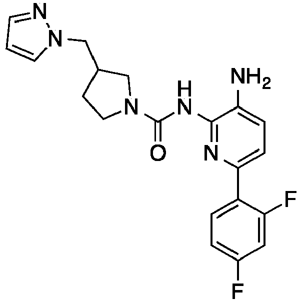 | 398 | 399 | *δ* 8.00 - 7.94 (m, 1H), 7.55 -7.43 (m, 3H), 7.16 (d, *J* = 8.0 Hz, 1H), 7.07 - 6.98 (m, 3H), 6.25 (s, 1H), 4.71 (s, 2H), 4.19 - 4.17 (m, 2H), 3.58 - 3.42 (m, 3H), 3.25 - 3.20 (m, 1H), 2.83 - 2.78 (m, 1H), 2.04 - 1.93 (m, 1H), 1.76 - 1.71 (m, 1H). |
| **36** | 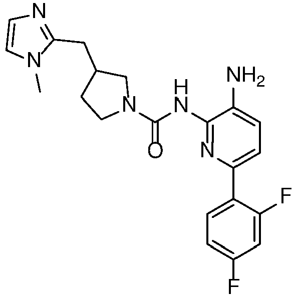 | 412 | 413 | δ 8.25 (s, 1 H), 7.92-7.91 (m, 1H), 7.36 (dd, *J* = 6.0, 2.4 Hz, 1H), 7.31- 7.25 (m, 1H), 7.17 - 7.13 (m, 2H), 7.01 (s, 1H), 6.75 (s, 1H), 5.21 (s, 2H), 3.63 (q, *J* = 6.8 Hz, 1H), 3.61 (s, 3H), 3.57 - 3.51 (m, 1H), 3.42 -3.38 (m, 1H), 3.11 (t, *J* = 8.4 Hz, 1H), 2.77 - 2.72 (m, 2H), 2.70 - 2.62 (m, 1H), 2.06 - 2.04 (m, 1 H), 1.70 - 1.65 (m, 1H). |
| **37** | 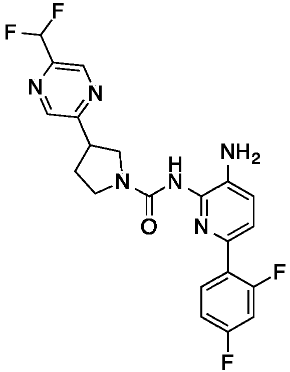 | 446 | 447 | δ 8.93 (s, 1H), 8.83 (s, 1H), 8.37 (s, 1H), 7.98-7.91 (m, 1H), 7.38 (dd , *J* = 8.4, 2.4 Hz, 1H), 7.31- 6.99 (m, 4H), 5.23 (s, 2H), 3.96 - 3.92 (m, 1H), 3.83 - 3.79 (m, 1H), 3.69 - 3.62 (m, 2H), 3.57 - 3.51 (m, 1H), 2.42 - 2.35 (m, 1H), 2.24 - 2.15 (m, 1H). |
| **39**** | 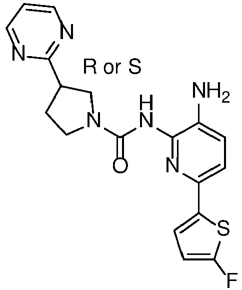 | 384 | 385 | δ 8.80 (d, *J* = 4.8 Hz, 2H), 8.29 (s, 1H), 7.48 (d, J= 8.4 Hz, 1H), 7.41(t, *J* = 4.8 Hz, 1H), 7.16 (t, *J*= 4.0 Hz, 1H), 7.11 (d, *J*= 8.0 Hz, 1H), 6.66 (q, *J* = 2.0 Hz, 1H), 5.11 (s, 2H), 3.91 - 3.85 (m, 1H), 3.77 - 3.71 (m, 2H), 3.63 - 3.57 (m, 1H), 3.54 - 3.48 (m, 1H), 2.37 - 2.31 (m, 1H), 2.28 - 2.21 (m, 1H). |
| **40**** | 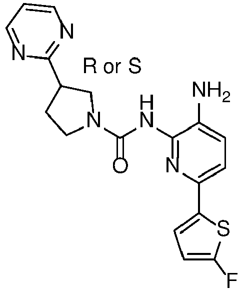 | 384 | 385 | δ 8.80 (d, *J* = 4.8 Hz, 2H), 8.29 (s, 1H), 7.48 (d, *J*= 8.4 Hz, 1H), 7.41(t, *J* = 4.8 Hz, 1H), 7.16 (t, *J*= 4.0 Hz, 1H), 7.11 (d, *J* = 8.0 Hz, 1H), 6.66 (q, *J* = 2.0 Hz, 1H), 5.11 (s, 2H), 3.91 - 3.85 (m, 1H), 3.77 - 3.71 (m, 2H), 3.63 - 3.57 (m, 1H), 3.54 - 3.48 (m, 1H), 2.37 - 2.31 (m, 1H), 2.28 - 2.21 (m, 1H). |
| **41**** | 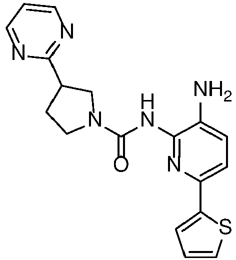 | 366 | 367 | & 8.80 (q, *J* = 3.2 Hz, 2H), 8.29 (s, 1H), 7.49-7.46 (m, 2H), 7.42-7.39 (m, 2H), 7.12 (d, *J*= 8.0 Hz, 1H), 7.06 (t, *J* = 3.6 Hz, 1H), 5.10 (s, 2H), 3.87 (d, *J* = 10.4 Hz, 1H), 3.75 (d, *J* = 6.4 Hz, 2H), 3.61 (t, *J* = 3.2 Hz, 1H), 3.52 (t, *J* = 9.6 Hz, 1H), 2.36 - 2.22 (m, 2H). |
| **42**** | 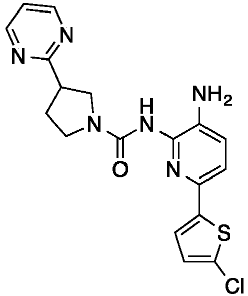 | 400 | 401 | & 8.80 (d, *J* = 4.8 Hz, 2H), 8.28 (s, 1H), 7.48 (d, *J* = 8.4 Hz, 1H), 7.41 (t, *J* = 4.8 Hz, 1H), 7.33 (d, *J* = 4.0 Hz, 1H), 7.12 (t, *J* = 4.4 Hz, 1H), 7.05 (d, *J* = 4.0 Hz, 1H), 5.19 (s, 2H), 3.67 (d, *J* = 11.2 Hz, 1H), 3.74 (d, *J* = 5.2 Hz, 2H), 3.59 (d, *J* = 8.4 Hz, 1H), 3.52 (t, *J* = 6.8 Hz, 1H), 2.37-2.24 (m, 2H). |
| **43**** | 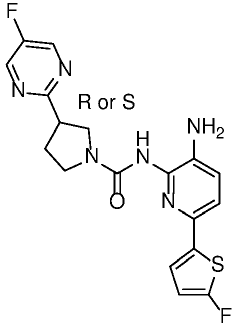 | 402 | 403 | δ 8.89 (s, 2H), 8.29 (s, 1H), 7.47 (d, *J* = 4.0 Hz, 1H), 7.17 -7.10 (m, 2H), 6.66 (q, *J* = 2.0 Hz, 1H), 5.12 (s, 2H), 3.90 -3.86 (m, 1H), 3.79-3.69 (m, 2H), 3.61-3.47 (m, 2H), 2.37-2.33 (m, 1H), 2.24-2.19 (m, 1H) . |
| **44**** | 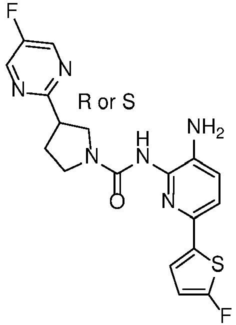 | 402 | 403 | δ 8.89 (s, 2H), 8.29 (s, 1H), 7.47 (d, *J* = 4.0 Hz, 1H), 7.17 -7.10 (m, 2H), 6.66 (q, *J* = 2.0 Hz, 1H), 5.12 (s, 2H), 3.90-3.86 (m, 1H), 3.79-3.69 (m, 2H), 3.61-3.47 (m, 2H), 2.37-2.33 (m, 1H), 2.24-2.19 (m, 1H) . |
| **45**** | 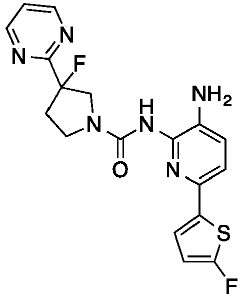 | 402 | 403 | δ 8.95 (d, *J* = 5.2 Hz, 2H), 8.46 (s, 1H), 7.60 (t, *J* = 8.4 Hz, 1H), 7.49 (d, *J* = 8.4 Hz, 1H), 7.17(t, *J* = 4.0 Hz, 1H), 7.12 (d, *J* = 8.0 Hz, 1H), 6.68 - 6.66 (m, 1H), 5.15 (s, 2H), 4.12 - 4.04 (m, 2H), 3.86 (t, *J* = 10.0 Hz, 1H), 3.66 (t, *J* = 8.0 Hz, 1H), 2.68 - 2.54 (m, 2H). |
| **46** | 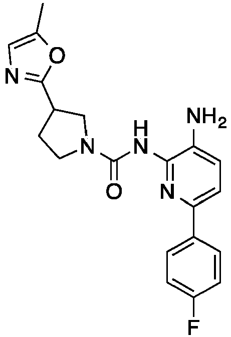 | 381 | 382 | δ 8.35 (s, 1H), 7.97 (q, *J* = 6.4 Hz, 2H), 7.54 (d, *J* = 8.4 Hz, 1H), 7.24-7.15 (m, 3H), 6.76 (s, 1H), 5.13 (s, 2H), 3.81 (t, *J* = 6.4 Hz, 1H), 3.68 - 3.59 (m, 2H), 3.57-3.60 (m, 2H), 2.33-2.28 (m, 1H), 2.25 (s, 3H), 2.20 - 2.15 (m, 1H). |
| **47** | 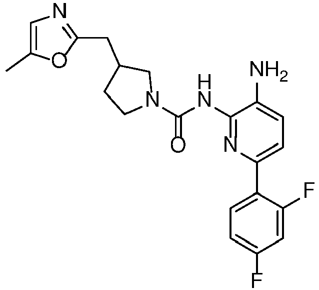 | 413 | 414 | **CD₃OD** as solvent δ 7.92 - 7.88 (m, 2H), 7.48 (d, *J* = 8 Hz, 1H), 7.26 (d, *J* = 8.4 Hz, 1H), 7.14 -7.10 (m, 2H), 6.69 (s, 1H) 3.78-3.71 (m, 1H), 3.67-3.61 (m, 1H), 3.54-3.47 (m, 1H), 3.26-3.21 (m, 1H), 2.89 (d, *J* = 7.2 Hz, 2H), 2.78-2.73 (m, 1H), 2.30 (d, *J* = 0.8 Hz, 3H), 2.17-2.14 (m, 1H), 1.84-1.77 (m, 1H). |
| **48**** | 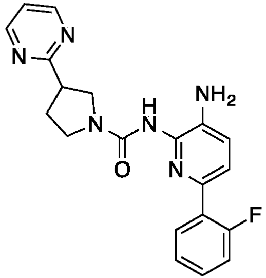 | 378 | 379 | δ 8.80 (d, *J* = 5.2 Hz, 2H), 8.33 (s, 1H), 7.93 - 7.89 (m, 1H), 7.43-7.36 (m, 2H), 7.36-7.31 (m, 1H), 7.27-7.22 (m, 2H), 7.16 (d, *J* = 8.4 Hz, 1H), 5.22 (s, 2H), 3.90 - 3.87 (m, 1H), 3.78 - 3.75 (m, 2H), 3.63 - 3.60 (m, 1H), 3.56 - 3.50 (m, 1H), 2.37 - 2.33 (m, 1H), 2.27 - 2.22 (m, 1H). |
| **49**** | 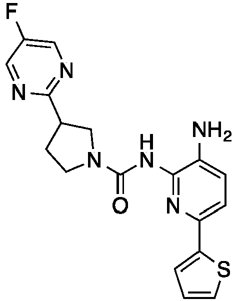 | 384 | 385 | δ 8.78 (s, 2H), 8.18 (s, 1H), 7.38 - 7.35 (m, 2H), 7.30 (dd , *J* = 5.2, 0.8 Hz, 1H), 7.00 (d, *J* = 8.4 Hz, 1H), 6.94 (dd , *J* = 5.2, 3.6 Hz, 1H), 4.99 (s, 2H), 3.80 - 3.75 (m, 1H), 3.68 - 3.58 (m, 2H), 3.48 - 3.46 (m, 1H), 3.42 - 3.37 (m, 1H), 2.28 - 2.19 (m,1H), 2.16 - 2.01 (m,1H). |
| **50** | 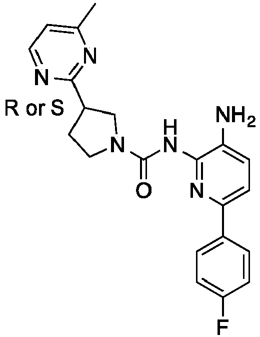 | 392 | 393 | δ 8.63 (d, *J* = 5.2 Hz, 1H), 8.30 (s, 1H), 7.99 - 7.95 (m, 2H), 7.53 (d, *J* = 8.0 Hz, 1H), 7.27 (d, *J* = 5.2 Hz, 1H), 7.24-7.19 (m, 2H), 7.15 (d, *J* = 8.0 Hz, 1H), 5.12 (s, 2H), 3.90 - 3.85 (m, 1H), 3.76 - 3.61 (m, 3H), 3.54 - 3.52 (m, 1H), 2.47 (s, 3H), 2.33-2.26 (m, 2H). |
| **51** | 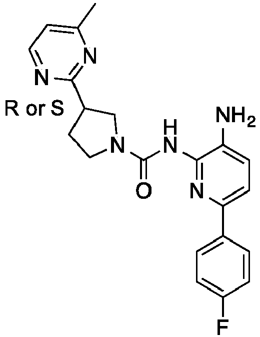 | 392 | 393 | δ 8.66 (d, *J* = 5.2 Hz, 1H), 8.28 (s, 1H), 7.97 - 7.93 (m, 2H), 7.51 (d, *J* = 8.4 Hz, 1H), 7.25 (d, *J* = 5.2 Hz, 1H), 7.21-7.17 (m, 2H), 7.14 (d, *J* = 8.0 Hz, 1H), 5.10 (s, 2H), 3.88 - 3.84 (m, 1H), 3.74 - 3.59 (m, 3H), 3.52 - 3.46 (m, 1H), 2.45 (s, 3H), 2.32-2.19 (m, 2H). |
| **52**** | 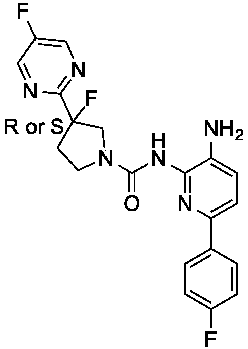 | 414 | 415 | δ 9.02 (s, 2H), 8.47 (s, 1H), 7.97 -7.94 (m, 2H), 7.53 (d, *J*= 8.4 Hz, 1H), 7.24 - 7.14 (m, 3H), 5.10 (s, 2H), 4.12-3.98 (m, 2H), 3.85 (t, *J* = 8.4 Hz, 1H), 3.69 - 3.62 (m, 1H), 2.68 - 2.55 (m, 2H). |
| **53**** | 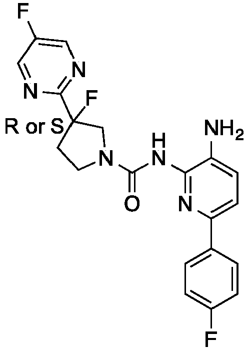 | 414 | 415 | δ 9.02 (s, 2H), 8.47 (s, 1H), 7.97 -7.94 (m, 2H), 7.53 (d, *J*= 8.4 Hz, 1H), 7.22 - 7.14 (m, 3H), 5.10 (s, 2H), 4.12-4.01 (m, 2H), 3.85 (t, *J* = 8.4 Hz, 1H), 3.69-3.62 (m, 1H), 2.66-2.55 (m, 2H). |
| **54**** | 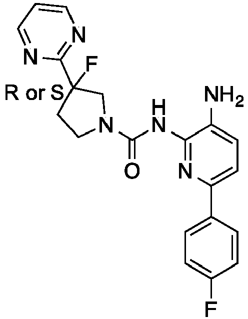 | 396 | 397 | δ 8.95 (d, *J* = 5.2 Hz, 2H), 8.49 (s, 1H), 7.98 (t, *J*= 8.4 Hz, 2H), 7.62 - 7.55 (m, 2H), 7.24-7.16 (m, 3H), 5.13 (s, 2H), 4.14 - 4.04 (m, 2H), 3.90 - 3.86 (m, 1H), 3.68 (t, *J* = 5.2 Hz, 1H), 2.67 - 2.57 (m, 2H). |
| **55**** | 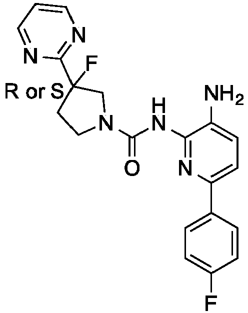 | 396 | 397 | δ 8.95 (d, *J* = 5.2 Hz, 2H), 8.49 (s, 1H), 7.98 (t, *J*= 8.4 Hz, 2H), 7.62 - 7.55 (m, 2H), 7.24-7.16 (m, 3H), 5.13 (s, 2H), 4.14 - 4.04 (m, 2H), 3.90 - 3.86 (m, 1H), 3.68 (t, *J* = 5.2 Hz, 1H), 2.67 - 2.57 (m, 2H). |

| | | | | |
|---|---|---|---|---|
| **** reference examples** | | | | |

### HDAC2 and HDAC1 Enzymatic Assay (HDAC2 and HDAC1 IC50 data)

The following describes an assay protocol for measuring the deacetylation of a peptide substrate by HDAC2 or HDAC1.

HDAC protein composition and respective substrate peptides are summarized below.

| **Assay name** | **Expression Construct** | **Regulatory subunit** | **Substrate peptide** |
|---|---|---|---|
| HDAC1 | Full length Human HDAC1 with C-terminal His-tag and C-terminal FLAG-tag, expressed in baculovirus expression system. | None | FAM-TSRHK(Ac)KL-NH2 |
| HDAC2 | Full length Human HDAC2 with C-terminal FLAG-tag, expressed in baculovirus expression system. | None | FAM-TSRHK(Ac)KL-NH2 |

### Assay set up:

HDAC reactions are assembled in 384 well plates (Greiner) in a total volume of 20 □L as following:
HDAC proteins are pre-diluted in the assay buffer comprising: 100mM HEPES, pH 7.5, 0.1% BSA, 0.01% Triton X-100, 25mM KC1 and dispensed into 384 well plate (10uL per well).

Test compounds are serially pre-diluted in DMSO and added to the protein samples by acoustic dispensing (Labcyte Echo). Concentration of DMSO is equalized to 1% in all samples.

Control samples (0%-inhibition in the absence of inhibitor, DMSO only) and 100%-inhibition (in the absence of enzyme) are assembled in replicates of four and used to calculate the %-inhibition in the presence of compounds.

At this step compounds can be pre-incubated with enzyme if desired.

The reactions are initiated by addition of 10uL of the FAM-labeled substrate peptide pre-diluted in the same assay buffer. Final concentration of substrate peptide is 1uM (HDAC1-2). The reactions are allowed to proceed at room temperature. Following incubation, the reactions are quenched by addition of 50 □L of termination buffer (100 mM HEPES, pH7.5, 0.01% Triton X-100, 0.1% SDS). Terminated plates are analyzed on a microfluidic electrophoresis instrument (Caliper LabChip^{®} 3000, Caliper Life Sciences/Perkin Elmer) which enables electrophoretic separation of de-acetylated product from acetylated substrate. A change in the relative intensity of the peptide substrate and product is the parameter measured. Activity in each test sample is determined as the product to sum ratio (PSR): P/(S+P), where P is the peak height of the product, and S is the peak height of the substrate. Percent inhibition (Pᵢₙₕ) is determined using the following equation: Pᵢₙₕ= (PSR_{0%inh} - PSR_{compound})/(PSR_{0%inh} - PSR_{100%inh})*100, in which: PSR_{compound} is the product/sum ratio in the presence of compound, PSR_{0%inh} is the product/sum ratio in the absence of compound and the PSR_{100%inh} is the product/sum ratio in the absence of the enzyme. To determine IC50 of compounds (50%-inhibition) the %-inh data (Pᵢₙₕ versus compound concentration) are fit by a 4 parameter sigmoid dose-response model using XLfit software (IDBS).

The results of this assay for certain compounds are reported in **Table 2,** below. In the table, "A" indicates a IC50 value of less than 0.5 µM; "B" a IC50 value from 0.5 µM to 1.0 µM; "C" a IC50 value of greater than 1.0 µM and less than or equal to 2.0 µM; and "D" indicates an IC50 value of greater than 2.0 µM. NT = Not Tested.

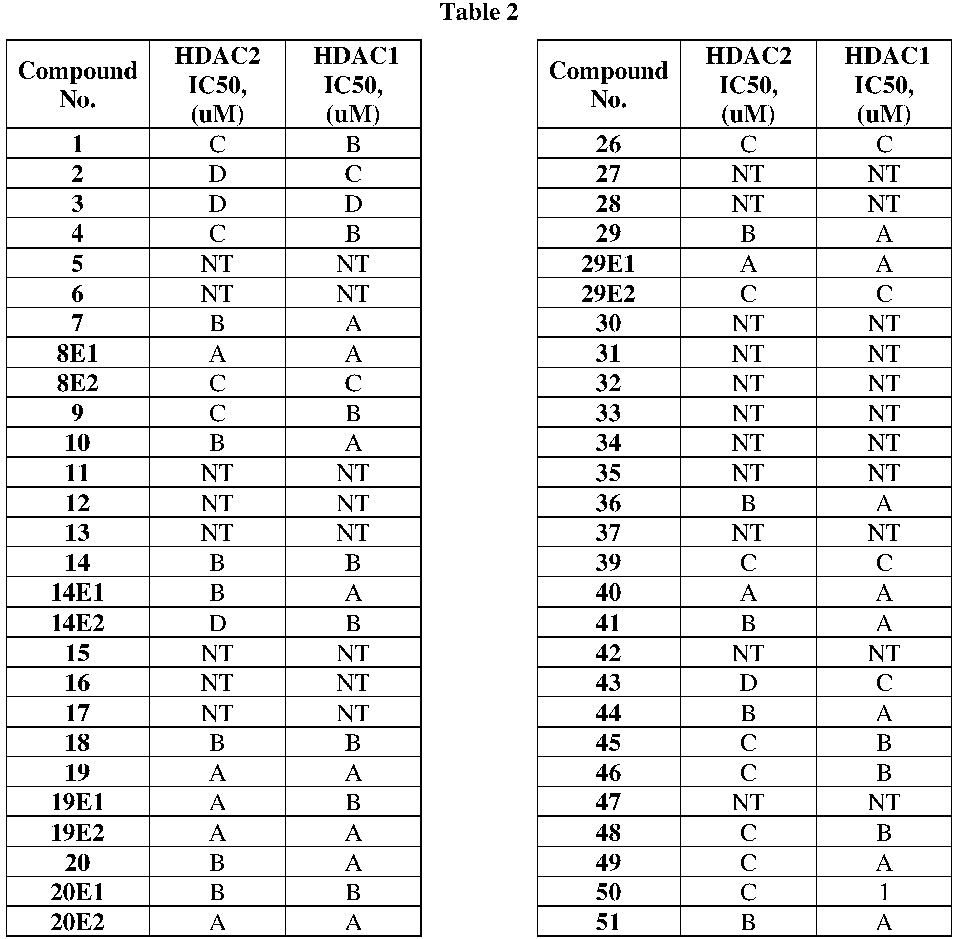

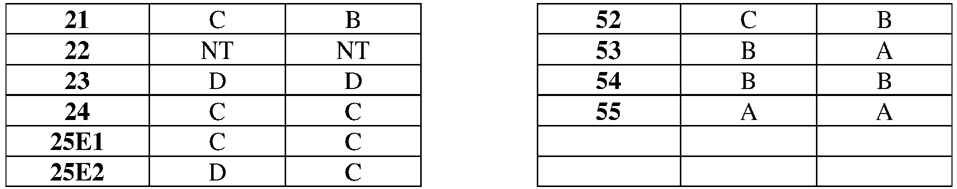

### HDAC2 Enzymatic Inhibition Assay in SH-SY5Y Cell Lysate with an Exogenous Substrate

SH-SY5Y cells (Sigma) were cultured in Eagle's Modified Essential Medium supplemented with 10% fetal bovine serum and pen/strep. Twenty-four hours prior to compound dosing 20 uL of cells were plated in white 384 well plates at a density of 1,500 cells/well. Compounds were serially diluted in neat DMSO and then diluted 1:100 v/v into media without FBS and mixed. Media was removed from the plated cells and the diluted compounds in serum free media (1% v/v final DMSO) were added and incubated at 37_{°}C for five hours. Ten uL of HDAC-Glo 2 reagent with 0.1% Triton X-100 was then added, the plate was mixed and allowed to develop at room temperature for 100 minutes. Plates were then read with a Spectramax LMax luminometer employing a 0.4s integration time. Dose response curves were constructed with normalized data where CI-994 at 100 uM was defined as 100% inhibition and DMSO alone as 0% inhibition.

The results of this assay for certain compounds are reported in **Table 3,** below. In the table, "A" indicates a IC50 value of less than 0.5 µM; "B" a IC50 value from 0.5 µM to 1.0 µM; "C" a IC50 value of greater than 1.0 µM and less than or equal to 2.0 µM; and "D" indicates an IC50 value of greater than 2.0 µM. NT = Not Tested.

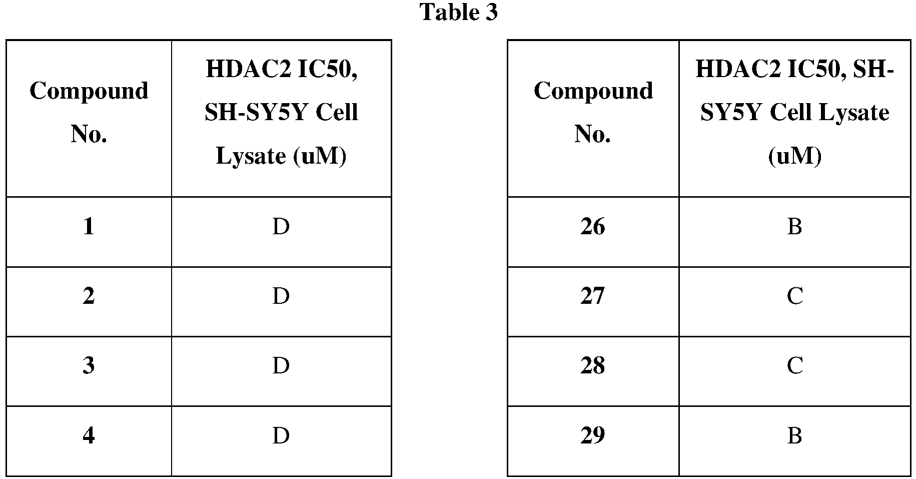

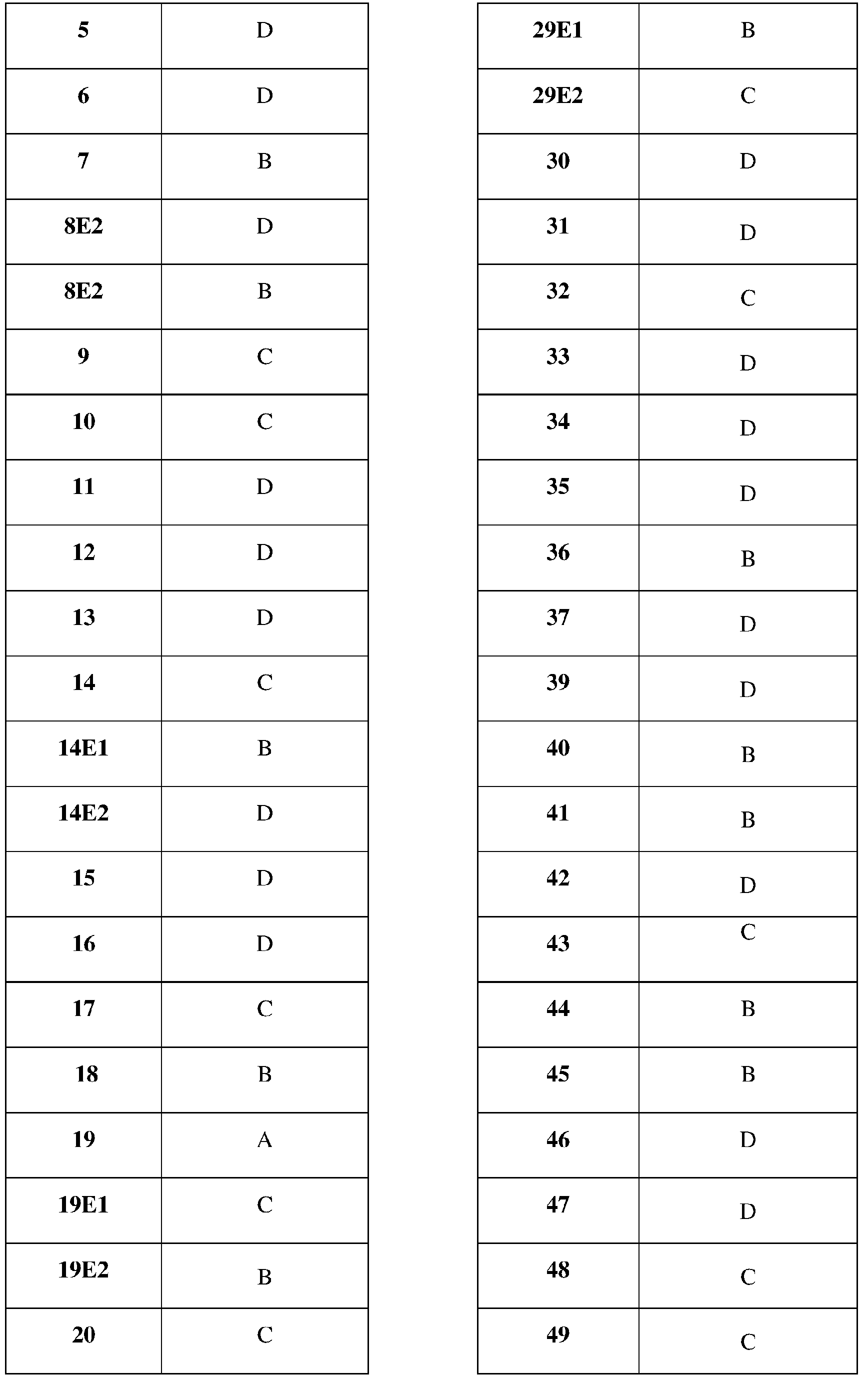

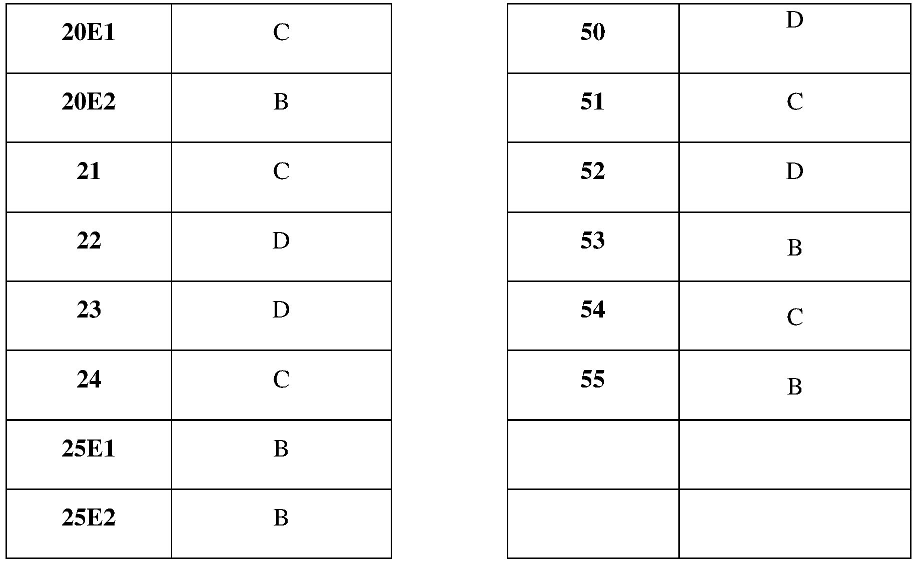

### Comparison of pyrrolidine rings substituted at the 3-position with heteroaromatic rings or methylene-linked heteroaromatic rings to pyrrolidines substituted with non-aromatic groups at the 3-position for pyrrolidine ureas

**Table 4** below shows direct comparison of the activity levels between certain compounds possessing non-aromatic substitution at the pyrrolidine-3-position and inventive compounds possessing aromatic substitution at the 3-position of the pyrrolidinyl motif (i.e., R¹ with or without spacer group X). As shown by the data, an increase in potency in the HDAC2 SH-SY5Y cell lysate assay results when the non-cyclic, non-aromatic substituents for R¹ in **Comparators A-C** are replaced with the aromatic pyrimidinyl in **Compounds 19** and **20.** A similar trend is seen for other compounds in **Table 4. Compound 20** with a 5-F-pyrimidine directly linked to the 3-position is >2-fold more potent than **Comparator A.**

**Table 4**

| **No.** | **Structure** | **HDAC2 IC50, SH-SY5Y Cell Lysate (uM)** |
|---|---|---|
| **Comparator A** | 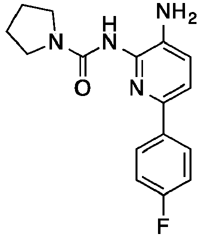 | 2.64 |
| **Comparator B** | 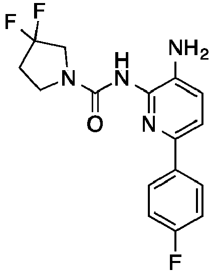 | 14 |
| **Comparator C** | 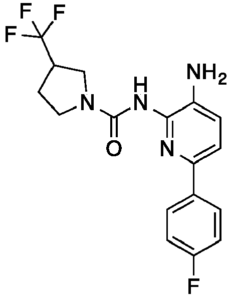 | 6.36 |
| **19** | 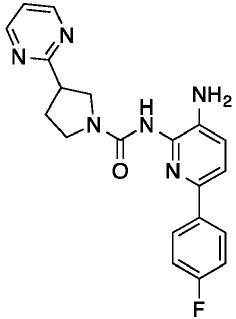 | 0.421 |
| **20** | 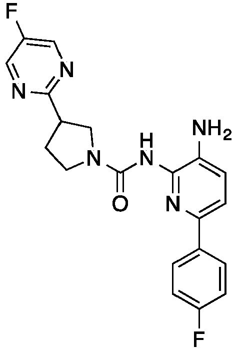 | 1.13 |
| **18** | 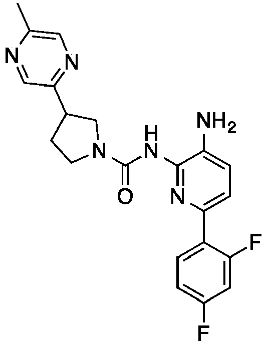 | 0.550 |
| **29**** | 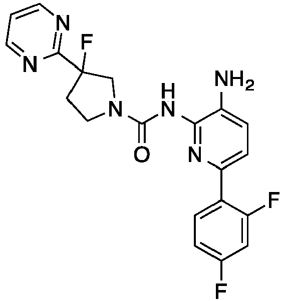 | 0.768 |
| **Comparator E** | 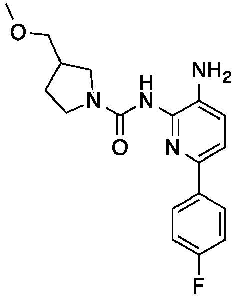 | >100 |
| **24** | 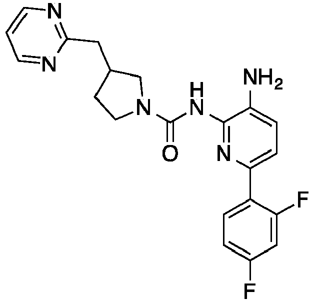 | 1.42 |
| **26** | 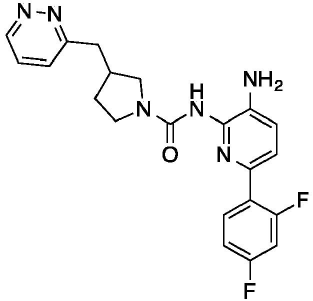 | 0.535 |

| | | |
|---|---|---|
| **"reference example** | | |

Unless otherwise defined, all technical and scientific terms used herein are accorded the meaning commonly known to one with ordinary skill in the art.

## Claims

1. A compound of the Formula **VI** or **VII:** 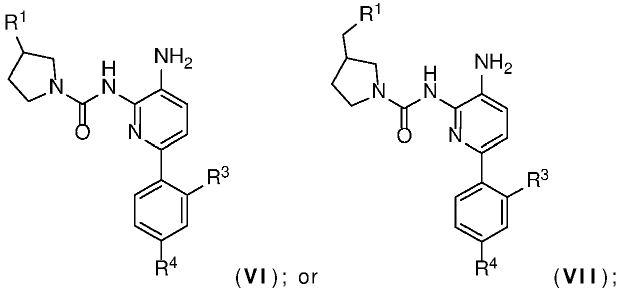 or a pharmaceutically acceptable salt thereof, wherein
R¹ is phenyl or heteroaryl, each of which are optionally substituted with 1 to 3 groups selected from R^{c};
R³ is hydrogen or fluoro;
R⁴ is fluoro; and
R^{c} is halo, (C₁-C₄)alkyl, halo(C₁-C₄)alkyl, (C₁-C₄)alkoxy, halo(C₁-C₄)alkoxy, (C₁-C₄)alkylO(C₁-C₄)alkyl, (C₁-C₄)alkylNH(C₁-C₄)alkyl, (C₁-C₄)alkylN((C₁-C₄)alkyl)₂,-(C₁-C₄)alkylheteroaryl, or -(C₁-C₄)alkylheterocyclyl, wherein said heteroaryl and heterocyclyl are each optionally and independently substituted with 1 to 3 groups selected from (C₁-C₄)alkyl, halo(C₁-C₄)alkyl, (C₁-C₄)alkoxy, and halo;
wherein heteroaryl represents a 5- to 12-membered aromatic radical containing 1 to 4 heteroatoms selected from N, O, and S; and
heterocyclyl represents a 4- to 12-membered saturated or partially unsaturated heterocyclic ring containing 1 to 4 heteroatoms independently selected from N, O, and S.

2. The compound of Claim 1, wherein R¹ is phenyl or 5- to 6-membered monocyclic heteroaryl, each of which is optionally substituted with one or more groups selected from R^{c}.

3. The compound of Claim 1 or 2, wherein R¹ is phenyl, pyridinyl, pyrazinyl, pyridazinyl, or pyrimidinyl, each of which is optionally substituted with 1 to 2 groups selected from R^{c}.

4. The compound of any one of Claims 1 to 3, wherein R¹ is thiazolyl, thiadiazolyl, imidazolyl, pyrazolyl, or oxazolyl, each of which is optionally substituted with 1 to 2 groups selected from R^{c}.

5. The compound of any one of Claims 1 to 4, wherein R^{c} is halo, (C₁-C₄)alkyl, or (C₁-C₄)alkylO(C₁-C₄)alkyl.

6. The compound of any one of Claims 1 to 5, wherein R^{c} is fluoro, methyl, or CH₂OCH₃.

7. The compound of any one of Claims 1 to 6, wherein R^{c} is halo, halo(C₁-C₄)alkyl, or (C₁-C₄)alkyl.

8. The compound of any one of Claims 1 to 7, wherein R^{c} is fluoro, methyl, or CHF₂.

9. The compound of Claim 1, wherein the compound is selected from 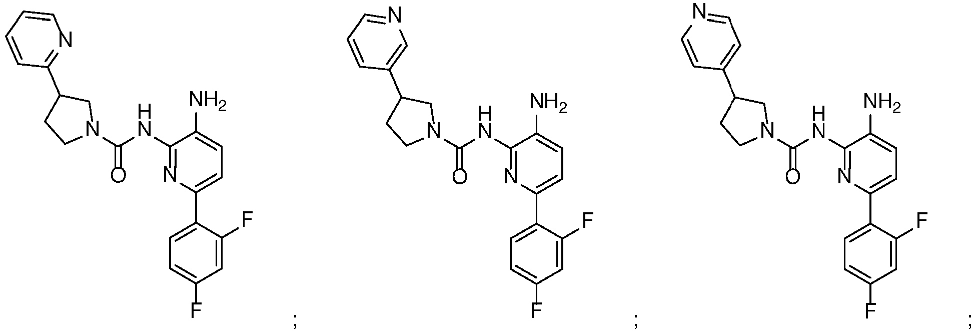 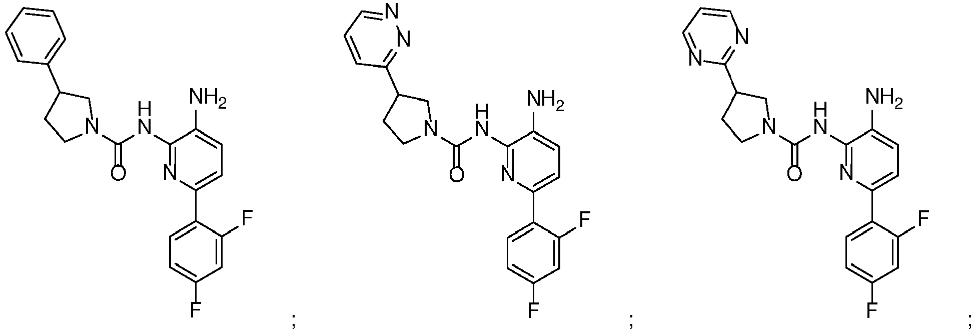 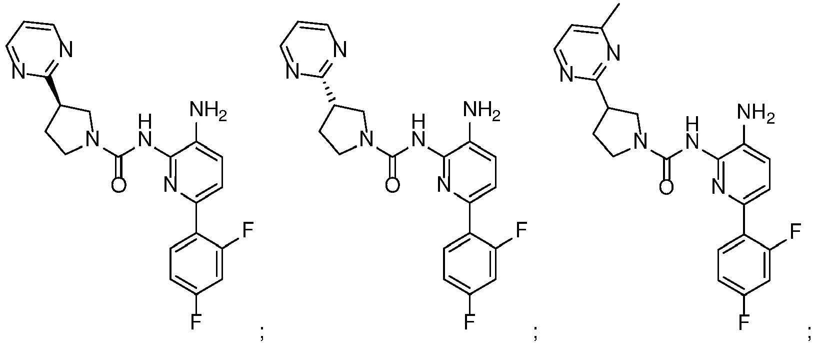 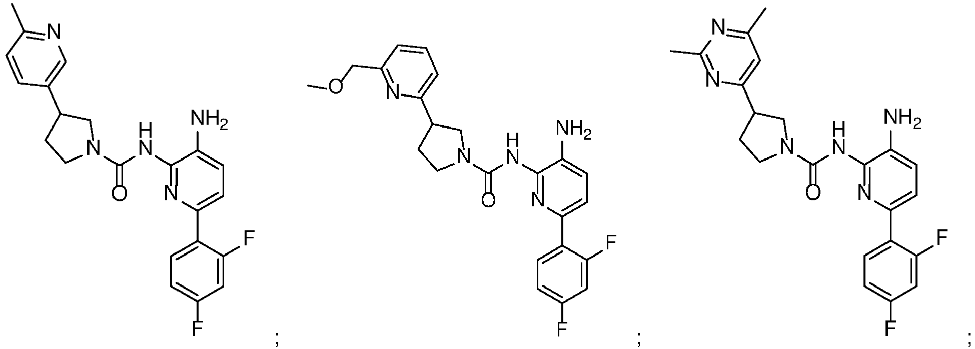 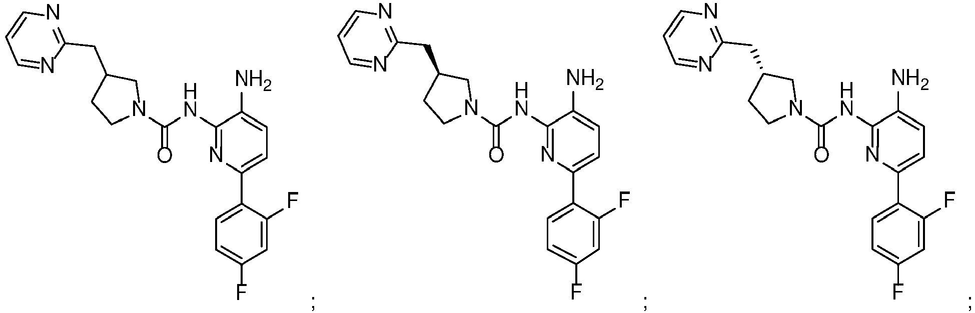 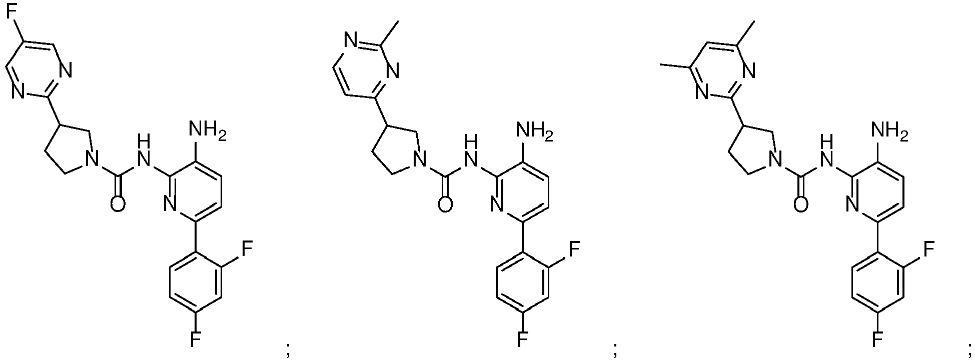 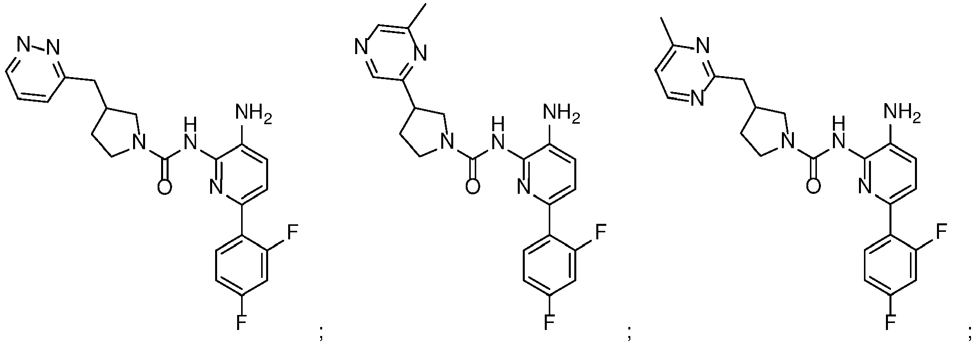 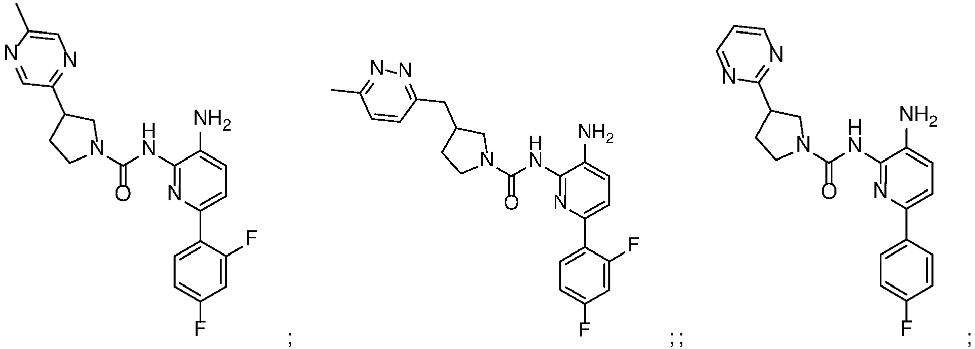 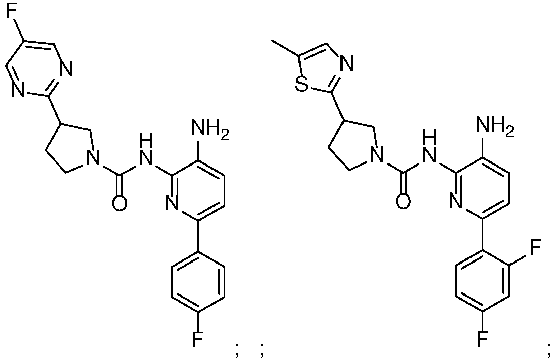 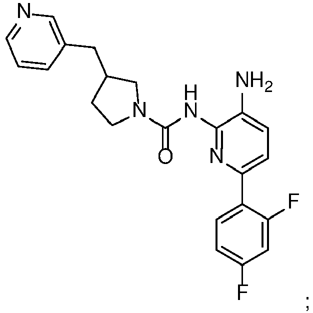 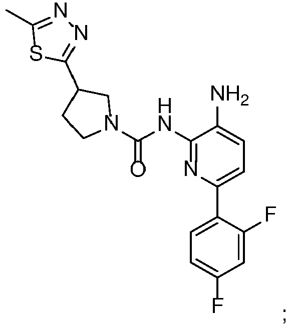 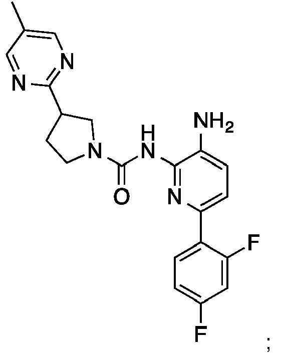 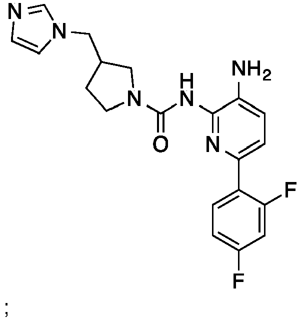 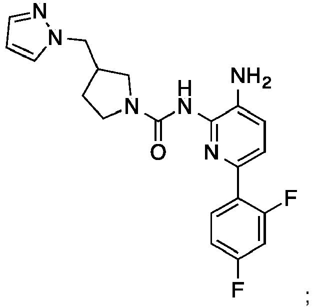 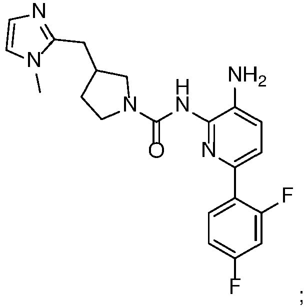 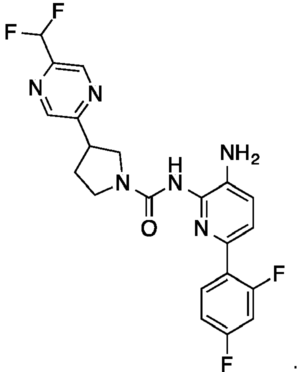 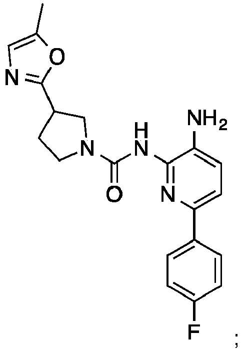 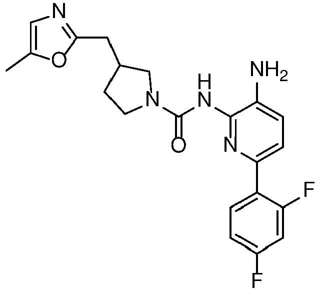 ; and 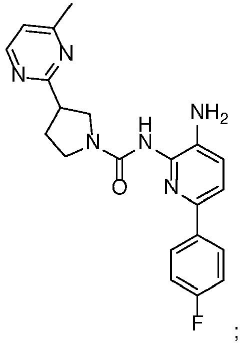 or a pharmaceutically acceptable salt thereof.

10. A composition comprising a compound of any one of Claims 1 to 9, or a pharmaceutically acceptable salt thereof; and a pharmaceutically acceptable carrier.

11. The compound of any one of Claims 1 to 9, or a pharmaceutically acceptable salt thereof, or the composition of Claim 10, for use in treating a condition selected from a neurological disorder, memory or cognitive function disorder or impairment, extinction learning disorder, fungal disease or infection, inflammatory disease, hematological disease, psychiatric disorders, and neoplastic disease.

12. The compound or composition for use of Claim 11, wherein the condition is:
a. a cognitive function disorder or impairment associated with Alzheimer's disease, posterior cortical atrophy, normal-pressure hydrocephalus, Huntington's disease, seizure induced memory loss, schizophrenia, Rubinstein Taybi syndrome, Rett Syndrome, depression, Fragile X, Lewy body dementia, stroke, vascular dementia, vascular cognitive impairment (VCI), Binswanger's Disease, fronto-temporal lobar degeneration (FTLD), ADHD, dyslexia, major depressive disorder, bipolar disorder and social, cognitive and learning disorders associated with autism, traumatic brain injury (TBI), chronic traumatic encephalopathy (CTE), multiple sclerosis (MS), attention deficit disorder, anxiety disorder, conditioned fear response, panic disorder, obsessive compulsive disorder, posttraumatic stress disorder (PTSD), phobia, social anxiety disorder, substance dependence recovery, Age Associated Memory Impairment (AAMI), Age Related Cognitive Decline (ARCD), ataxia, Parkinson's disease, or Parkinson's disease dementia; or
b. a hematological disease selected from acute myeloid leukemia, acute promyelocytic leukemia, acute lymphoblastic leukemia, chronic myelogenous leukemia, myelodysplastic syndromes, and sickle cell anemia; or
c. a neoplastic disease; or
d. a disorder of learning extinction selected from fear extinction and post-traumatic stress disorder; or
e. hearing loss or a hearing disorder; or
f. fibrotic diseases, such as pulmonary fibrosis, renal fibrosis, cardiac fibrosis, and scleroderma; or
g. bone pain in patients with cancer; or
h. neuropathic pain.

13. The compound or composition for use of Claim 12, wherein the condition is Alzheimer's disease, Huntington's disease, frontotemporal dementia, Friedreich's ataxia, post-traumatic stress disorder (PTSD), Parkinson's disease, or substance dependence recovery.

14. The compound or composition for use of Claim 11, wherein the condition is selected from Alzheimer's disease, Huntington's disease, fronto-temporal lobar degeneration, Friedreich's ataxia, post-traumatic stress disorder, Parkinson's disease, Parkinson's disease dementia, substance dependence recovery, memory or cognitive function disorder or impairment, neurological disorder with synaptic pathology, disorder of learning distinction, psychiatric disorders, cognitive function or impairment associated with Alzheimer's disease, Lewy body dementia, schizophrenia, Rubinstein Taybi syndrome, Rett Syndrome, Fragile X, multiple sclerosis, age associated memory impairment, age related cognitive decline, and social, cognitive and learning disorders associated with autism.

## Patentansprüche

1. Verbindung der Formel **VI** oder **VII:** 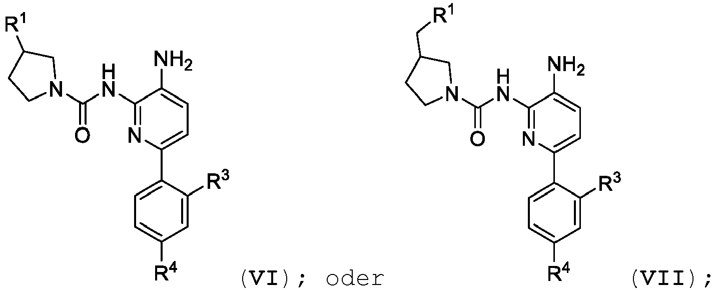
oder ein pharmazeutisch verträgliches Salz davon, wobei
R¹ Phenyl oder Heteroaryl ist, die jeweils optional mit 1 bis 3 Gruppen substituiert sind, ausgewählt aus R^{c};
R³ Wasserstoff oder Fluor ist;
R⁴ Fluor ist; und
R^{c} Halo, (C₁-C₄)Alkyl, Halo (C₁-C₄)Alkyl, (C₁-C₄)Alkoxy, Halo (C₁-C₄)Alkoxy, (C₁-C₄)AlkylO(C₁-C₄)Alkyl, (C₁-C₄)AlkylNH(C₁-C₄)Alkyl, (C₁-C₄)AlkylN((C₁-C₄)Alkyl)₂,- -(C₁-C₄)Alkylheteroaryl, oder -(C₁-C₄)Alkylheterocyclyl ist, wobei das Heteroaryl und Heterocyclyl jeweils optional und unabhängig voneinander mit 1 bis 3 Gruppen substituiert sind, die ausgewählt sind aus (C₁-C₄)Alkyl, Halogen (C₁-C₄) alkyl, (C₁-C₄)Alkoxy und Halogen;
wobei Heteroaryl einen 5- bis 12-gliedrigen aromatischen Rest darstellt, der 1 bis 4 Heteroatome, ausgewählt aus N, O und S, enthält; und
Heterocyclyl einen 4- bis 12-gliedrigen gesättigten oder teilweise ungesättigten heterocyclischen Ring darstellt, der 1 bis 4 Heteroatome enthält, die unabhängig voneinander aus N, O und S ausgewählt sind.

2. Verbindung nach Anspruch 1, wobei R¹ Phenyl oder 5-bis 6-gliedriges monocyclisches Heteroaryl ist, das jeweils optional mit einer oder mehreren Gruppen, ausgewählt aus R^{c}, substituiert ist.

3. Verbindung nach Anspruch 1 oder 2, wobei R¹ Phenyl, Pyridinyl, Pyrazinyl, Pyridazinyl oder Pyrimidinyl ist, die jeweils optional mit 1 bis 2 Gruppen, ausgewählt aus R^{c}, substituiert sind.

4. Verbindung nach einem der Ansprüche 1 bis 3, wobei R¹ Thiazolyl, Thiadiazolyl, Imidazolyl, Pyrazolyl oder Oxazolyl ist, von denen jedes optional mit 1 bis 2 Gruppen, ausgewählt aus R^{c}, substituiert ist.

5. Verbindung nach einem der Ansprüche 1 bis 4, wobei R^{c} Halogen, (C₁-C₄)Alkyl oder (C₁-C₄)AlkylO(C₁-C₄)Alkyl ist.

6. Verbindung nach einem der Ansprüche 1 bis 5, wobei R^{c} Fluor, Methyl oder CH₂OCH₃ ist.

7. Verbindung nach einem der Ansprüche 1 bis 6, wobei R^{c} Halo, Halo (C₁-C₄)Alkyl oder (C₁-C₄)Alkyl ist.

8. Verbindung nach einem der Ansprüche 1 bis 7, wobei R^{c} Fluor, Methyl oder CHF₂ ist.

9. Verbindung nach Anspruch 1, wobei die Verbindung ausgewählt ist aus 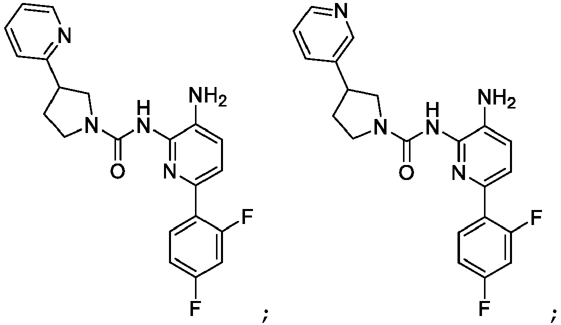 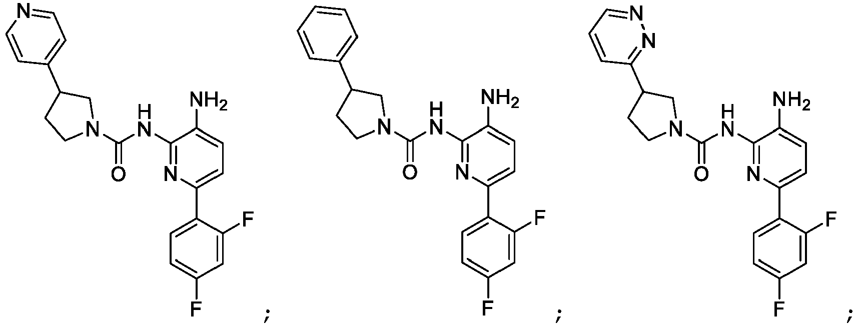 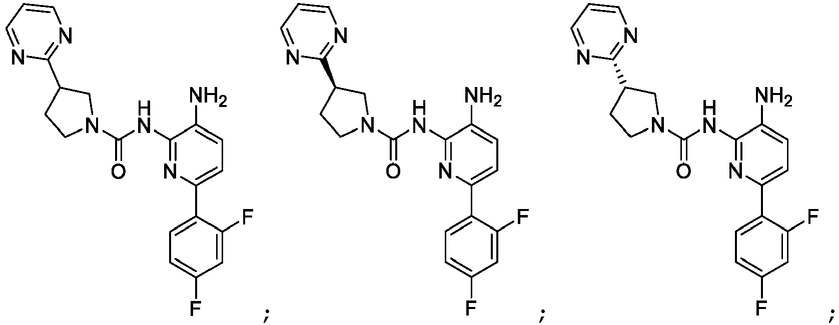 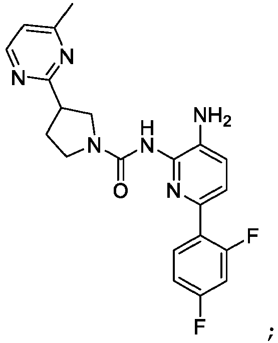 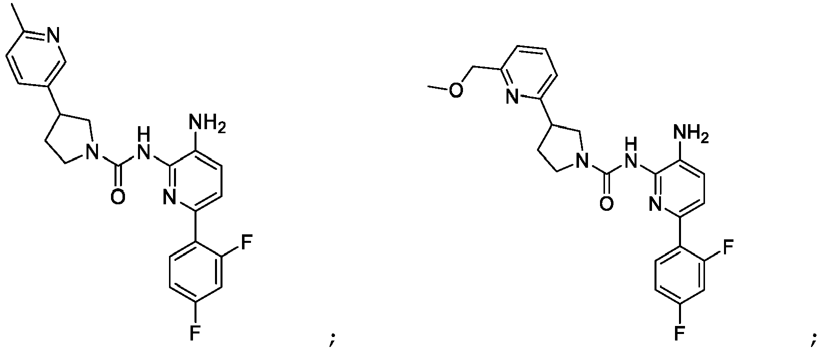 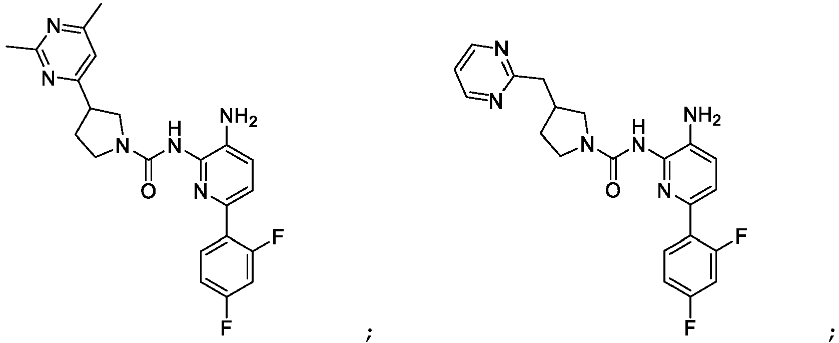 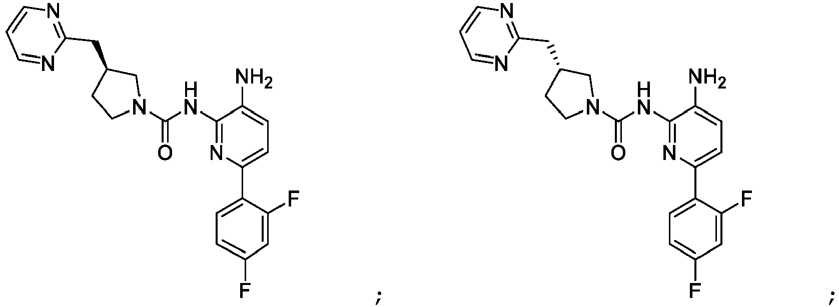 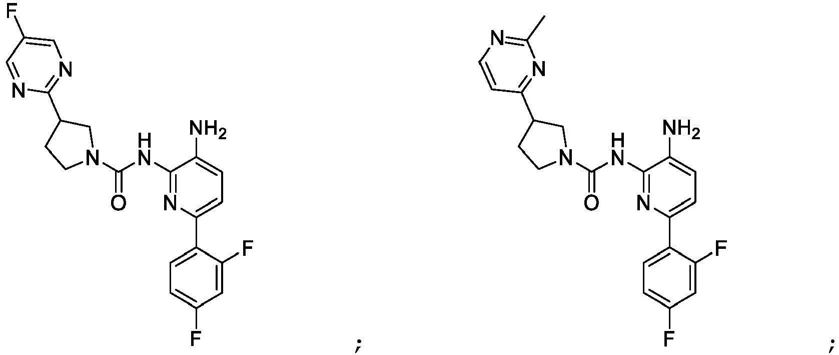 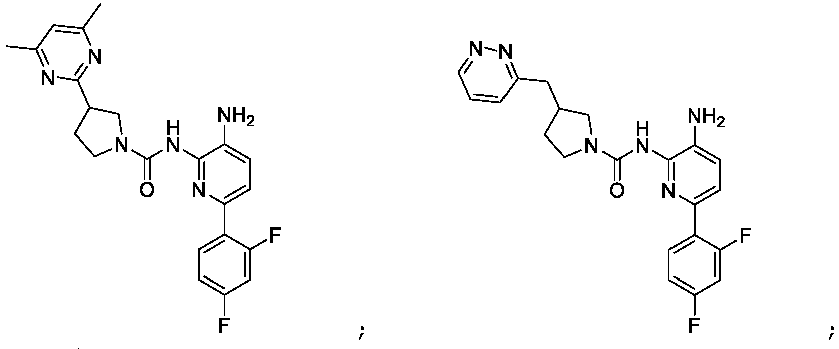 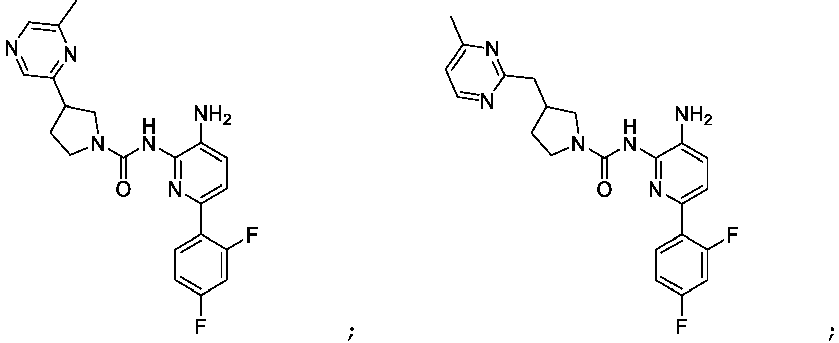 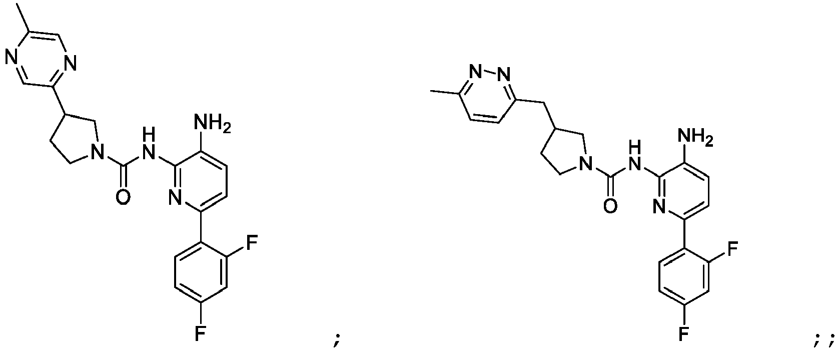 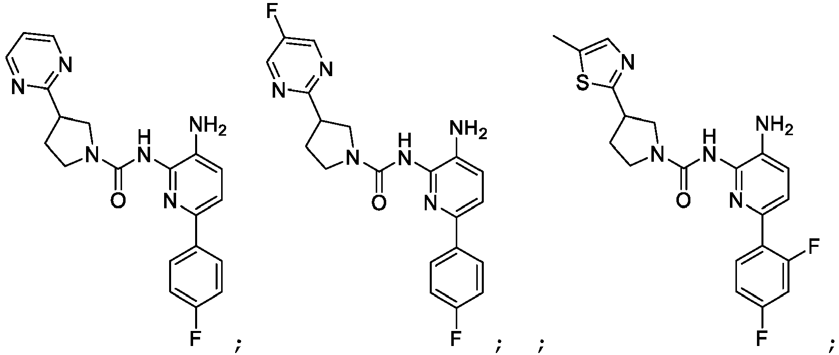 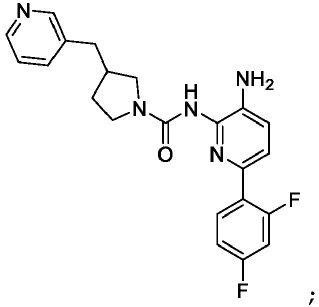 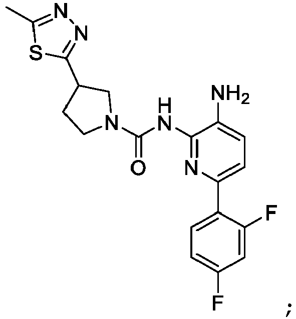 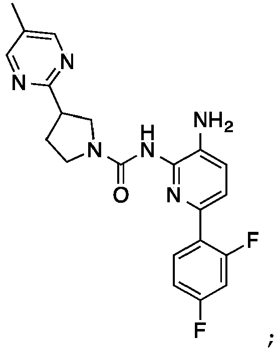 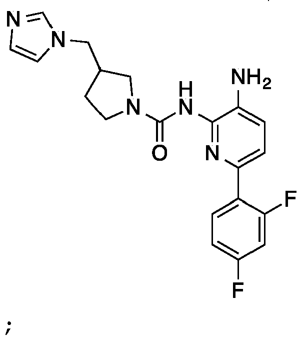 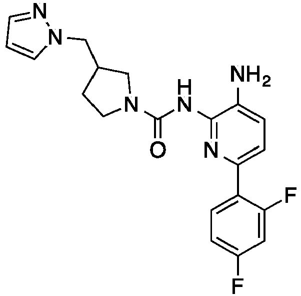 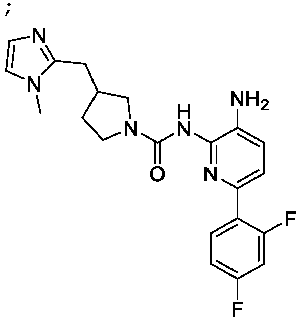 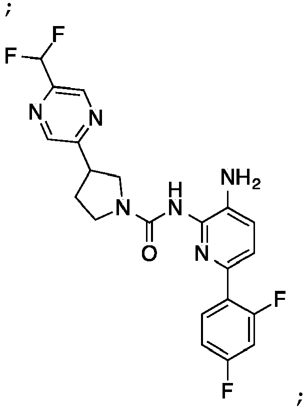 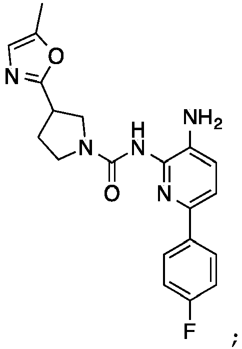 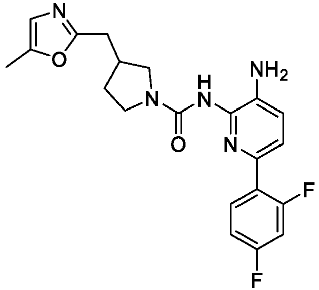 ; und 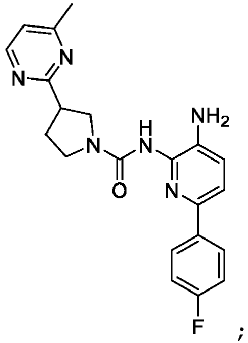 oder ein pharmazeutisch verträgliches Salz davon.

10. Zusammensetzung, umfassend eine Verbindung nach einem der Ansprüche 1 bis 9 oder ein pharmazeutisch verträgliches Salz davon; und einen pharmazeutisch verträglichen Träger.

11. Verbindung nach einem der Ansprüche 1 bis 9 oder ein pharmazeutisch verträgliches Salz davon oder die Zusammensetzung nach Anspruch 10 zur Verwendung bei der Behandlung eines Zustands, ausgewählt aus einer neurologischen Störung, einer Störung oder Beeinträchtigung des Gedächtnisses oder der kognitiven Funktion, einer Störung des Extinktionslernens, einer Pilzerkrankung oder -Infektion, einer entzündlichen Erkrankung, einer hämatologischen Erkrankung, psychiatrischen Störungen und einer neoplastischen Erkrankung.

12. Verbindung oder Zusammensetzung zur Verwendung nach Anspruch 11, wobei der Zustand Folgendes ist:
a. eine Störung oder Beeinträchtigung der kognitiven Funktion, die der Alzheimer-Krankheit, der posterioren kortikalen Atrophie, dem Normaldruckhydrozephalus, der Huntington-Krankheit, dem anfallsbedingten Verlust des Gedächtnisses, der Schizophrenie, dem Rubinstein-Taybi-Syndrom und dem Rett-Syndrom, Depression, dem fragilen X, Lewy-Körper-Demenz, Schlaganfall, vaskulärer Demenz, vaskulärer kognitiver Beeinträchtigung (VCI), Morbus Binswanger, frontotemporaler Lobärdegeneration (FTLD), ADHS, Legasthenie, schwerer depressiver Störung, bipolarer Störung und sozialen, kognitiven und Lernstörungen, die dem Autismus zugeordnet sind, traumatischer Hirnverletzung (TBI), chronischer traumatischer Enzephalopathie (CTE), Multipler Sklerose (MS), Aufmerksamkeitsdefizitstörung, Angststörung, konditionierte Angstantwort, Panikstörung, Zwangsstörung, posttraumatischer Belastungsstörung (PTSD), Phobie, sozialer Angststörung, Stoffabhängigkeit, altersbedingter Speicherbeeinträchtigung (AAMI), altersbedingtem kognitiven Verfall (ARCD), Ataxie, Parkinson-Krankheit oder Parkinson-Demenz zugeordnet ist; oder
b. eine hämatologische Erkrankung, ausgewählt aus akuter myeloischer Leukämie, akuter promyeloischer Leukämie, akuter lymphoblastischer Leukämie, chronischer myeloischer Leukämie, myelodysplastischen Syndromen und Sichelzellenanämie; oder
c. eine neoplastische Erkrankung; oder
d. eine Störung der Lernextinktion, ausgewählt aus Angst-Extinktion und posttraumatischer Spannung; oder
e. Hörverlust oder eine Hörstörung; oder
f. fibrotische Erkrankungen wie Lungenfibrose, Nierenfibrose, Herzfibrose und Sklerodermie; oder
g. Knochenschmerzen bei Patienten mit Krebs; oder
h. neuropathische Schmerzen.

13. Verbindung oder Zusammensetzung zur Verwendung nach Anspruch 12, wobei der Zustand die Alzheimer-Krankheit, die Huntington-Krankheit, die frontotemporale Demenz, die Friedreich-Ataxie, die posttraumatische Belastungsstörung (PTSD), die Parkinson-Krankheit oder die Genesung von Stoffabhängigkeit ist.

14. Verbindung oder Zusammensetzung zur Verwendung nach Anspruch 11, wobei der Zustand ausgewählt ist aus der Alzheimer-Krankheit, der Huntington-Krankheit, der fronto-temporalen lobären Degeneration, der Friedreich-Ataxie und der posttraumatischen Spannung, der Parkinson-Krankheit, der Parkinson-Demenz, der Wiederherstellung der Stoffabhängigkeit, der Störung oder Beeinträchtigung des Gedächtnisses oder der kognitiven Funktion, der neurologischen Störung mit synaptischer Pathologie, der Störung der Lernunterscheidung, der psychiatrischen Störungen, der kognitiven Funktion oder Beeinträchtigung, die der Alzheimer-Krankheit, der Lewy-Körper-Demenz, der Schizophrenie, dem Rubinstein-Taybi-Syndrom, dem Rett-Syndrom, dem Fragilen X, der Multiplen Sklerose zugeordnet sind, der altersbedingten Beeinträchtigung des Gedächtnisses, dem altersbedingten kognitiven Verfall und den sozialen, kognitiven und Lernstörungen, die dem Autismus zugeordnet sind.

## Revendications

1. Composé de formule VI ou VII : 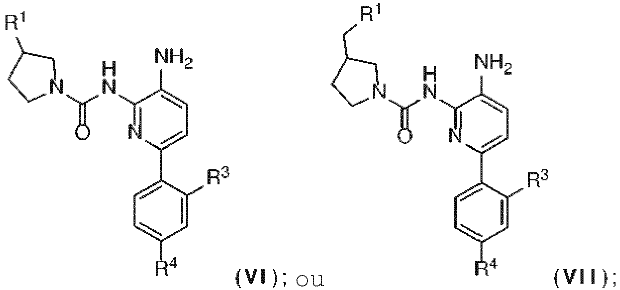 ou sel pharmaceutiquement acceptable de celui-ci, dans lequel
R¹ est un phényle ou un hétéroaryle, chacun étant éventuellement substitué par 1 à 3 groupes choisis parmi R^{c} ;
R³ est un hydrogène ou un fluoro ;
R⁴ est un fluor ; et
R^{c} est un halogène, un alkyle en C₁-C₄, un halogénoalkyle en C₁-C₄, un alcoxy en C₁-C₄, un halogénoalcoxy en C₁-C₄, un (alkyl en C₁-C₄)O(alkyle en C₁-C₄), un (alkyl en C₁-C₄)NH(alkyle en C₁-C₄), un (alkyl en C₁-C₄)NH (alkyle en C₁-C₄)₂, un -(alkyl en C₁-C₄)hétéroaryle ou un - (alkyl en C₁-C₄)hétérocyclyle, dans lequel lesdits hétéroaryle et hétérocyclyle sont chacun éventuellement et indépendamment substitués par 1 à 3 groupes choisis parmi un alkyle en C₁-C₄, un halogénoalkyle en C₁-C₄, un alcoxy en C₁-C₄ et un halogéno ; dans lequel l'hétéroaryle représente un radical aromatique de 5 à 12 chaînons contenant 1 à 4 hétéroatomes choisis parmi N, O et S ; et
l'hétérocyclyle représente un cycle hétérocyclique saturé ou partiellement insaturé de 4 à 12 chaînons contenant 1 à 4 hétéroatomes indépendamment choisis parmi N, O et S.

2. Composé selon la revendication 1, dans lequel R¹ est un phényle ou un hétéroaryle monocyclique à 5 ou 6 chaînons, dont chacun est éventuellement substitué par un ou plusieurs groupes choisis parmi R^{c}.

3. Composé selon la revendication 1 ou 2, dans lequel R¹ est un phényle, un pyridinyle, un pyrazinyle, un pyridazinyle ou un pyrimidinyle, dont chacun est éventuellement substitué par 1 ou 2 groupes choisis parmi R^{c}.

4. Composé selon l'une quelconque des revendications 1 à 3, dans lequel R¹ est un thiazolyle, un thiadiazolyle, un imidazolyle, un pyrazolyle ou un oxazolyle, dont chacun est éventuellement substitué par 1 ou 2 groupes choisis parmi R^{c}.

5. Composé selon l'une quelconque des revendications 1 à 4, dans lequel R^{c} est un halogène, un alkyle en C₁-C₄ ou un (alkyl en C₁-C₄)O(alkyle en C₁-C₄).

6. Composé selon l'une des revendications 1 à 5, dans lequel R^{c} est un fluoro, un méthyle ou CH₂OCH₃.

7. Composé selon l'une quelconque des revendications 1 à 6, dans lequel R^{c} est un halogène, un halogénoalkyle en C₁-C₄ ou un alkyle en C₁-C₄.

8. Composé selon l'une des revendications 1 à 7, dans lequel R^{c} est un fluoro, un méthyle, ou CHF₂.

9. Composé selon la revendication 1, dans lequel le composé est choisi parmi 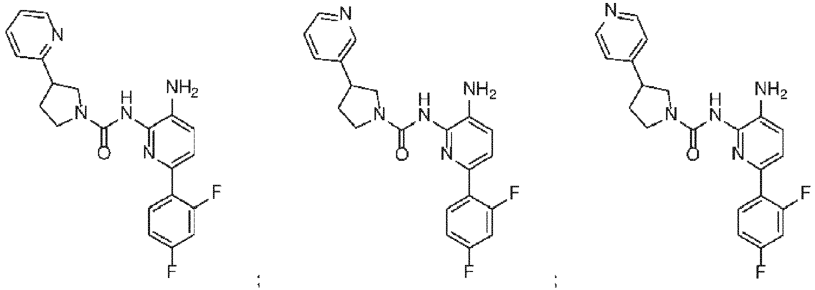 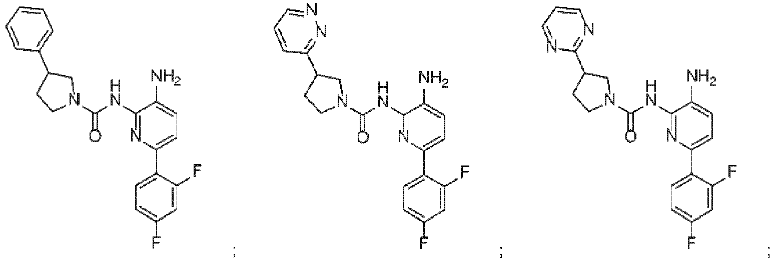 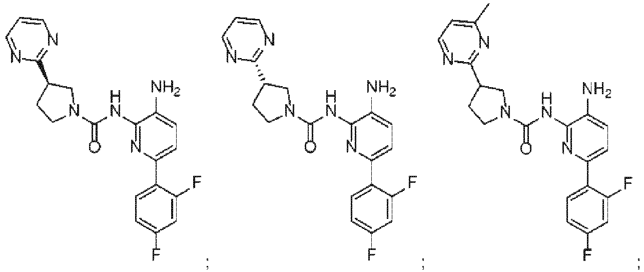 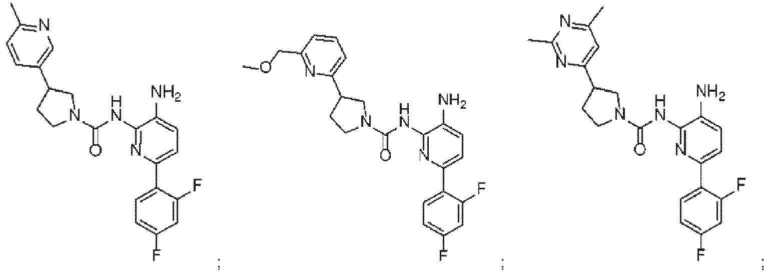 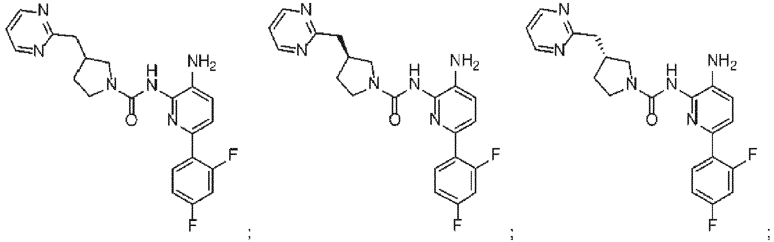 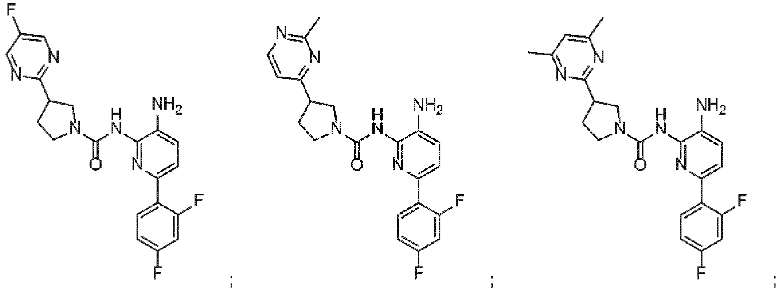 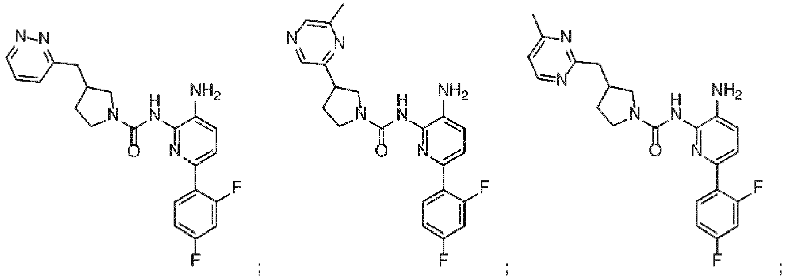 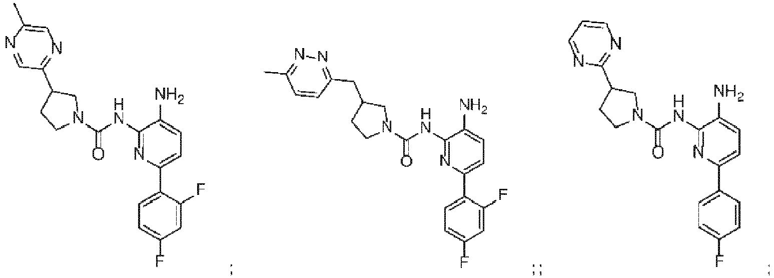 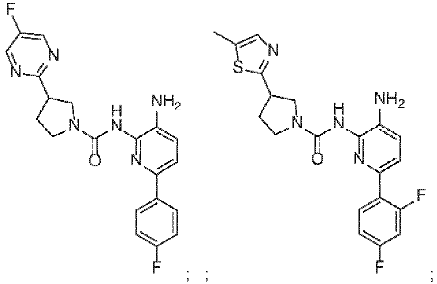 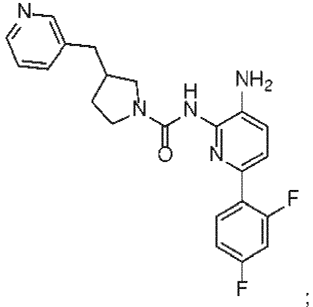 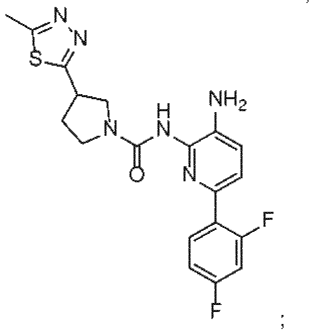 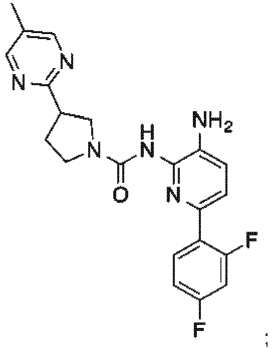 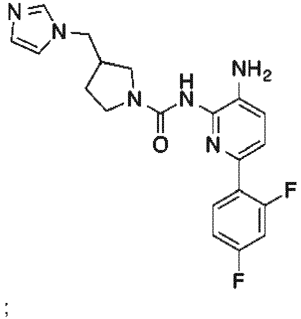 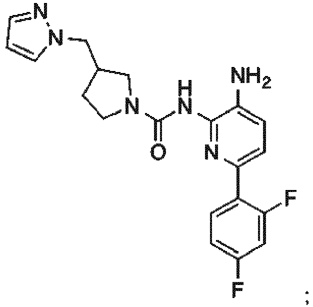 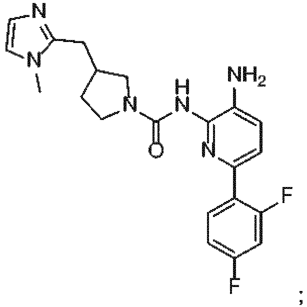 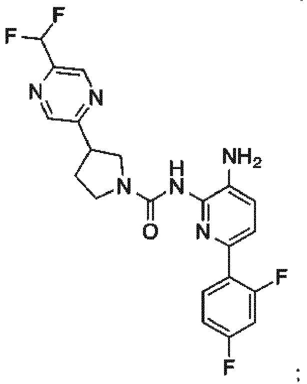 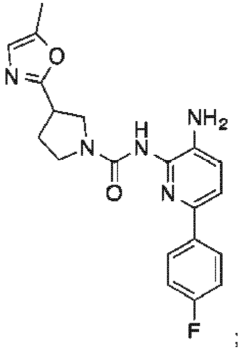 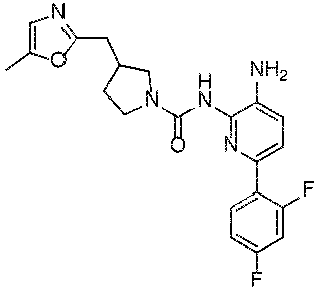 ; et 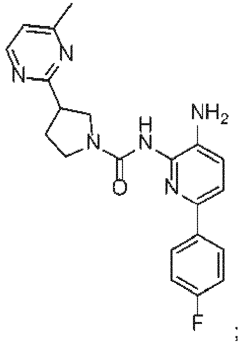 ou un sel pharmaceutiquement acceptable de celui-ci.

10. Composition comprenant un composé selon l'une quelconque des revendications 1 à 9, ou un sel pharmaceutiquement acceptable de celui-ci ; et un véhicule pharmaceutiquement acceptable.

11. Composé selon l'une quelconque des revendications 1 à 9, ou sel pharmaceutiquement acceptable de celui-ci, ou composition selon la revendication 10, destiné à être utilisé dans le traitement d'une pathologie choisie parmi un trouble neurologique, un trouble ou une déficience de la mémoire ou de la fonction cognitive, un trouble d'apprentissage par extinction, une maladie ou une infection fongique, une maladie inflammatoire, une maladie hématologique, des troubles psychiatriques et une maladie néoplasique.

12. Composé ou composition destiné à être utilisé selon la revendication 11, dans lequel la pathologie est :
a. un trouble ou une déficience de la fonction cognitive associé à la maladie d'Alzheimer, à l'atrophie corticale postérieure, à l'hydrocéphalie à pression normale, à la maladie de Huntington, à la perte de mémoire induite par des convulsions, à la schizophrénie, au syndrome de Rubinstein Taybi, au syndrome de Rett, à la dépression, au X fragile, à la démence à corps de Lewy, à l'accident vasculaire cérébral, à la démence vasculaire, à la déficience cognitive vasculaire (VCI), à la maladie de Binswanger, à la dégénérescence lobaire fronto-temporale (DLFT), au TDAH, à la dyslexie, au trouble dépressif majeur, au trouble bipolaire et aux troubles sociaux, cognitifs et d'apprentissage associés à l'autisme, au traumatisme crânien (TCC), à l'encéphalopathie traumatique chronique (CTE), à la sclérose en plaques (SEP), au trouble déficitaire de l'attention, au trouble anxieux, à la réaction de peur conditionnée, au trouble de panique, au trouble obsessionnel compulsif, au trouble de stress post-traumatique (SSPT), à la phobie, au trouble d'anxiété sociale, au rétablissement de la dépendance à une substance, aux troubles de la mémoire associés à l'âge (AAMI), au déclin cognitif lié à l'âge (ARCD), à l'ataxie, à la maladie de Parkinson ou à la démence liée à la maladie de Parkinson ; ou
b. une maladie hématologique choisie parmi la leucémie myéloïde aiguë, la leucémie promyélocytaire aiguë, la leucémie lymphoblastique aiguë, la leucémie myéloïde chronique, les syndromes myélodysplasiques et l'anémie falciforme ; ou
c. une maladie néoplasique ; ou
d. un trouble d'extinction de l'apprentissage choisi parmi l'extinction de la peur et le trouble de stress post-traumatique ; ou
e. une perte auditive ou un trouble auditif ; ou
f. des maladies fibrotiques, telles que la fibrose pulmonaire, la fibrose rénale, la fibrose cardiaque et la sclérodermie ; ou
g. des douleurs osseuses chez les patients atteints de cancer ; ou
h. une douleur neuropathique.

13. Composé ou composition destiné à être utilisé selon la revendication 12, dans lequel l'état est la maladie d'Alzheimer, la maladie de Huntington, la démence frontotemporale, l'ataxie de Friedreich, le trouble de stress post-traumatique (TSPT), la maladie de Parkinson ou le rétablissement d'une dépendance à une substance.

14. Composé ou composition destiné à être utilisé selon la revendication 11, dans lequel la pathologie est choisie parmi la maladie d'Alzheimer, la maladie de Huntington, la dégénérescence lobaire fronto-temporale, l'ataxie de Friedreich, le trouble de stress post-traumatique, la maladie de Parkinson, la démence due à la maladie de Parkinson, la guérison d'une dépendance à une substance, le trouble ou la déficience de la mémoire ou de la fonction cognitive, le trouble neurologique avec pathologie synaptique, le trouble de la distinction des apprentissages, les troubles psychiatriques, la fonction ou la déficience cognitive associée à la maladie d'Alzheimer, la démence à corps de Lewy, la schizophrénie, le syndrome de Rubinstein Taybi, le syndrome de Rett, le X fragile, la sclérose en plaques, les troubles de la mémoire liés à l'âge, le déclin cognitif lié à l'âge et les troubles sociaux, cognitifs et d'apprentissage liés à l'autisme.
